# EUROPÄISCHE PATENTSCHRIFT

(11) **EP 2 280 976 B1**
(45) Veröffentlichungstag und Bekanntmachung des Hinweises auf die Patenterteilung: **01.01.2014**
(21) Anmeldenummer: 09745510.9
(22) Anmeldetag: 02.05.2009
(51) Int. Cl.: C07D 487/04, A61K 31/519, A61P 7/00

(54) **SUBSTITUIERTE IMIDAZO- UND TRIALZOLOPYRIMIDINE, IMIDAZO- UND PYRAZOLOPYRAZINE UND IMIDAZOTRIAZINE ALS GSK3BETA-INHIBIT0REN**
SUBSTITUTED IMIDAZO- AND TRIAZOLOPYRIMIDINES, IMIDAZO- AND PYRAZOLOPYRAZINES AND IMIDAZOTRIAZINES AS GSK3BETA-INHIBITORS
IMIDAZOPYRIMIDINES ET TRIAZOLOPYRIMIDINES SUBSTITUÉES, IMIDAZOPYRAZINES, PYRAZOLOPYRAZINES ET IMIDAZOTRIAZINES SUBSTITUÉES SERVANT D INHIBITEURS DE GSK3-BÊTA

(30) Priorität: 15.05.2008 DE 102008023801
(43) Veröffentlichungstag der Anmeldung: 09.02.2011
(73) Patentinhaber: Bayer Intellectual Property GmbH, 40789 Monheim (DE)
(72) Erfinder: SIEGEL, Stephan, 10779 Berlin (DE); WILMEN, Andreas, 50676 Köln (DE); SVENSTRUP, Niels, 42553 Velbert (DE); GNOTH, Mark, Jean, 40822 Mettmann (DE); TERSTEEGEN, Adrian, 42111 Wuppertal (DE); RESTER, Ulrich, 42115 Wuppertal (DE)
(74) Vertreter: BIP Patents
(86) Internationale Anmeldenummer: PCT/EP2009/003167
(87) Internationale Veröffentlichungsnummer: WO 2009/138176

(56) Entgegenhaltungen:
- WO-A-2005/035532
- WO-A-2008/113469
- DATABASE CA [Online] CHEMICAL ABSTRACTS SERVICE, COLUMBUS, OHIO, US; FISHMAN, PNINA ET AL: "Adenosine receptor ligands for the modulation of glycogen synthase kinase 3.beta. (GSK-3.beta.) activity, and therapeutic uses" XP002536566 gefunden im STN Database accession no. 2002:638281 & US 2002/115635 A1 (FISHMAN, PNINA ET AL) 22. August 2002 (2002-08-22)

## Beschreibung

Die Erfindung betrifft substituierte Imidazo- und Triazolopyrimidine, Imidazo- und Pyrazolopyrazine und Imidazotriazine und Verfahren zu ihrer Herstellung sowie ihre Verwendung zur Herstellung von Arzneimitteln zur Behandlung und/oder Prophylaxe von Krankheiten, insbesondere von hämatologischen Erkrankungen, vorzugsweise von Leukopenien und Neutropenien.

Glykogen Synthase Kinase 3 (GSK3) gehört zur Familie der Serine/Threonin-Kinasen. Spezifische Substrate sind unter anderem Zytoskelettproteine und Transkriptionsfaktoren. Zwei Isoformen, GSK3α und GSK3β, wurden bisher identifiziert (Woodgett JR., Trends Biochem. Sci. (1991), 16(5), 177-81). Beide Isoformen sind in vornehmlich ruhenden, nicht proliferierenden Zellen konstitutiv aktiv.

GSK3β kommt eine zentrale Bedeutung innerhalb des Wnt/Wingless-Signaltransduktionsweges zu. Dieser stellt einer der wichtigsten, evolutionärkonservierten Signalsysteme dar. Wnt-Signale kontrollieren sehr frühe musterbildende Prozesse während der Embryogenese, sie induzieren Mesodermbildung und viele Organe, und sie steuern die Proliferation und Differenzierung von Stammzellen (Wodarz A., Nusse R., Annu. Rev. Cell Dev. Biol. (1998), 14, 59-88; Kirstetter et al., Nat Immunol. (2006), 7(10), 1048-56). Der Wnt-Signalweg ist intrazellulär aufgegliedert, wodurch unterschiedlichste Prozesse gesteuert werden können. Innerhalb der Wnt-Kaskade ist die Glykogen Synthase Kinase 3 Bestandteil eines Multiproteinkomplexes, zu dem u.a. das Strukturmoleküle Axin, das Tumorsuppressor-Protein APC sowie der Transkriptionskofaktor β-Catenin gehören. β-Catenin ist dabei das wichtigste Substrat der GSK3β. Die Konsequenz dieser GSK3β-vermittelten Phophorylierung ist der proteasomale Abbau von β-Catenin. Inhibition der GSK3-Aktivität führt zu einer Akkumulation des β-Catenins in der Zelle mit einer sich anschließenden Translokation in den Zellkern. Dort fungiert β-Catenin als ein Kofaktor in Transkriptionskomplexe und damit für die Expression definierter Zielgene mit verantwortlich.

Strahlen- oder Chemotherapien gehören zu den Standardansätzen bei der Krebsbekämpfung. Beide Therapieformen sind im Bezug auf ihre Zielzellen unspezifisch, d.h. es werden nicht nur Tumorsondern auch nicht-transformierte, proliferierende Zellen getroffen. Zu diesen nicht-transformierten, proliferienden Zellen gehören auch hämatopoetische Vorläuferzellen, die sich u.a. zu neutrophilen Granulozyten entwickeln. Eine signifikante Verringerung der Anzahl an Neutrophilen wird als Neutropenie bezeichnet. Eine durch Chemo- oder Strahlentherapie induzierte Neutropenie resultiert klinisch in einer erhöhten Infektanfälligkeit. Bei ausgeprägter Neutropenie erhöht sich die Morbidität und unter Umständen auch die Mortalität einer Therapie (O'Brien et al., British Journal of Cancer (2006), 95, 1632 - 1636).

Inhibition der GSK3-Aktivität führt zu einer gesteigerten Proliferations- und Differenzierungsrate hämatopoetischer Stammzellen und kann dementsprechend zur therapeutischen Intervention hinsichtlich einer therapieinduzierten Neutropenie genutzt werden.

W02006/044687 beschreibt die Verwendung von Imidazopyrimidinylaminen, Pyrazolo- und Imidazopyrazinen als Kinase Inhibitoren zur Behandlung von Krebs und WO01/083485 offenbart Imidazo- und Triazolopyrimidine unter anderem zur Behandlung von Asthma und Krebs. WO2005/044793 offenbart unter anderem die Verwendung von Imidazopyrimidinylaminen als CRF (corticotropin releasing factor) Rezeptor Antagonisten zur Behandlung von Depressionen. W02007/138072 beschreibt die Verwendung von 6-Alkyl-substituierten Triazolopyrazinen zur Behandlung von degenerativen und inflammatorischen Erkrankungen. WO99/064401 beschreibt unter anderem Imidazopyrazine als Somatostatin Rezeptor Liganden zur Behandlung von Diabetes. W02004/026877, US2006/0183746, US2006/0106023 und W02007/058873 beschreiben die Verwendung von Imidazopyrazinylaminen zur Behandlung von Krebs. WO 2007/145921 beschreibt Imidazopyrazine als Protein Kinase Inhibitoren zur Behandlung von Krebs. WO03/000693 beansprucht Imidazotriazine als PDE10-Inhhibitoren zur Behandlung von neurodegenerativen Krankheiten. WO 2005/035532 offenbart substituierte Triazolopyrazine und ihre Verwendung als GSK3-Inhibitoren.

Eine Aufgabe der vorliegenden Erfindung ist daher die Bereitstellung neuer Verbindungen als GSK3β-Inhibitoren zur Behandlung von hämatologischen Erkrankungen, vorzugsweise von Neutropenie bei Menschen und Tieren.

Gegenstand der Erfindung sind Verbindungen der Formel 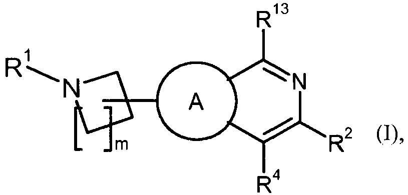 in welcher
- A: für eine Gruppe der Formel 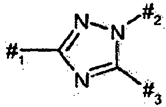 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für Wasserstoff, C₁-C₆-Alkyl, C₃-C₆-Cycloalkyl, C₃-C₆-Cycloalkylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
- R⁴: für Wasserstoff, Chlor oder Fluor steht,
- R¹³: für eine Gruppe der Formel 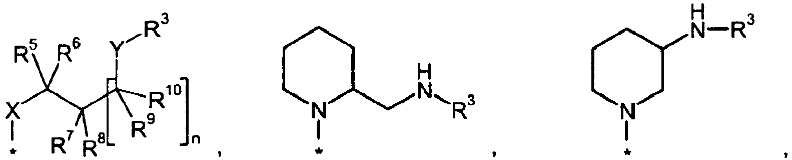 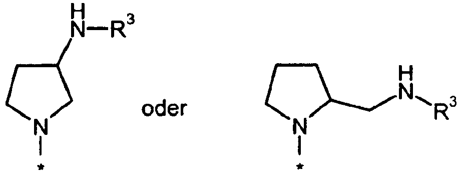 steht,
wobei
* die Anknüpfstelle an den Heterocyclus bedeutet,
n für die Zahl 0 oder 1 steht,
X für NR¹¹, S oder O steht,
wobei
R¹¹ für Wasserstoff, C₁-C₃-Alkyl oder Cyclopropyl steht,
- Y: für NR¹², S oder O steht,
wobei
R¹² für Wasserstoff, C₁-C₃-Alkyl oder Cyclopropyl steht,
- R³: für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht,
wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
- R⁵: für Wasserstoff, C₁-C₃-Alkyl oder Cyclopropyl steht,
- R⁶: für Wasserstoff oder C₁-C₃-Alkyl steht,
- R⁷: für Wasserstoff, C₁-C₃-Alkyl oder Cyclopropyl steht,
- R⁸: für Wasserstoff oder C₁-C₃-Alkyl steht,
- R⁹: für Wasserstoff, C₁-C₃-Alkyl oder Cyclopropyl steht,
- R¹⁰: für Wasserstoff oder C₁-C₃-Alkyl steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N-*Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden, Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylcarbonyl, Alkoxycarbonyl, Alkylaminocarbonyl, Alkylcarbonylamino, Alkylsulfonyl, Alkylsulfonylamino und Alkylaminosulfonyl stehen für einen linearen oder verzweigten Alkylrest mit 1 bis 6, bevorzugt 1 bis 4 Kohlenstoffatomen, beispielhaft und vorzugsweise für Methyl, Ethyl, n-Propyl, iso-Propyl, n-Butyl, tert-Butyl, n-Pentyl und n-Hexyl.
Alkoxy steht beispielhaft und vorzugsweise für Methoxy, Ethoxy, n-Propoxy, iso-Propoxy, n-Butoxy und tert-Butoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N-*Dimethylamino, *N,N-*Diethylamino, *N*-Ethyl-*N-*methylamino, *N-*Methyl-*N*-n-propylamino, *N*-iso-Propyl-*N*-n-propylamino und *N*-tert-Butyl-*N-*methylamino. C₁-C₄-Alkylamino steht beispielsweise für einen Monoalkylaminorest mit 1 bis 4 Koh-lenstoffatomen oder für einen Dialkylaminorest mit jeweils 1 bis 4 Kohlenstoffatomen pro Alkylsubstituent.
Alkylcarbonyl steht beispielhaft und vorzugsweise für Methylcarbonyl, Ethylcarbonyl, n-Propylcarbonyl, iso-Propylcarbonyl, n-Butylcarbonyl und tert-Butylcarbonyl.
Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl und tert-Butoxycarbonyl.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N*-Dimethylaminocarbonyl, *N,N-*Diethylaminocarbonyl, *N-*Ethyl-*N-*methylaminocarbonyl, *N-*Methyl-*N*-n-propylaminocarbonyl, *N*-iso-Propyl-*N*-n-propylaminocarbonyl und *N-*tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylamino-carbonylrest mit jeweils 1 bis 4 Kohlenstoffatomen pro Alkylsubstituent.
Alkylcarbonylamino steht beispielhaft und vorzugsweise für Methylcarbonylamino, Ethyl-carbonylamino, n-Propylcarbonylamino, iso-Propylcarbonylamino, n-Butylcarbonylamino und tert-Butylcarbonylamino.
Alkylsulfonyl steht beispielhaft und vorzugsweise für Methylsulfonyl, Ethylsulfonyl, n-Propylsulfonyl, iso-Propylsulfonyl, n-Butylsulfonyl und tert-Butylsulfonyl.
Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, iso-Propylaminosulfonyl, tert-Butylaminosulfonyl, *N,N-*Dimethylaminosulfonyl, *N,N-*Diethylaminosulfonyl, *N*-Ethyl-*N*-methylaminosulfonyl, *N-*Methyl-*N-*n-propylaminosulfonyl, *N*-iso-Propyl-*N*-n-propylaminosulfonyl und *N-tert*-Butyl-*N-*methylaminosulfonyl. C₁-C₄-Alkylaminosulfonyl steht beispielsweise für einen Monoalkylaminosulfonylrest mit 1 bis 4 Kohlenstoffatomen oder für einen Dialkylamino-sulfonylrest mit jeweils 1 bis 4 Kohlenstoffatomen pro Alkylsubstituent.
Alkylsulfonylamino steht beispielhaft und vorzugsweise für Methylsulfonylamino, Ethyl-sulfonylamino, n-Propylsulfonylamino, iso-Propylsulfonylamino, n-Butylsulfonylamino und tert-Butylsulfonylamino.
Cycloalkyl steht für eine monocyclische Cycloalkylgruppe mit in der Regel 3 bis 6 Kohlen-stoffatomen, beispielhaft und vorzugsweise für Cycloalkyl seien genannt Cyclopropyl, Cyclobutyl, Cyclopentyl und Cyclohexyl.

Halogen steht für Fluor, Chlor, Brom und Jod, vorzugsweise für Fluor und Chlor.

In den Formeln der Gruppe, die für A stehen kann, steht der Endpunkt der Linie, neben der jeweils ein #₁, #₂ oder #₃ steht, nicht für ein Kohlenstoffatom beziehungsweise eine CH₂-Gruppe sondern ist Bestandteil der Bindung zu dem Atom, an das A gebunden ist.

In den Formeln der Gruppe, die für R¹³ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹³ gebunden ist.

Der Heterozyklus der Formel 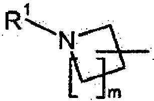 kann über eine beliebige Position des Heterozyklus an A gebunden sein und ist nicht festgelegt auf die beiden Positionen, die sich in direkter Nachbarschaft zu der gezeichneten Bindung befinden.

Bevorzugt sind Verbindungen der Formel (I), in welcher
- A: für eine Gruppe der Formel 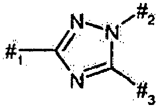 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zaht 1, 2 oder 3 steht,
- R¹: für Wasserstoff, C₁-C₆-Alkyl, C₃-C₆-Cycloalkyl, C₃-C₆-Cycloalkylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
- R⁴: für Wasserstoff, Chlor oder Fluor steht,
- R¹³: für eine Gruppe der Formel 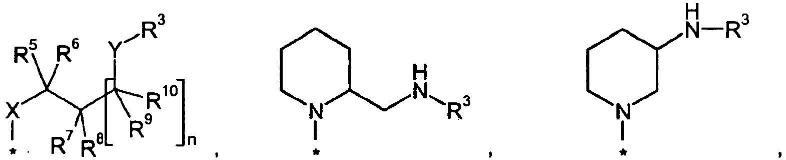 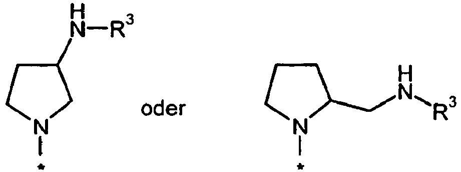 steht,
wobei
* die Anknüpfstelle an den Heterocyclus bedeutet,
n für die Zahl 0 oder 1 steht,
X für NR¹¹ oder O steht,
wobei
R¹¹ für Wasserstoff oder Methyl steht,
- Y: für NR¹² steht,
wobei
R¹² für Wasserstoff oder Methyl steht,
- R³: für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht,
wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
- R⁵: für Wasserstoff oder Methyl steht,
- R⁶: für Wasserstoff oder Methyl steht,
- R⁷: für Wasserstoff oder Methyl steht,
- R⁸: für Wasserstoff oder Methyl steht,
- R⁹: für Wasserstoff oder Methyl steht,
- R¹⁰: für Wasserstoff oder Methyl steht,
und ihre Salze, ihre Solvate und die Solvate ihrer Salze.

Bevorzugt sind auch Verbindungen der Formel (I), in welcher
- A: fur eine Gruppe der Formel 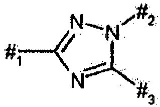 steht,
wobei
#₁ die Anknüpfstelle an den mit R¹ substituierten Heterocyclus bedeutet,
#₂, die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für Wasserstoff, C₁-C₆-Alkyl, C₃-C₆-Cycloalkyl, C₃-C₆-Cycloalkylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
- R⁴: für Wasserstoff oder Chlor steht,
- R¹³: für eine Gruppe der Formel 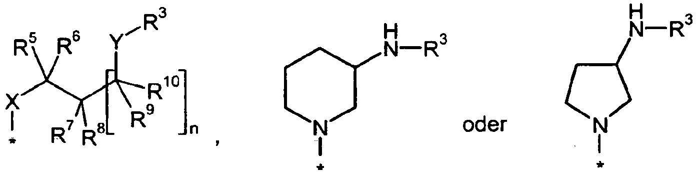 steht,
wobei
* die Anknüpfstelle an den Heterocyclus bedeutet,
n für die Zahl 0 steht,
X für NR¹¹ steht,
wobei
R¹¹ für Wasserstoff oder Methyl steht,
- Y: für NR¹² steht,
wobei
R¹² für Wasserstoff oder Methyl steht,
- R³: für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht,
wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkylamino, Alkylcarbonyl, Alkoxycarbonyl, Alkylanynocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
- R⁵: für Wasserstoff oder Methyl steht,
- R⁶: für Wasserstoff steht,
- R⁷: für Wasserstoff oder Methyl steht,
- R⁸: für Wasserstoff steht,
- R⁹: für Wasserstoff steht,
- R¹⁰: für Wasserstoff steht,
und ihre Salze, ihre Solvate und die Solvate ihrer Salze.

Bevorzugt sind auch Verbindungen der Formel (I), in welcher
- A: für eine Gruppe der Formel 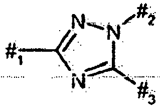 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die ZahL 1, 2 oder 3 steht,
- R¹: für Wasserstoff, C₁-C₆-Alkyl, C₃-C₆-Cycloalkyl, C₃-C₆-Cycloalkylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹³: für eine Gruppe der Formel 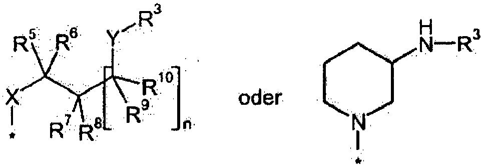 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl oder 1,3-Thiazol-2-yl steht,
wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht, und ihre Salze, ihre Solvate und die Solvate ihrer Salze.

Bevorzugt sind auch Verbindungen der Formel (I), in welcher
- A: für eine Gruppe der Formel 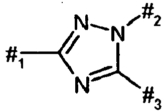 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für C₁-C₄-Alkyl, Cyclopropyl, Cyclopropylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹³: für eine Gruppe der Formel 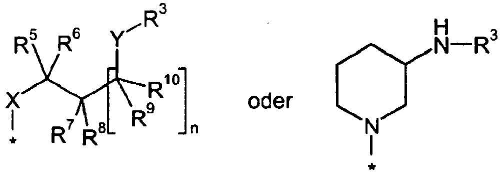 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl oder 1,3-Thiazol-2-yl steht,
wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für eine Gruppe der Formel 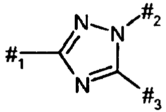 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für C₁-C₄-Alkyl, Cyclopropyl, Cyclopropylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl und Methyl,
- R⁴: für Wasserstoff steht,
- R¹³: für eine Gruppe der Formel 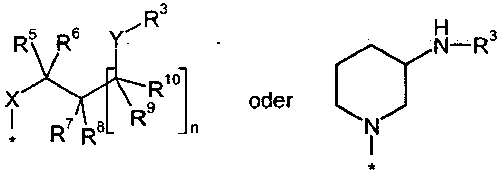 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff steht,
R⁶ für Wasserstoff steht,
R⁷ für Wasserstoff steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für eine Gruppe der Formel 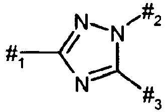 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für C₁-C₄-Alkyl, Cyclopropyl, Cyclopropylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl und Methyl,
- R⁴: für Wasserstoff steht,
- R¹³: für eine Gruppe der Formel 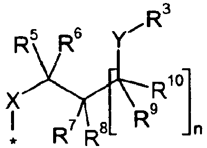 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff steht,
R⁶ für Wasserstoff steht,
R⁷ für Wasserstoff steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für eine Gruppe der Formel 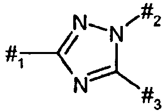 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
- m: für die Zahl 1, 2 oder 3 steht,
- R¹: für C₁-C₄-Alkyl, Cyclopropyl, Cyclopropylmethyl oder Hydroxycarbonylmethyl steht,
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl und Methyl,
- R⁴: für Wasserstoff steht,
- R¹³: für eine Gruppe der Formel 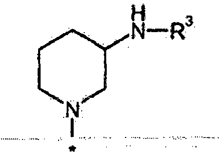 steht,
wobei
* die Anknüpfstelle an den Heterocyclus bedeutet,
R³ für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
und ihre Salte, ihre Solvate und die Solvate ihrer Salze.

Bevorzugt sind auch Verbindungen der Formel (I), in welcher
- A: für eine Gruppe der Formel 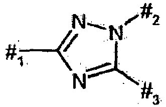 steht,
wobei
#₁ die Anknüpfstelle an den mit R¹ substituieren Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist.

Bevorzugt sind auch Verbindungen der Formel (I), in welche
- A: für eine Gruppe der Formel 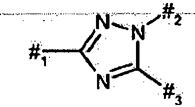 steht,
wobei
#₁ die Anknüpistelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist.

Bevorzugt sind auch Verbindungen der Formel (I), in welcher
- A: für eine Gruppe der Formel 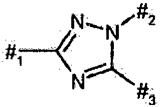 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist.

Bevorzugt sind auch Verbindungen der Formel (I), in welcher m für die Zahl 1, 2 oder 3 steht.

Bevorzugt sind auch Verbindungen der Formel (I), in welcher R¹ für C₁-C₄-Alkyl, Cyclopropyl, Cyclopropylmethyl oder Hydroxycarbonylmethyl steht.

Bevorzugt sind auch Verbindungen der Formel (I), in welcher R¹ für C₁-C₄-Alkyl, Cyclopropyl oder Cyclopropylmethyl steht.

Bevorzugt sind auch Verbindungen der Formel (I), in welcher
- R²: für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl und Methyl.

Bevorzugt sind auch Verbindungen der Formel (I), in welcher R⁴ für Wasserstoff steht.

Bevorzugt sind auch Verbindungen der Formel (I), in welcher n für die Zahl 0 steht.

Bevorzugt sind auch Verbindungen der Formel (I), in welcher X für NR¹¹ steht, wobei R¹¹ für Wasserstoff steht.

Bevorzugt sind auch Verbindungen der Formel (I), in welcher Y für NR¹² steht, wobei R¹² für Wasserstoff steht.

Bevorzugt sind auch Verbindungen der Formel (I), in welcher
- R³: für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl.

Bevorzugt sind auch Verbindungen der Formel (I), in welcher R⁵, R⁶, R⁷, R⁸, R⁹ und R¹⁰ für Wasserstoff stehen.

Bevorzugt sind auch Verbindungen der Formel (I), in welcher
- R¹³: für eine Gruppe der Formel 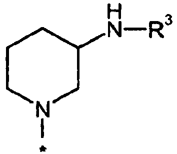 steht,
wobei
* die Anknüpfstelle an den Heterocyclus bedeutet,
R³ für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl.

Bevorzugt sind auch Verbindungen der Formel (I), in welcher
- R¹³: für eine Gruppe der Formel 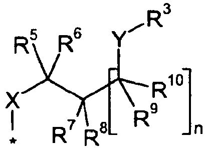 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl steht, wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff steht,
R⁶ für Wasserstoff steht,
R⁷ für Wasserstoff steht,
R⁸ für Wasserstoff steht,
R⁹ für Wasserstoff steht,
R¹⁰ für Wasserstoff steht.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 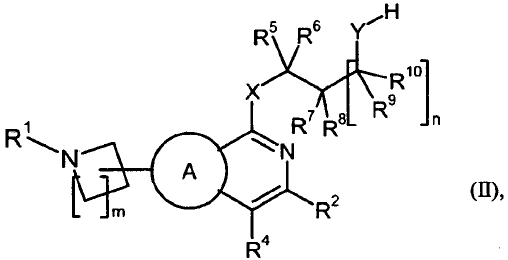 in welcher
   A, m, X, Y, R¹, R², R⁴, R⁵, R⁶, R⁷, R⁸, R⁹ und R¹⁰ die oben angegebene Bedeutung haben,
   mit Verbindungen der Formel

   R³-X¹ (III),

   in welcher
   R³ die oben angegebene Bedeutung hat, und
   X¹ für Halogen, bevorzugt Chlor oder Fluor, steht,
   umgesetzt werden,
   oder
[B] die Verbindungen der Formel 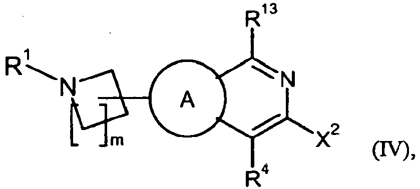 in welcher
   A, m, R¹, R⁴ und R¹³ die oben angegebene Bedeutung haben, und
   X² für Iod, Brom, Chlor oder Trifluormethansulfonyl, bevorzugt Iod oder Brom, steht,
   mit Verbindungen der Formel

   Q-R² (V),

   in welcher
   R² die oben angegebene Bedeutung hat, und
   Q für -B(OH)₂, einen Boronsäure-Ester, bevorzugt Boronsäurepinakolester, oder -BF₃⁻K⁺ steht,
   unter Suzuki-Kupplungsbedingungen umgesetzt werden,
   oder
[C] die Verbindungen der Formel 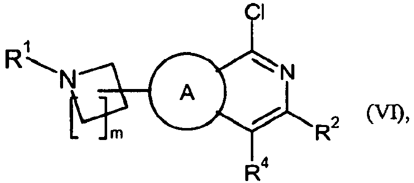 in welcher
   A, m, R¹, R² und R⁴ die oben angegebene Bedeutung haben,
   mit Verbindungen der Formel

   H-R¹³ (IX),

   in welcher
   R¹³ die oben angegebene Bedeutung hat,
   umgesetzt werden.

Die Umsetzung nach Verfahren [A] erfolgt im Allgemeinen in inerten Lösungsmitteln, gegebenenfalls in Gegenwart einer Base, gegebenenfalls in einer Mikrowelle, bevorzugt in einem Temperaturbereich von 50°C bis 200°C bei Normaldruck bis 3 bar.

Basen sind beispielsweise Alkalicarbonate, wie z.B. Natrium-, Kalium- oder Caesiumcarbonat, oder organische Basen wie Trialkylamine, z.B. Triethylamin, *N*-Methylmorpholin, *N*-Methyl-piperidin, 4-Dimethylaminopyridin oder Diisopropylethylamin, oder andere Basen wie beispielsweise Natriumhydrid oder Kalium-tert.-butylat, bevorzugt ist Diisopropylethylamin oder Natriumhydrid.

Inerte Lösungsmittel sind beispielsweise Halogenkohl nwasserstoffe wie Methylenchlorid oder Trichlormethan, Alkohole wie Methanol, Ethanol, n-Prupanol oder Isopropanol, oder Ether wie Dioxan oder Tetrahydrofuran, oder andere Lösungsmittel wie beispielsweise Dimethylsulfoxid, Dimethylformamid oder N-Methylpyrrolidon, oder Gemische dieser Lösungsmittel, bevorzugt ist Isopropanol oder Dimethylsulfoxid.

Die Umsetzung nach Verfahren [B] erfolgt im Allgemeinen in inerten Lösunesmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)(1,3-dimesityl-1,3-dihydro-2H-imidazol-2-yliden)palladium oder Palladium(II)acetat/Dicyclohexyl-(2',4',6'-triisopropyl-biphenyl-2-yl)-phosphin. Als Palladiumquelle kann auch Tris(dibenzylidenaceton)dipalladium eingesetzt werden.

Zusatzreagenzien sind beispielsweise Kaliumacetat, Cäsium-, Kalium- oder Natriumcarbonat, Kalium-tert.-butylat, Cäsiumfluorid oder Kaliumphosphat, bevorzugt sind Zusatzreagenzien wie z.B. Kaliumacetat und/oder wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gernische der Lösungsmittel mit Alkoholen wie Methanol oder Ethanol und/oder Wasser, bevorzugt ist Dioxan oder Acetonitril oder ein Gemisch aus einem dieser Lösungsmittel mit Wasser.

Die Umsetzung nach Verfahren [C] erfolgt nach den unter Verfahren [A] angegebenen Reaktionsbedingungen.

Die Verbindungen der Formeln (III), (V) und (IX) sind bekannt oder lassen sich nach bekannten Verfahren aus den entsprechenden Ausgangsverbindungen synthetisieren.

Die Verbindungen der Formel (VI) sind bekannt, lassen sich nach bekannten Verfahren aus den entsprechenden Ausgangsverbindungen synthetisieren oder können analog den im Beispielteil (Beispiel 30A bis 55A) beschriebenen Verfahren hergestellt werden.

Die Verbindungen der Formel (II) sind bekannt oder können hergestellt werden, indem Verbindungen der Formel 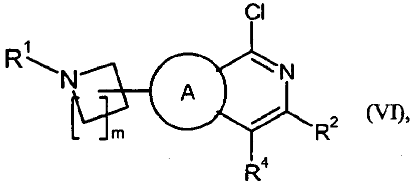 in welcher
A, m, R¹, R² und R⁴ die oben angegebene Bedeutung haben,
mit Verbindungen der Formel 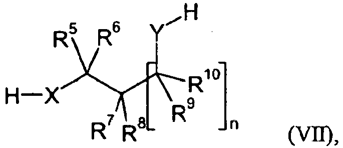 in welcher
n, X, Y, R⁵, R⁶, R⁷, R⁸, R⁹ und R¹⁰ die oben angegebene Bedeutung haben,
umgesetzt werden.

Die Umsetzung erfolgt nach den unter Verfahren [A] angegebenen Reaktionsbedingungen.

Der Rest Y ist während der Umsetzung gegebenenfalls mit einer dem Fachmann bekannten Schutzgruppe geschützt, die nach der Reaktion nach Standardverfahren abgespalten wird.

Die Verbindungen der Formel (VII) sind bekannt oder lassen sich nach bekannten Verfahren aus den entsprechenden Ausgangsverbindungen synthetisieren.

Die Verbindungen der Formel (IV) sind bekannt oder können hergestellt werden, indem Verbindungen der Formel 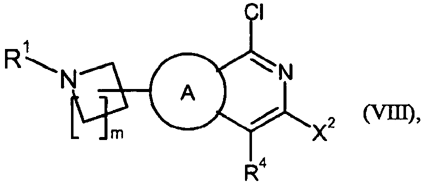 in welcher
A, m, R¹, R⁴ und X² die oben angegebene Bedeutung haben,
mit Verbindungen der Formel

H-R¹³ (IX),

in welcher
R¹³ die oben angegebene Bedeutung hat,
umgesetzt werden.

Die Umsetzung erfolgt nach den unter Verfahren [A] angegebenen Reaktionsbedingungen.

Die Verbindungen der Formel (VIII) sind bekannt, lassen sich nach bekannten Verfahren aus den entsprechenden Ausgangsverbindungen synthetisieren oder können analog den im Beispielteil (Beispie) 9A bis 11A und Beispiel 50A bis 53A) besohriebenen Verfahren hergestellt werden.

Die Herstellung der Ausgangsverbindungen und der Verbindungen der Formel (I) kann durch das folgende Syntheseschema verdeutlicht werden.

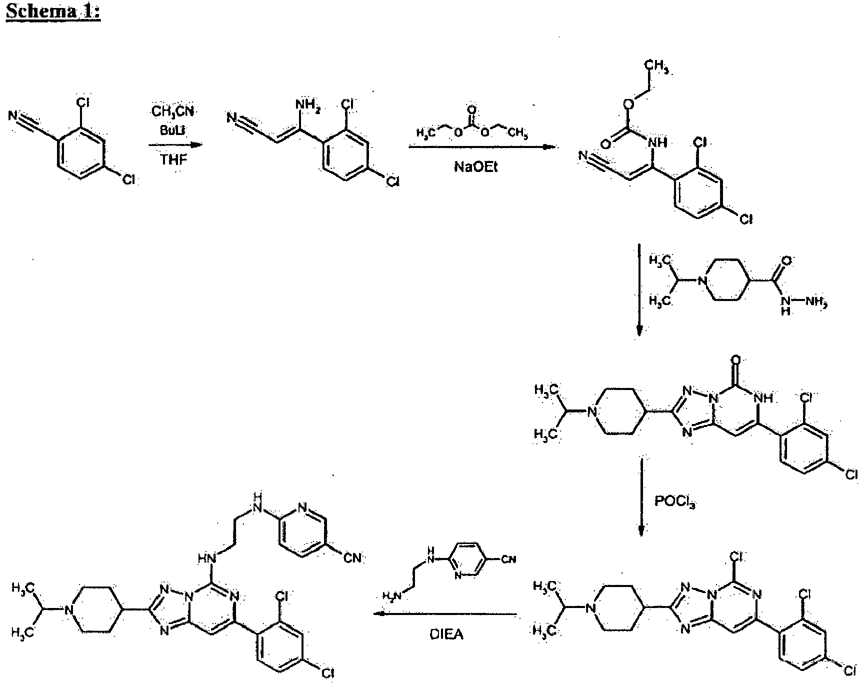

Die erfindungsgemäßen Verbindungen zeigen ein nicht vorhersehbare, wertvolles pharmakologisches und pharmakokinetisches Wirkspektrum.

Sie eigenen sich daher zur Verwendung als Arzneimittel zur Behandlung und/oder Prophylaxe von Krankheiten bei Menschen und Tieren.

Die erfindungsgemäßen Verbindungen eignen sich daher zur Prophylaxe und/oder Behandlung von neurodegenerativen Erkrankungen wie z.B. Alzbeimer, Parkinson, Schizophrenie, Degeneration, Dementia, Depressionen; Aggression, zerebrovaskulär Ischämie, Schlafstörungen, Huntington-Chorea, neurotraumatische Erkrankungen wie z.B. Schlaganfall; Typ 2 Diabetes Mellitus und assoziierte Erkrankungen wie z.B. das metabolische Syndrom oder Fettleibigkeit, Typ 1 Diabetes Mellitus, Diabetische Nephrophatie, Diabetische Neurophatie, Diabetische Retinophatie, Glomerulonephritis, Hyperkalzämie, Hyperglykämie, Hyperlipidimie, Glukose-Galaktose-Malabsorption, allgemeine endokrine Dysfunktionen wie z.B. Pankreatitis, hämatologische Erkrankungen, wie zum Beispiel erworbene und angeborene Neutropenie, medikamentös induzierte Neutropenie, parasitär induzierte Neutropenie, Chemotherapie-induzierte Neutropenie, Granulozytopenie, erworbene und angeborene Leukopenie, erworbene und angeborene Anämie, hämolytische Anämie, Sichelzellenanämie, erworbene und angeborenen Thrombozytopenie, Leukozytenfunktionssörungen, Störungen der Blutgerinnung, Graft-versus-host-Reaktion; Krebs, wie zum Beispiel Mammakarzinom, Kolontumor, gastrointestinale Tumore, Hodgkin-Lymphom, Non- Hodgkin-Lymphom, Kaposisarkom, Lebertumor, Pankreastumor, Hauttumor, Knochenmarkstumor, Leukämien wie z.B. akute lymphatische Leukämie, akute myeloische Leukämie, chronische myeloische Leukämie, chronische lymphatische Leukämie, MLL-Leukämie, Prostatatumore, Lungenkrebs, Nierentumore; Asthma, progrediente, nicht vollständig reversible Obstruktion der Atemwege, Lungenentzündung, Lungenfehlfunktion; Entzündungserkrankungen wie z.B. Autoimmunerkrankungen wie Multiple Sklerose, rheumatoide Arthritis, Infektionen durch gram-negative und gram-positive Bakterien, virale Infektionen, Pilzinfektionen wie z. B. durch Candida albicans, HIV- und HIV-assoziierte Infektionen, Hepatitis der Typen A, B und C, parasitäre Infektionen; Malaria; Haarausfall; verminderte Beweglichkeit von Spermien; Wundheilung; Glaukom; Osteoporose, Knochenmarkserkrankungen, Knochen- und Gelenkserkrankungen; Herzkreislauferkrankungen wie z.B. Herzfehler, Herzinsuffizienz, Herzfibrose, Herzrhythmusstörungen, Myokardinfarkt, Medikamenten- oder Substanzinduzierte Kardiotoxizität, Atherosklerose, Bluthochdruck; Sepsis; inflammatorische Erkrankungen; Pemphigus Vulgaris.

Die erfindungsgemäßen Verbindungen eignen sich besorders zur Prophylaxe und/oder Behandlung von neurodegenerativen Erkrankungen wie z.B. Alzheimer und Schizophrenie, von Typ 2 Diabetes Mellitus und assoziierten Erkrankungen, von Krebs, von Leukopenien und/oder von Neutropenien.

Die erfindungsgemäßen Verbindungen eignen sich besonders zur Prophylaxe und/oder Behandlung von Leukopenien und/oder von Neutropenien.

Die erfindungsgemäßen Verbindungen können darüber hinaus auch zur effizienten ex vivo Vermehrung von adulten hämatopoetischen Stammzellen aus dem Knochenmark und/oder aus peripherem Blut und/oder zur *ex vivo* Vermehrung von embryonalen Stammstellen aus Nabelschnurblut eingesetzt werden.

Die erfindungsgemäßen Verbindungen können darüber hinaus auch zur ex vivo Vermehrung embryonaler und/oder adulter Stammzellen sowie zur ex vivo Differenzierung embryonaler und/oder adulter Stammzellen verwendet werden.

Diese so expandierten Zellen können dann zur Verkürzung der durch myeloablative Therapien induzierten Zytopenien oder im Rahmen von therapeutischen Transplantationsverfahren oder bei hämatologischen Systemerkrankungen, wie z.B. Leukämien, oder mit nach der Expansion gentechnisch veränderter Zellen für Gentherapien verwendet werden.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:

Eine Kombination der erfindungsgemäßen Verbindungen mit in der Klinik verwendeten chemotherapeutischen Agenzien können bei unterschiedlichen Tumorerkrankungen zu einem signifikant verbesserten Behandlungserfolg führen. Bei den chemotherapeutischen Agenzien handelt es sich um Substanzen, welche entweder die Teilungsrate von Tumorzellen inhibieren und/oder die Neovaskularisierung von soliden Tumoren unterbindet. Dazu gehören u.a. Substanzen aus der Gruppe der Taxane wie z.B. Paclitaxel, oder Docetaxel, Substanzen, welche die Mitose von Tumorzellen inhibieren wie z.B. Vinblastin, Vincristin, Vindesin oder Vinorelbin. Substanzen aus der Klasse der Platinum-Derivate wie z.B. Cisplatin, Carboplatin, Oxaliplatin, Nedaplatin oder Lobaplatin. Weiterhin gehören zu den chemotherapeutischen Agentien Substanzen aus der Klasse der alkylierenden Agentien, wie z.B. Cyclophosphamid, Ifosfamid, Melphalan, Chlorambucil, Pipobroman, Triethylen-Melamin, Busulfan, Carmustin, Lomustin, Streptozin, Dacarbazin oder Temozolomid. Zu den chemotherapeutischen Agenzien werden auch Anti-Metabolite wie z.B. Folsäure-Antagonisten, Pyrimidin-Analoga, Purin-Analoga oder Adenosin-Desaminase-Inhibitoren gezählt. In diese Substanzklasse gehören u.a. Methotrexat, 5-Fluorouracil, Floxuridin, Cytarabin, Pentostatin und Gemcitabin. Als chemotherapeutische Agenzien werden auch Naturstoffe oder deren Derivate eingesetzt, zu denen u.a. Enzyme, Anti-Tumor-Antikörper und Lymphokine gezählt werden. Dazu gehören z.B. Bleomycin, Dactinomycin, Daunorubicin, Doxorubicin, Epirubicin, Idarubicin, ara-V, Paclitaxel, Mithramycin, Mitomycin-C, L-Asparaginase, Interferone (z.B. IFN-alhpa) und Etoposid. Andere chemotherapeutische Agentien mit anti-proliferativer und/oder antiangiogenetischer Wirkung sind Sorafenib, Sunitinib, Bortezomib, DAST Inhibitor (BAY 73-4506), ZK-Epothilon u.a.

Weiterer Gegenstand der vorliegenden Erfindung ist ein Verfahren zur ex vivo Vermehrung von adulten hämatopoetischen Stammzellen aus dem Knochenmark, aus peripherem Blut oder Nabelschnurblut, das dadurch gekennzeichnet ist, dass eine wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 1500 mg je 24 Stunden zur Erzielung wirksamer Ergebnisse zu verabreichen. Bei oraler Applikation beträgt die Menge etwa 5 bis 2000 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- abs.: absolut
- Boc: *tert*-Butoxycarbonyl
- CDCl₃: Deuterochloroform
- CO₂: Kohlendioxid
- d: Tag
- DIEA: *N,N-*Diisopropylethylamin
- DMAP: 4-*N,N-*Dimethylaminopyridin
- DMF: Dimethylformamid
- DMSO: Dimethylsulfoxid
- d. Th.: der Theorie
- EDC: *N*'-(3-Dimethylaminopropyl)-*N*-ethylcarbodiimid x HCl
- eq.: Äquivalent
- ESI: Elektrospray-Ionisation (bei MS)
- ges.: gesättigt
- h: Stunde
- HOBt: 1-Hydroxy-1H-benzotriazol x H₂O
- HPLC: Hochdruck-, Hochleistungsflüssigchromatographie
- konz.: konzentriert
- LC-MS: Flüssigchromatographie-gekoppelte Massenspektrometrie
- min.: Minuten
- MS: Massenspektrometrie
- MW: Molekulargewicht [g/mol]
- NMR: Kernresonanzspektroskopie
- PyBOP: 1-Benzotriazolyloxy-tripyrrolidinophosphoniumhexafluorophosphat
- R_{f}: Retentionsindex (bei DC)
- RP-HPLC: Reverse Phase HPLC
- RT: Raumtemperatur
- Rₜ: Retentionszeit (bei HPLC)
- TBTU: (Benzotriazol-1-yloxy)bisdimethylaminomethyliumfluoroborat
- TFA: Trifluoressigsäure
- THF: Tetrahydrofuran

### LC-MS Methoden:

Methode 1: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.
Methode 2: Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%ige Ameisensäure / 1; Eluent B: Acetonitril + 500 µl 50%ige Ameisensäure / 1; Gradient: 0.0 min 10%B → 7.0 min 95%B → 9.0 min 95%B; Ofen: 35 °C; Fluss: 0.0 min 1.0 ml/min→ 7.0 min 2.0 ml/min→ 9.0 min 2.0 ml/min; UV-Detektion: 210 nm
Methode 3: Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
Methode 4: Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.
Methode 5: Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.
Methode 6: Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min;; Ofen: 50°C; UV-Detektion: 210 nm.
Methode 7: Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.
Methode 8: Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A **→** 1.5 min 10%A → 2.2 min 10%A; Ofen: 50°C; Fluss: 0.33 ml/min; UV-Detektion: 210 nm.
Methode 9: Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 3.0 min 10% A → 4.0 min 10%A → 4.01 min 100%A → 5.00 min 100%A; Fluss: 0.0 min/3.0 min/4.0 min/4.01 min 2.5 ml/min, 5.00 min 2 ml/min; Ofen: 50°C; UV-Detektion: 210 nm.

### GC-MS Methoden:

Methode 10: Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Präparative HPLC:

Methode 11: Präparative HPLC: Säule: Reprosil C18; Gradient: Acetonitril/Wasser.

Als Mikrowellenreaktor wurde ein "single mode" Gerät vom Typ Emrys™ Optimizer verwendet.

### Ausgangsverbindungen

### Beispiel 1A

### tert-Butyl-{2-[(5-cyanopyridin-2-yl)amino]ethyl}carbamat

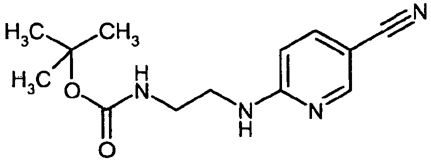

5.5 g (39.7 mmol) 6-Chlornicotinsäurenitril wurden in 70 ml DMSO gelöst und mit 10.2 g (63.5 mmol) N-Boc-Ethylendiamin und 11 g (79.4 mmol) Kaliumcarbonat versetzt. Es wurde 12 h bei 90°C nachgerührt. Der Rückstand wurde in einem Gemisch von Wasser und Essigsäureethylester aufgenommen. Die organische Phase wurde mit gesättigter wässriger Natriumchloridlösung gewaschen, über Magnesiumsulfat getrocknet und am Rotationsverdampfer eingeengt. Der Rückstand wurde an Kieselgel 60 chromatographiert (Laufmittel: Cyclohexan / Essigsäureethylester 10:1 bis 2:1). Es wurden 7.9 g (77% d. Th.) des Produktes als Feststoff erhalten.
LCMS (Methode 6): Rₜ = 1.46 min. (m/z = 263 (M+H)⁺)
¹H-NMR (400MHz, DMSO-d₆): δ = 8.37 (d, 1H), 7.66 (d, 1H) 7.6 (s, 1H), 6.87 (t, 1H), 6.53 (d, 1H), 3.32 (q, 2H), 3.09 (q, 2H), 1.37 (s, 9H).

### Beispiel 2A

### 6-[(2-Aminoethyl)amino]nicotinnitril Dihydrochlorid

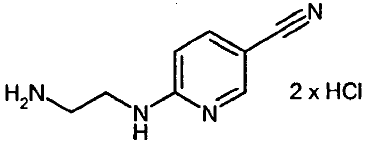

7.9 g (30 mmol) tert-Butyl-{2-[(5-eyanopyridin-2-yl)amino]ethyl}carbamat (Beispiel 1A) wurden in 100 ml 4N Chlorwasserstoff in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 7 g (94% d. Th.) des Produktes als Feststoff erhalten.
LCMS (Methode 4): Rₜ = 0.51 min. (m/z = 162 (M+H)⁺)
¹H-NMR (400MHz, DMSO-d₆): δ = 8.44 (s, 1H), 7.76 (d, 1H), 6.67 (d, 1H), 3.58 (t, 2H), 2.98 (q, 2H).

### Beispiel 3A

### tert-Butyl-(6-chlorpyridin-2-yl)carbamat

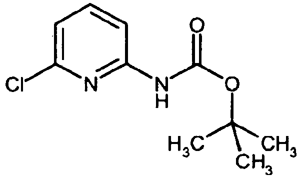

23.4 g (181.8 mmol) 2-Chlor-5-aminopyridin wurden unter Argon mit 150 ml THF versetzt und auf 0°C gekühlt. Es wurden 73.3 g (400 mmol) Bis-(trimethylsilyl)-natriumamid und 43.65 g (200 mmol) Di-tert-butyldicarbonat, gelöst in 150 ml THF, zugetropft. Nach 15 min wurde das Kühlbad entfernt und weitere 15 min bei RT nachgerührt. Das THF wurde abrotiert und der Rückstand mit Essigsäureethylester und 0.5N Salzsäure versetzt und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Dichlormethan/Methanol 100% → 100:3). Es wurden 36.54 g (88% d. Th.) des Produktes als Feststoff erhalten.
LCMS (Methode 3): Rₜ = 2.41 min. (m/z = 175 (M+H)⁺).
¹H-NMR (400MHz, DMSO-d₆): δ = 10.11 (s, 1H), 7.78 (d, 2H), 7.1 (t, 1H), 1.47 (s, 9H).

### Beispiel 4A

### tert-Butyl-(6-chlor-3-formylpyridin-2-yl)carbamat

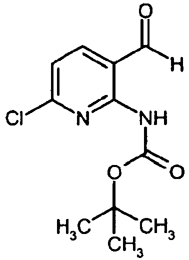

Die Reaktionsapparatur wurde ausgeheizt, und die Reaktion erfolgte unter Argon und wurde gerührt. 15 g (65.6 mmol) tert-Butyl-(6-chlorpyridin-2-yl)carbamat (Beispiel 3A) und 19 g (164 mmol) 1,2-Bis(dimethylamino)ethan wurden in 270 ml THF vorgelegt und auf -78°C abgekühlt. Es wurden 102.5 ml (164 mmol) Butyllithium (1.6N) zugetropft. Nach Beendigung des Zutropfens wurde die Reaktion langsam auf -10°C erwärmt und bei -10°C für 2 h gehalten. Dann wurde wieder auf -78°C abgekühlt, und es wurden 10 ml (131 mmol) DMF dazugegeben. Die Reaktion wurde langsam auf RT erwärmt und das Reaktionsgemisch wurde auf 1 1 Essigsäureethylester und 350 ml 1N Salzsäure gegeben, 15 min nachgerührt und die organische Phase wurde abgetrennt. Es wurde mit Wasser und gesättigter Natriumhydrogencarbonat-Lösung gewaschen, über Magnesiumsulfat getrocknet und am Rotationsverdampfer eingeengt. Der Rückstand wurde mit Diethylether versetzt und der Feststoff wurde abgesaugt und nachgetrocknet. Es wurden 12.3 g (73% d. Th.) des Produktes als Feststoff erhalten.
LCMS (Methode 3): Rₜ = 2.19 min. (m/z = 255 (M+H)⁻).
¹H-NMR (400MHz, DMSO-d₆): δ = 10.37 (s, 1H), 9.83 (s, 1H), 8.2 (d, 1H), 7.42 (d, 1H), 1.46 (s, 9H).

### Beispiel 5A

### tert-Butyl-{6-chlor-3-[(hydroxyimino)methyl]pyridin-2-yl}carbamat

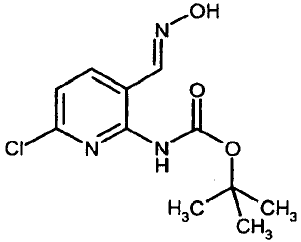

15.45 g (60.2 mmol) tert-Butyl-(6-chlor-3-formylpyridin-2-yl)carbamat (Beispiel 4A) wurde in 750 ml Ethanol vorgelegt und mit einer Lösung aus 225 ml Wasser und 9.38 g (120.4 mmol) Natriumacetat versetzt und 5 min gerührt. Eine Lösung aus 225 ml Wasser und 8.36 g (114.4 mmol) Hydroxylamin Hydrochlorid wurde zugegeben und 4 h bei RT gerührt. Bei 20°C wurde das Reaktionsgemisch am Rotationsverdampfer eingeengt. Der Rückstand wurde in Essigsäureethylester aufgenommen, zweimal mit gesättigter Natriumhydrogencarbonat-Lösung und einmal mit gesättiger Natriumchlorid-Lösung gewaschen. Die organische Phase wurde abgetrennt, über Magnesiumsulfat getrocknet und bei 20°C am Rotationsverdampfer eingeengt. Es wurden 15.5 g (80% d. Th.) des Produktes als Feststoff erhalten.
LCMS (Methode 3): Rₜ = 2.08 min. (m/z = 270 (M+H)⁻).
¹H-NMR (400MHz, DMSO-d₆): δ = 11.71 (s, 1H), 9.91 (s, 1H), 8.14 (s, 1H), 8.02 (d, 1H), 7.3 (d, 1H), 1.49 (s, 9H).

### Beispiel 6A

### 2-Amino-6-chlorpyridin-3-carbaldehydoxim Hydrochlorid

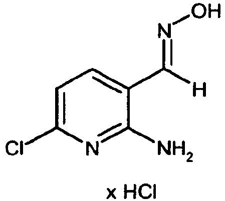

15.5 g (57 mol) tert-Butyl-{6-chlor-3-[(hydroxyimino)methyl]pyridin-2-yl}carbamat (Beispiel 5A) wurden in 285 ml 4N Chlorwasserstoff in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 11 g (94% d. Th.) des Produktes als Feststoff erhalten.
LCMS (Methode 6): Rₜ = 1.09 min. (m/z = 172 (M+H)⁺)
¹H-NMR (400MHz, DMSO-d₆): δ = 8.27 (s, 1H), 7.61 (d, 1H), 6.65 (d, 1H).

### Beispiel 7A

### 2-Amino-6-chlorpyridin-3-carbonitril

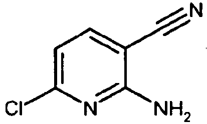

11.15 g (53.6 mmol) 2-Amino-6-chlorpyridin-3-carbaldehydoxim Hydrochlorid (Beispiel 6A) wurden in Dioxan vorgelegt, mit 13 ml (161 mmol) Pyridin versetzt und auf 0°C abgekühlt. Es wurden 8.3 ml (58.95 mmol) Trifluoressigsäureanhydrid zugegeben, man erwärmte die Reaktion auf RT und rührte anschließend 2 h bei 60°C. Das Reaktionsgemisch wurde in einem Gemisch von Essigsäureethylester und Natriumhydrogencarbonat-Lösung aufgenommen. Die organische Phase wurde mit gesättigter wässriger Natriumchlorid-Lösung gewaschen, über Magnesiumsulfat getrocknet und am Rotationsverdampfer eingeengt. Der Rückstand wurde in Dichlormethan:Diethylether 3:1 suspendiert, und der Feststoff wurde abgesaugt und getrocknet. Es wurden 5.56 g (66% d. Th.) des Produktes als Feststoff erhalten.
LCMS (Methode 6): Rₜ = 1.0 min. (m/z = 154 (M+H)⁺).
¹H-NMR (400MHz, DMSO-d₆): δ = 7.91 (d, 1H), 7.38 (s, 2H), 6.69 (d, 1H).

### Beispiel 8A

### tert-Butyl-{2-[(6-amino-5-cyanopyridin-2-yl)amino]ethyl}carbamat

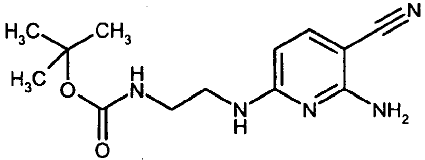

2 g (13 mmol) 2-Amino-6-chlorpyridin-3-carbonitril (Beispiel 7A) wurden in 15 ml DMSO vorgelegt und mit 2.71 g (16.93 mmol) N-Boc-Ethylenamin und 3.4 ml (19.54 mmol) N,N-Diisopropylethylamin versetzt. Das Reaktionsgemisch wurde 1.5 h bei 115°C in dem Mikrowellenreaktor bestrahlt. Das Reaktionsgemisch wurde in einem Gemisch von Essigsäureethylester und Wasser aufgenommen. Die organische Phase wurde mit gesättigter wässriger Natriumchlorid-Lösung gewaschen, über Magnesiumsulfat getrocknet und am Rotationsverdampfer eingeengt. Es wurden 23.38 g (88% d. Th.) des Produktes als Feststoff erhalten.
LCMS (Methode 3): Rₜ = 1.7 min. (m/z = 278 (M+H)⁺).
¹H-NMR (400MHz, DMSO-d₆): δ = 7.3 (s, 1H), 7.0 (br, s, 1H), 6.83 (s, 1H), 6.25 (s, 2H), 5.78 (d, 1H), 3.25 (q, 2H), 3.06 (q, 2H), 1.36 (s, 9H).

### Beispiel 9A

### 2-Amino-6-[(2-aminoethyl)amino]pyridin-3-carbonitril Dihydrochlorid

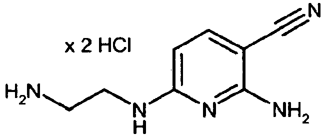

6.76 g (24.38 mmol) tert-Butyl-{2-[(6-amino-5-cyanopyridin-2-yl)amino]ethyl}carbamat (Beispiel 8A) wurden in 122 ml 4N Chlorwasserstofflösung in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 5.43 g (89% d. Th.) des Produktes als Feststoff erhalten.
LCMS (Methode 6): Rₜ = 0.92 min. (m/z = 177 (M+H)⁺)
¹H-NMR (400MHz, DMSO-d₆): δ = 8.1 (s, 2H), 7.5 (d, 1H), 5.96 (d, 1H), 3.53 (q, 2H), 3.0 (q, 2H).

### Beispiel 10A

### 4-(Trifluoracetyl)morpholin

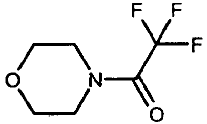

15 g (172 mmol) Morpholin wurden in 750 ml Dichlormethan vorgelegt, und es wurden bei 0°C 29 ml (206 mmol) Trifluoressigsäureanhydrid und 119 ml (688 mmol) N,N-Diisopropylethylamin zugegeben. Das Reaktionsgemisch wurde auf RT erwärmt und 3 h bei RT nachgerührt. Das Reaktionsgemisch wurde eingeengt und der Rückstand in Essigsäureethylester aufgenommen und nacheinander mit wässriger Natriumhydrogencarbonat-Lösung, 1N Salzsäure und wieder mit wässriger Natriumhydrogencarbonat-Lösung gewaschen. Die organische Phase wurde über Magnesiumsulfat getrocknet und am Rotationsverdampfer eingeengt. Es wurden 28 g (88% d. Th.) des Produktes als Öl erhalten.
LCMS (Methode 9): Rₜ = 1.22 min. (m/z = 184 (M+H)⁺)
¹H-NMR (400MHz, DMSO-d₆): δ = 3.65 (m, 2H), 3.56 (m, 2H).

### Beispiel 11A

### tert-Butyl-[6-chlor-3-(trifluoracetyl)pyridin-2-yl]carbamat

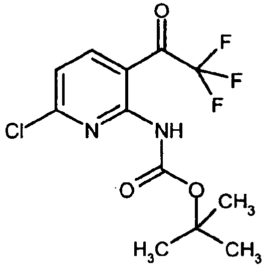

8 g (35 mmol) tert-Butyl-(6-chlorpyridin-2-yl)carbamat (Beispiel 3A) wurden in 100 ml THF vorgelegt und auf -50°C gekühlt. Es wurden 55 ml (87 mmol) Butyllithium (1.6N) zugetropft. Nach Beendigung des Zutropfens wurde die Reaktion langsam auf -10°C erwärmt und bei 0°C für 2 h gerührt. Anschließend wurde wieder auf -40°C abgekühlt, und es wurden 12.8 g (70 mmol) 4-(Trifluoracetyl)morpholin (Beispiel 10A), gelöst in 4 ml THF, zugegeben. Die Reaktionslösung wurde 1 h bei -40°C nachgerührt, danach bei -40°C auf 1 1 Essigsäureethylester und 350 ml Ammoniumchloridlösung gegossen und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Cyclohexan/ Essigsäureethylester 10:1). Es wurden 9 g (79% d. Th.) des Produktes als Öl erhalten.
¹H-NMR (400MHz, DMSO-d₆): δ = 10.96 (s, 1H), 7.99 (d, 1H), 7.4 (d, 1H), 1.43 (s, 9H).

### Beispiel 12A

### tert-Butyl-[6-({2-[(tert-butoxycarbonyl)amino]ethyl}amino)-3-(trifluoracetyl)pyridin-2-yl]carbamat

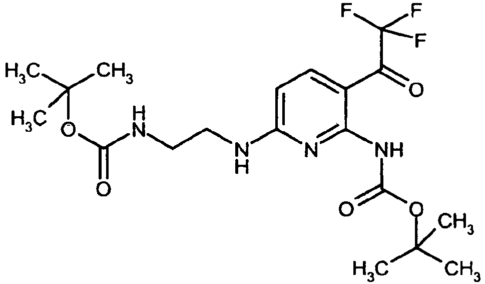

5 g (15.4 mmol) tert-Butyl-[6-chlor-3-(trifluoracetyl)pyridin-2-yl]carbamat (Beispiel 11A) wurden in 37.5 ml DMSO vorgelegt und mit 3.2 g (20 mmol) N-Boc-Ethylendiamin und 4 ml (23 mmol) N,N-Diisopropylethylamin versetzt. Das Reaktionsgemisch wurde 0.5 h bei 90°C in dem Mirkrowellenreaktor bestrahlt. Das Reaktionsgemisch wurde in einem Gemisch von Essigsäureethylester und Wasser aufgenommen. Die organische Phase wurde mit gesättigter wässriger Natriumchlorid-Lösung gewaschen, über Magnesiumsulfat getrocknet und am Rotationsverdampfer eingeengt. Man chromatographierte das Reaktionsgemisch an Kieselgel (Laufmittel Cyclohexan/Essigsäureethylester 5:1 → 1:1). Es wurden 2.5 g (34% d. Th.) des Produktes als Feststoff erhalten.
LCMS (Methode 6): Rₜ = 2.44 min. (m/z = 449 (M+H)⁺).
¹H-NMR (400MHz, DMSO-d₆): δ = 10.75 (s, 1H), 8.44 (s, 1H), 7.70 (d, 1H), 6.77 (s, 1H), 6.28 (d, 1H), 3.48 (br, s, 2H), 3.17 (br, s, 2H), 1.46 (s, 9H), 1.30 (s, 9H).

### Beispiel 13A

### 1-{2-Amino-6-[(2-aminoethyl)amino]pyridin-3-yl}-2,2,2-trifluorethanon Hydrochlorid

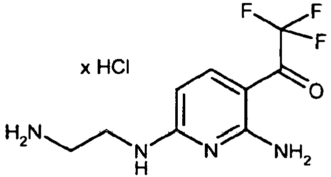

2.5 g (5.57 mmol) tert-Butyl-[6-({2-[(tert-butoxycarbonyl)amino]ethyl}amino)-3-(trifluoracetyl)-pyridin-2-yl]carbamat (Beispiel 12A) wurden in 15 ml 4N Chlorwasserstofflösung in Dioxan gelöst und 20 h nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 1.4 g (89% d. Th.) des Produktes als Feststoff erhalten.
LCMS (Methode 6): Rₜ = 0.73 min. (m/z = 249 (M+H)⁺).

### Beispiel 14A

### 4-Amino-2-(methylsulfonyl)-1,3-thiazol-5-carbonitril

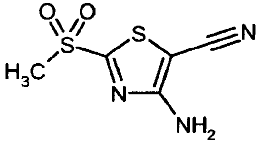

In 200 ml Dichlormethan wurden 2.7 g (15.77 mmol) 4-Amino-2-(methylthio)-1,3-thiazol-5-carbonitril gelöst und 11.97 g (34.7 mmol) 3-Chlorperbenzoesäure zugegeben. Man ließ für 30 min bei RT rühren, gab dann 6 ml DMSO zu und dann gesättigte wässrige Natriumhydrogencarbonat-Lösung und extrahiert dreimal mit Dichlormethan. Nach Trocknen der organischen Phase über Magnesiumsulfat erhielt man nach Entfernen des Lösungsmittels 2.22 g (46% d. Th.) des Produktes als Öl, das ohne weitere Aufreinigung eingesetzt wurde.
LCMS (Methode 3): Rₜ = 1.19 min. (m/z = 204 (M+H)⁺).

### Beispiel 15A

### tert-Butyl-{2-[(4-amino-5-cyano-1,3-thiazol-2-yl)amino]ethyl}carbamat

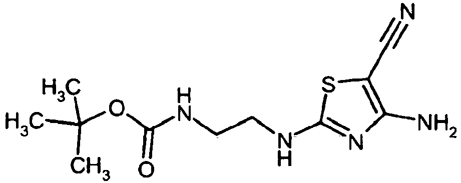

In 24 ml DMSO wurden 2.2 g (7.22 mmol) 4-Amino-2-(methylsulfonyl)-1,3-thiazol-5-carbonitril (Beispiel 14A) gelöst und 1.74 g (10.84 mmol) N-Boc-ethylendiamin und 933 mg (7.22 mmol) DIEA zugegeben. Man ließ für 16 h bei 120°C rühren und gab nach beendeter Reaktion Wasser und Essigsäureethylester zu. Man extrahierte die wässrige Phase dreimal mit Essigsäureethylester. Die vereinigten organischen Phasen wurden über Magnesiumsulfat getrocknet und mittels Kieselgelchromatographie gereinigt. Man erhielt 633 mg (31% d. Th.) des Produktes.
LCMS (Methode 6): Rₜ = 1.45 min. (m/z = 284 (M+H)⁺).
¹H-NMR (400MHz, DMSO-d₆): δ = 8.35 (s, br, 1H), 6.90 (t, 1H), 6.68 (s, 2H), 3.22 (s, br, 2H), 3.07 (dd, 2H), 1.37 (s, 9H).

### Beispiel 16A

### 4-Amino-2-[(2-aminoethyl)amino]-1,3-thiazol-5-carbonitril-trifluoracetat

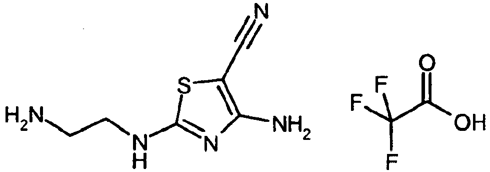

Analog der Herstellung des Cyanopyridins (Beispiel 2A) wurden aus 130 mg (0.46 mmol) des Boc-geschützten Amin (Beispiel 15A) und 1.05 g (9.18 mmol) Trifluoressigsäure nach Entfernen aller flüchtigen Bestandteile 130 mg (96% d. Th.) des Produktes erhalten.
LCMS (Methode 4): Rₜ = 0.61 min. (m/z = 184 (M+H)⁺).
¹H-NMR (400MHz, DMSO-d₆): δ = 8.45 (t, 1H), 7.84 (s, br, 2H), 6.80 (s, br, 1H), 3.93 (s, 1H), 3.43 (dd, 2H), 3.01 (dd, 2H).

### Beispiel 17A

### tert-Butyl 3-[(5-cyanopyridin-2-yl)amino]piperidin-1-carboxylat

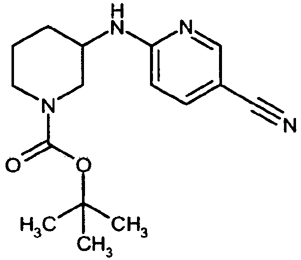

1.0 g (4.99 mmol) tert-Butyl-3-aminopiperidin-1-carboxylat und 1.383 g (9.99 mmol) 6-Chlorpyridin-3-carbonitril und 1.29 g (9.99 mmol) Diisopropylethylamin wurden in 40 ml DMSO suspendiert und für 45 min in einem Mikrowellenreaktor auf 140°C erhitzt. Der Ansatz wurde durch Kugelrohrdestillation weitgehend vom DMSO befreit, mit Wasser versetzt und der ausfallende Niederschlag abfiltriert. Nach Trocknen im Hochvakuum erhielt man 2.24 g (46% d. Th.) des Produktes.
LCMS (Methode 3): Rₜ = 2.23 min. (m/z = 303 (M+H)⁺).

### Beispiel 18A

### tert-Butyl 3-[(6-amino-5-cyanopyridin-2-yl)amino]piperidin-1-carboxylat

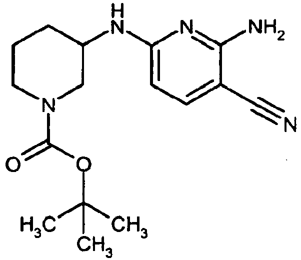

2.15 g (10.7 mmol) tert-Butyl-3-aminopiperidin-1-carboxylat, 1.50 g (9.77 mmol) 2-Amino-6-chlorpyridin-3-carbonitril (Beispiel 7A) und 1.89 g (14.7 mmol) Diisopropylethylamin wurden in 6 ml DMSO suspendiert und für 8 h in einem Mikrowellenreaktor auf 130°C erhitzt. Das Reaktionsgemisch wurde mit Ethylacetat (100 ml) und Wasser (40 ml) verdünnt, die organische Phase wurde getrennt und mit gesättigter wässriger Natriumchlorid-Lösung (50 ml) gewaschen, über Magnesiumsulfat getrocknet und eingeengt. Der Rückstand wurde chromatographiert auf Kieselgel (Laufmittel: Cyclohexan-Ethylacetat 4:1 bis 1:1). Es wurden 2.04 g (60% d. Th.) des Produktes als Feststoff isoliert.
LCMS (Methode 6): Rₜ = 1.69 min. (m/z = 318 (M+H)⁺)

### Beispiel 19A

### 6-(Piperidin-3-ylamino)pyridin-3-carbonitril Hydrochlorid

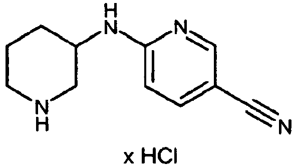

In 4.3 ml einer Salzsäurelösung in Dioxan (4 M) wurden 2.24 g (7.4 mmol) tert-Butyl-3-[(5-cyanopyridin-2-yl)amino]piperidin-1-carboxylat (Beispiel 17A) gelöst und 3 h bei RT gerührt. Nach vollständiger Reaktion wurde das Lösungsmittel komplett entfernt. Es wurden 1.74 g (90% d. Th.) des Produktes als Feststoff erhalten.
LCMS (Methode 8): R, = 0.27 min. (m/z = 203 (M+H)⁺)
¹H-NMR (400MHz, DMSO-d₆): δ = 9.13 (m, 1H), 9.0 (m, 1H), 8.44 (d, 1H), 7.89 (m, 1H), 7.74 (dd, 1H), 6.63 (d, 1H), 5.58 (s, br), 4.19 (s, br, 1H), 3.57 (s, 1H), 3.34 (d, 1H), 3.14 (d, 1H), 2.88 (m, 1H), 2.7-2.81 (m, 1H), 1.82-2.0 (m, 2H), 1.63-1.79 (m, 1H), 1.48-1.59 (m, 1H).

### Beispiel 20A

### 2-Amino-6-(piperidin-3-ylamino)pyridin-3-carbonitril Hydrochlorid

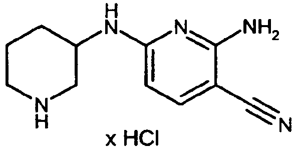

In 40 ml einer Salzsäurelösung in Dioxan (4 M) wurden 2.00 g (6.3 mmol) tert-Butyl 3-[(6-amino-5-cyanopyridin-2-yl)amino]piperidin-1-carboxylat (Beispiel 7A) gelöst und 2 h bei RT gerührt. Nach vollständiger Reaktion wurde das Lösungsmittel zur Hälfte eingeengt, und 20 ml Diethylether wurde zugegeben. Der Niederschlag wurde abfiltriert und getrocknet. Es wurden 1.80 g (100% d. Th.) des Produktes als Feststoff erhalten.
LCMS (Methode 8): Rₜ = 0.25 min. (m/z = 218 (M+H)⁺)
¹H-NMR (400MHz, DMSO-d₆): δ = 9.38 (br m, 1H), 8.97 (br m, 1H), 8.25 (br m, 1H), 7.53 (m, 1H), 7.40 (br s, 2H), 6.01 (d, 1H), 4.16 (br m, 1H), 3.34 (br m, 1H), 3.10 (m, 1H), 2.89 (m, 2H), 2.00-1.84 (m, 2H), 1.73 (m, 1H), 1.55 (m, 1H).

### Beispiel 21A

### tert-Butyl 3-({6-[(tert-butoxycarbonyl)amino]-5-(trifluoracetyl)pyridin-2-yl}amino)piperidin-1-carboxylat

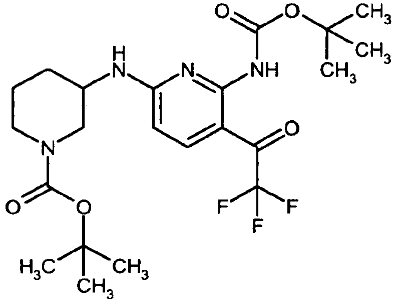

561 mg (2.8 mmol) tert-Butyl-3-aminopiperidin-1-carboxylat, 700 mg (2.16 mmol) tert-Butyl-[6-chlor-3-(trifluoracetyl)pyridin-2-yl]carbamat (Beispiel 11A) und 0.56 ml (3.23 mmol) Diisopropylethylamin wurden in 14 ml DMSO suspendiert und für 45 min in einem Mikrowellenreaktor auf 90°C erhitzt. Das Reaktionsgemisch wurde mit Ethylacetat (100 ml) verdünnt und mit gesättigter wässriger Ammoniumchloridlösung (3 x 40 ml), dann gesättigter wässriger Natriumhydrogencarbonatlösung (40 ml) gewaschen, und die organische Phase wurde getrocknet über Magnesiumsulfat verdünnt und eingeengt. Der Rückstand wurde chromatographiert auf Kieselgel (Laufmittel: Cyclohexan-Ethylacetat 5:1 bis 1:1). Es wurden 670 mg (63% d. Th.) des Produktes isoliert.
LCMS (Methode 6): Rₜ = 2.70 min. (m/z = 489 (M+H)⁺)

### Beispiel 22A

### 1-[2-Amino-6-(piperidin-3-ylamino)pyridin-3-yl]-2,2,2-trifluorethanon Hydrochlorid

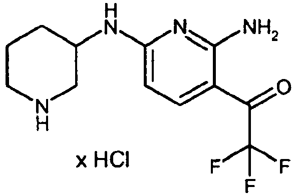

In 25 ml einer Salzsäurelösung in Dioxan (4 M) wurden 670 mg (1.37 mmol) tert-Butyl 3-({6-[(tert-butoxycarbonyl)amino]-5-(trifluoracetyl)pyridin-2-yl}amino)piperidin-1-carboxylat (Beispiel 21A) gelöst und 20 h bei RT gerührt. Nach vollständiger Reaktion wurde das Reaktionsmischung mit Diethylether (100 ml) verdünnt, und der Niederschlag wurde abfiltriert und mit Diethylether (100 ml) gewaschen und getrocknet. Es wurden 286 mg (64% d. Th.) des Produktes als Feststoff erhalten.
LCMS (Methode 6): Rₜ = 0.81 min. (m/z = 289 (M+H)⁺)
¹H-NMR (400MHz, DMSO-d₆): δ = 9.26 (br s, 1H), 9.07 (br s, 1H), 8.8.34 (br s, 1H), 7.59 (d, 1H), 6.22 (br, 2H), 6.03 (d, 1H), 4.25 (br m, 1H), 3.36 (m, 1H), 3.13 (m, 1H), 2.93 (m, 2H), 2.00-1.85 (m, 2H), 1.73 (m, 1H), 1.56 (m, 1H).

### Beispiel 23A

### tert-Butyl-(6-chlor-3-propanoylpyridin-2-yl)carbamat

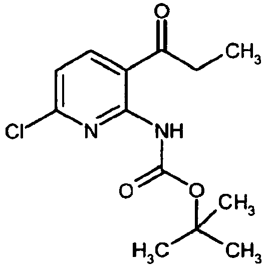

7.00 g (30.6 mmol) tert-Butyl-(6-chlorpyridin-2-yl)carbamat (Beispiel 3A) wurden in 90 ml THF unter Argon vorgelegt und auf -50°C gekühlt. Es wurden 47.8 ml (76.5 mmol) Butyllithium (1.6 M in Hexan) zugetropft. Nach Beendigung des Zutropfens wurde die Reaktion langsam auf 0°C erwärmt und bei 0°C für 1 h gehalten. Anschließend wurde wieder auf -40°C abgekühlt, und 9.85 g (61.2 mmol) N-Propionylmorpholin gelöst in 10 ml THF wurden zugegeben. Die Reaktionslösung wurde 1 h bei -40°C nachgerührt, danach bei -40°C auf 1 1 Essigsäureethylester und 350 ml Ammoniumchloridlösung gegossen, die organische Phase wurde abgetrennt und mit Wasser und gesättigter wässriger Natriumhydrogecarbonatlösung gewaschen. Die organische Phase wurde über Magnesiumsulfat getrocknet und am Roationsverdampfer eingeengt. Man chromatographierte das Rohprodukt auf Kieselgel (Laufmittel Cyclohexan/Essigsäureethylester 10:1 bis 1:1). Es wurden 2800 mg (32% d. Th.) des Produktes erhalten.
LCMS (Methode 6): Rₜ = 2.13 min. (m/z = 283 (M-H)⁻)
¹H-NMR (400MHz, DMSO-d₆): δ = 10.53 (br s, 1H), 8.19 (d, 1H), 7.31 (d, 1H), 2.94 (q, 2H), 1.45 (s, 9H), 1.06 (t, 3H).

### Beispiel 24A

### tert-Butyl-[6-({2-[(tert-butoxycarbonyl)amino]ethyl}amino)-3-propanoylpyridin-2-yl]carbamat

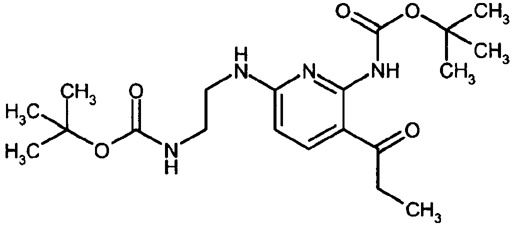

730 mg (2.4 mmol) tert-Butyl-(6-chlor-3-propanoylpyridin-2-yl)carbamat (Beispiel 23A) wurden in 7 ml DMSO vorgelegt und mit 512 mg (3.2 mmol) N-Boc-Ethylendiamin und 640 µl (3.7 mmol) N,N-Diisopropylethylamin versetzt. Das Reaktionsgemisch wurde 45 min bei 90°C in dem Mikrowellenreaktor bestrahlt. Das Reaktionsgemisch wurde in einem Gemisch von Essigsäureethylester und Wasser aufgenommen. Die organische Phase wurde mit gesättigter wässriger Ammoniumchlorid-Lösung und nachfolgend mit gesättigter wässriger Natriumhydrogencarbonatlösung gewaschen, über Magnesiumsulfat getrocknet und am Rotationsverdampfer eingeengt. Man chromatographierte das Reaktionsgemisch an Kieselgel (Laufmittel Cyclohexan/Essigsäureethylester 5:1 **→** 1:1). Es wurden 530 mg (53% d. Th.) des Produktes als Feststoff erhalten.
LCMS (Methode 6): Rₜ = 2.19 min. (m/z = 409 (M+H)⁺).
¹H-NMR (400MHz, DMSO-d₆): δ = 11.47 (s, 1H), 7.93 (br m, 1H), 7.64 (br m, 1H), 6.82 (br s, 1H), 6.15 (d, 1H), 3.43 (br m, 2H), 3.14 (m, 2H), 2.83 (q, 2H), 2.56 (br s, 4H), 1.47 (s, 9H), 1.32 (s, 9H), 1.03 (t, 3H).

### Beispiel 25A

### 1-{2-Amino-6-[(2-aminoethyl)amino]pyridin-3-yl}propan-1-on Hydrochlorid

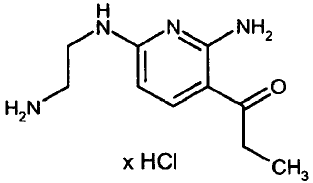

In 15 ml einer Salzsäurelösung in Dioxan (4 M) wurden 530 mg (1.30 mmol) tert-Butyl-[6-({2-[(tert-butoxycarbonyl)amino]ethyl}amino)-3-propanoylpyridin-2-yl]carbamat (Beispiel 24A) gelöst und 20 h bei RT gerührt. Nach vollständiger Reaktion wurde das Reaktionsmischung mit Diethylether (100 ml) verdünnt, und der Niederschlag wurde abfiltriert und mit Diethylether (100 ml) nachgewaschen und getrocknet. Es wurden 290 mg (91% d. Th.) des Produktes als Feststoff erhalten.
LCMS (Methode 6): Rₜ = 1.15 min. (m/z = 309 (M+H)⁺)

### Beispiel 26A

### tert-Butyl-3-({6-[(tert-butoxycarbonyl)amino]-5-propanoylpyridin-2-yl}amino)piperidin-1-carboxylat

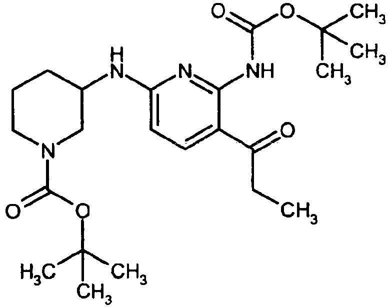

610 mg (3.0 mmol) tert-Butyl-3-aminopiperidin-1-carboxylat, 700 mg (2.3 mmol) tert-Butyl-(6-chlor-3-propanoylpyridin-2-yl)carbamat (Beispiel 23A) und 610 µl (3.5 mmol) Diisopropylethylamin wurden in 7 ml DMSO suspendiert und für 45 min in einem Mikrowellenreaktor auf 90°C erhitzt. Das Reaktionsgemisch wurde mit Ethylacetat (100 ml) verdünnt und mit gesättigter wässriger Ammoniumchloridlösung (3 x 40 ml), dann mit gesättigter wässriger Natriumhydrogencarbonatlösung (40 ml) gewaschen, und die organische Phase wurde getrocknet über Magnesiumsulfat, verdünnt und eingeengt. Der Rückstand wurde chromatographiert auf Kieselgel (Laufmittel: Cyclohexan-Ethylacetat 5:1 bis 1:1). Es wurden 380 mg (35% d. Th.) des Produktes isoliert.
LCMS (Methode 6): Rₜ = 2.42 min. (m/z = 449 (M+H)⁺)

### Beispiel 27A

### 1-[2-Amino-6-(piperidin-3-ylamino)pyridin-3-yl]propan-1-on Hydrochlorid

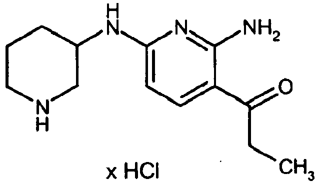

In 10 ml einer Salzsäurelösung in Dioxan (4 M) wurden 380 mg (0.85 mmol) tert-Butyl-3-({6-[(tert-Butoxycarbonyl)amino]-5-propanoylpyridin-2-yl}amino)piperidin-1-carboxylat (Beispiel 26A) gelöst und 20 h bei RT gerührt. Nach vollständiger Reaktion wurde das Reaktionsmischung mit Diethylether (100 ml) verdünnt, und der Niederschlag wurde abfiltriert und mit Diethylether (100 ml) gewaschen und getrocknet. Es wurden 170 mg (70% d. Th.) des Produktes als Feststoff erhalten.
LCMS (Methode 9): Rₜ = 0.95 min. (m/z = 249 (M+H)⁺)

### Beispiel 28A

### tert-Butyl-3-[(6-amino-5-nitropyridin-2-yl)amino]piperidin-1-carboxylat

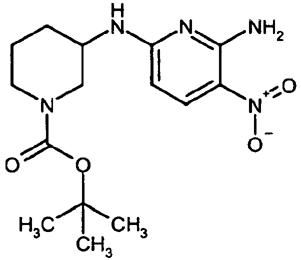

500 mg (2.11 mmol) tert-Butyl-3-aminopiperidin-1-carboxylat, 772 mg (4.22 mmol) 2-Amino-6-chlor-3-nitropyridin und 1.05 ml (6.34 mmol) Diisopropylethylamin wurden in 18 ml DMSO suspendiert und für 45 min in einem Mikrowellenreaktor auf 120°C erhitzt. Das Reaktionsgemisch wurde mittels präparativer RP-HPLC (Methode 11) aufgereinigt. Es wurden 600 mg (81% d. Th.) des Produktes als Feststoff isoliert.
LCMS (Methode 6): Rₜ = 1.77 min. (m/z = 338 (M+H)⁺)

### Beispiel 29A

### 3-Nitro-N⁶-(piperidin-3-yl)pyridin-2,6-diamin Hydrochlorid

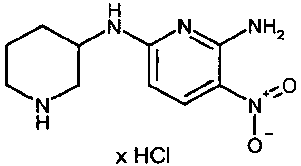

In 40 ml einer Salzsäurelösung in Dioxan (4 M) wurden 610 mg (1.62 mmol) tert-Butyl-3-[(6-amino-5-nitropyridin-2-yl)amino]piperidin-1-carboxylat (Beispiel 28A) gelöst und 30 min bei RT gerührt. Nach vollständiger Reaktion wurde das Lösungsmittel komplett entfernt. Es wurden 662 mg des Rohproduktes erhalten.
LCMS (Methode 4): Rₜ = 0.86 min. (m/z = 238 (M+H)⁺)

### Beispiel 30A

### 3-Amino-3-(2,4-dichlorphenyl)acrylnitril

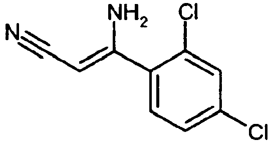

In einem Dreihalskolben mit mechanischem Rührer wurden unter Argon 90 g (889.46 mmol) Diisopropylamin in 1660 ml THF bei -70°C vorgelegt. 124.66 ml N-Butyllithium-Lösung (2.5 M in Hexan, 758.66 mmol) wurden so schnell zugetropft, dass die Temperatur nicht über -60°C steigt. Man ließ 10 min nachrühren und tropfte dann eine Lösung von 32.22 g (784.82 mmol) Acetonitril in 340 ml THF langsam zu und ließ die Suspension für 30 min rühren. Dann wurde eine Lösung von 90 g (523.21 mmol) 2,4-Dichlorbenzonitril in 340 ml THF zugetropft und 20 min bei -70°C nachgerührt. Man ließ langsam auf RT kommen und rührte für weitere 16 h bei RT. Es wurden 600 ml Wasser zugegeben, der größte Teil des THF abdestilliert und mit Wasser und Dichlormethan versetzt. Die organische Phase wurde mit gesättigter wässriger Natriumchloridlösung gewaschen. Nach Entfernen des Lösungsmittels erhielt man Kristalle, die durch Verrühren mit Diethylether gereinigt wurden. Es wurden 76.5 g (69% d. Th.) des Produktes als Feststoff erhalten.
LCMS (Methode 5): Rₜ = 3.07 min. (m/z = 213 (M+H)⁺)
¹H-NMR (400MHz, DMSO-d₆): δ = 7.70 (d, 1H), 7.47 (dd, 1H), 7.40 (d, 1H), 7.02 (s, breit, 2H), 3.79 (s, 1H).

### Beispiel 31A

### (2Z)-3-Amino-3-[4-(trifluormethyl)phenyl]prop-2-enonitril

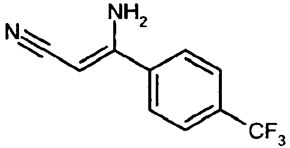

In einem Dreihalskolben mit mechanischem Rührer wurden unter Argon 28.1 g (278 mmol) Diisopropylamin in 450 ml THF bei -70°C vorgelegt. 148 ml N-Butyllithium-Lösung (1.6 M in Hexan, 237 mmol) wurden so schnell zugetropft, dass die Temperatur nicht über -60°C stieg. Man ließ 10 min nachrühren und tropfte dann eine Lösung von 12.9 ml (245 mmol) Acetonitril in 100 ml THF langsam zu und ließ die Suspension für 30 min rühren. Dann wurde eine Lösung von 28 g (164 mmol) 4-(Trifluormethyl)benzoesäurenitril in 100 ml THF zugetropft und 20 min bei -70°C nachgerührt. Man ließ langsam auf RT kommen und rührte für weitere 16 h bei RT. Es wurden 150 ml Wasser zugegeben, der größte Teil des THF abdestilliert und mit Wasser und Dichlormethan versetzt. Die organische Phase wurde mit gesättigter wässriger Natriumchloridlösung gewaschen. Nach Entfernen des Lösungsmittels erhielt man Kristalle, die durch Verrühren mit Diisopropylether (einmal 40 ml, zweimal 20 ml) gereinigt wurden. Die Kristalle wurden abfiltriert, mit Petrolether gewaschen und getrocknet. Es wurden 27 g (78% d. Th.) des Produktes als Feststoff erhalten.
LCMS (Methode 9): Rₜ = 2.05 min. (m/z = 213 (M+H)⁺)
¹H-NMR (400MHz, DMSO-d₆): δ = 7.79 (s, 4H), 7.98 (br s, 1H), 4.30 (s, 1H).

### Beispiel 32A

### Ethyl-[2-cyan-1-(2,4-dichlorphenyl)ethenyl]carbamat

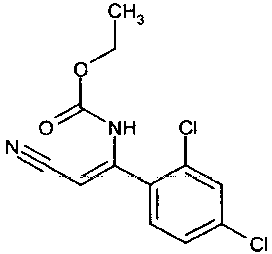

4.85 g (211 mmol) Natrium wurde in Ethanol (260 ml) gelöst, 30.0 g (141 mmol) 3-Amino-3-(2,4-dichlorphenyl)acrylnitril sowie 36.59 g (310 mmol) Diethylcarbonat wurden zugegeben, und die Reaktionslösung wurde 4 h unter Rückfluss gerührt. Das Reaktionsgemisch wurde mit Ethylacetat und Wasser versetzt, und der pH-Wert wurde auf pH = 5 mit konzentrierter Salzsäure eingestellt. Die organische Phase wurde abgetrennt, über Magnesiumsulfat getrocknet und einrotiert. Man chromatographierte den Rückstand an Kieselgel (Laufmittel Cyclohexan/Ethylacetat 7:1 bis 1:1), das Produkt hat einen R_{f} = 0.5 in Cyclohexan-Ethylaceat 1:1. Es wurden 17.1 g (43% d. Th.) des Produktes erhalten.
LCMS (Methode 6): Rₜ = 1.87 min. (m/z = 285 (M+H)⁺)
¹H-NMR (400MHz, DMSO-d₆): δ = 10.35 (s, 1H), 7.79 (d, 1H), 7.54 (m, 2H), 6.16 (s, 1H), 4.13 (q, 2H), 1.22 (t, 3H).

### Beispiel 33A

### Ethyl-{2-cyan-1-[4-(trifluormethyl)phenyl]ethenyl}carbamat

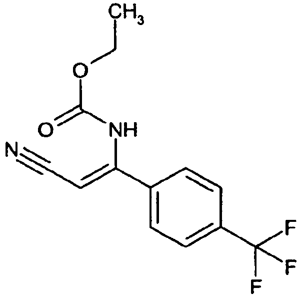

4.88 g (212 mmol) Natrium wurde in Ethanol (260 ml) gelöst, 30.0 g (141 mmol) 3-Amino-3-(2,4-dichlorphenyl)acrylnitril sowie 36.75 g (311 mmol) Diethylcarbonat wurden zugegeben, und die Reaktionslösung wurde 5 h unter Rückfluss gerührt. Das Reaktionsgemisch wurde mit Ethylacetat und Wasser versetzt, und der pH-Wert wurde auf pH = 5 mit 2M Salzsäure eingestellt. Die organische Phase wurde abgetrennt, über Magnesiumsulfat getrocknet und einrotiert. Man chromatographierte den Rückstand an Kieselgel (Laufmittel Cyclohexan/Ethylacetat 9:1 bis 2:1). Es wurden 13.3 g (33% d. Th.) des Produktes als ein Gemisch von E- und Z-Isomeren erhalten.
LCMS (Methode 6): Rₜ = 1.92 min. (m/z = 285 (M+H)⁺)

### Beispiel 34A

### (2Z)-3-Amino-3-(2-chlor-4-fluorphenyl)prop-2-enonitril

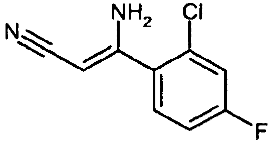

In einem Dreihalskolben mit mechanischem Rührer wurden unter Argon 35.3 g (273 mmol) Diisopropylamin in 450 ml THF bei -70°C vorgelegt. 145 ml N-Butyllithium-Lösung (1.6 M in Hexan, 237 mmol) wurden so schnell zugetropft, dass die Temperatur nicht über -60°C stieg. Man ließ 30 min nachrühren und tropfte dann eine Lösung von 12.7 ml (241 mmol) Acetonitril in 100 ml THF langsam zu und ließ die Suspension für 30 min rühren. Dann wurde eine Lösung von 25 g (160.7 mmol) 2-Chlor-4-fluorbenzonitril in 100 ml THF zugetropft und 20 min bei -70°C nachgerührt. Man ließ langsam auf RT kommen und rührte für weitere 16 h bei RT. Es wurden 150 ml Wasser zugegeben, der größte Teil des THF abdestilliert und mit Wasser und Dichlormethan versetzt. Die organische Phase wurde mit gesättigter wässriger Natriumchloridlösung gewaschen. Nach Entfernen des Lösungsmittels erhielt man ein Öl. Durch Verrühren mit Diisopropylether und anschließendes Absaugen konnte ein Feststoff erhalten werden. Nach Trocknen im Hochvakuum wurden 12.58 g (36% d. Th.) des Produktes erhalten. Dabei handelte es sich um ein Gemisch von E- und Z-Isomeren, das ohne weitere Reinigung eingesetzt wurde.
LCMS (Methode 8): Rₜ (Isomer 1) = 0.83 min. (m/z = 197 (M+H)⁺); Rₜ (Isomer 2) = 1.02 min. (m/z = 197 (M+H)⁺)
¹H-NMR (400MHz, DMSO-d₆): δ = 7.52 (dd, 1H), 7.44 (dd, 1H), 7.26 (m, 1H), 6.99 (s, br, 2H), 3.77 (s, 1H).

### Beispiel 35A

### (2Z)-3-Amino-3-(2,4-difluorphenyl)prop-2-enonitril

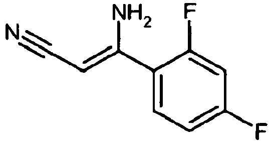

In einem Dreihalskolben mit mechanischem Rührer wurden unter Argon 13.3 g (132 mmol) Diisopropylamin in 350 ml THF bei -70°C vorgelegt. 70.5 ml N-Butyllithium-Lösung (1.6 M in Hexan, 112.8 mmol) wurden so schnell zugetropft, dass die Temperatur nicht über -60°C stieg. Man ließ 10 min nachrühren und tropfte dann eine Lösung von 6.14 ml (116.7 mmol) Acetonitril in 37 ml THF langsam zu und ließ die Suspension für 30 min rühren. Dann wurde eine Lösung von 11.04 g (77.8 mmol) 2,4-Difluorbenzonitril in 73 ml THF zugetropft und 90 min bei -70°C nachgerührt. Man ließ auf RT kommen. Es wurden 470 ml Wasser zugegeben, der größte Teil des THF abdestilliert und mit Wasser und Essigsäureethylester versetzt. Die organische Phase wurde zweimal mit je 150 ml Wasser und dann mit 110 ml gesättigter wässriger Natriumchloridlösung gewaschen. Nach Trocknen der organischen Phase mit Natriumsulfat und Entfernen des Lösungsmittels erhielt man einen Feststoff. Durch Verrühren mit Diisopropylether und anschließendes Absaugen konnte das Produkt erhalten werden. Nach Trocknen im Hochvakuum wurden 7.24 g (51% d. Th.) des Produktes erhalten. Dabei handelte es sich um ein Gemisch von E- und Z-Isomeren, das ohne weitere Reinigung eingesetzt wurde.
LCMS (Methode 6): Rₜ (Isomer 1) = 1.14 min. (m/z = 181 (M+H)⁺); Rₜ (Isomer 2) = 1.45 min. (m/z = 181 (M+H)⁺)
¹H-NMR (400MHz, DMSO-d₆): δ = 7.48-7.56 (m, 1H), 7.31-7.39 (m, 1H), 7.15 (dt, 1H), 6.94 (s, br, 2H), 3.95 (s, 1H).

### Beispiel 36A

### Ethyl-[(Z)-1-(2-chlor-4-fluorphenyl)-2-cyanoethenyl]carbamat

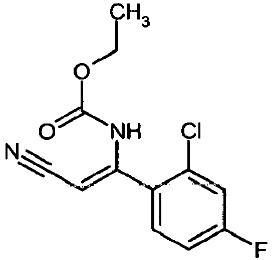

982 mg (42.7 mmol) Natrium wurde in Ethanol (53 ml) bei 40°C gelöst. 5.6 g (28.5 mmol) 3-Amino-3-(2-chlor-4-fluorphenyl)prop-2-enonitril sowie 7.4 g (62.7 mmol) Diethylcarbonat wurden zugegeben und die Reaktionslösung wurde 4 h unter Rückfluss gerührt. Das Reaktionsgemisch wurde mit 200 ml Ethylacetat und 150 ml Wasser versetzt und der pH-Wert mit Salzsäure auf pH = 5 eingestellt. Die organische Phase wurde abgetrennt, über Magnesiumsulfat getrocknet und einrotiert. Nach Entfernen des Lösungsmittels wurden 6.77 g (88% d. Th.) des Produktes als ein Gemisch von E- und Z-Isomeren erhalten.
LCMS (Methode 8): Rₜ (Isomer 1) = 0.99 min. (m/z = 269 (M+H)⁺); Rₜ (Isomer 2) = 1.10 min. (m/z = 269 (M+H)⁺)

### Beispiel 37A

### Ethyl-[(Z)-2-cyano-1-(2,4-difluorphenyl)ethenyl]carbamat

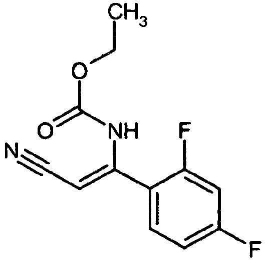

769 mg (33.44 mmol) Natrium wurde in Ethanol (41 ml) bei 40°C gelöst. 4.14 g (22.29 mmol) 3-Amino-3-(2,4-difluorphenyl)prop-2-enonitril sowie 5.79 g (49 mmol) Diethylcarbonat wurden zugegeben und die Reaktionslösung wurde 4 h unter Rückfluss gerührt. Das Reaktionsgemisch wurde mit 200 ml Ethylacetat und 150 ml Wasser versetzt und der pH-Wert mit Salzsäure auf pH = 5 eingestellt. Die organische Phase wurde abgetrennt, über Magnesiumsulfat getrocknet und einrotiert. Der Rückstand wurde mit Diisopropylether verrührt und der weiße Niederschlag abgesaugt. Weiteres Produkt konnte durch Trennung der Mutterlauge durch präparative HPLC (Methode 11) gewonnen werden. Zusammen wurden nach Trocknen im Hochvakuum 4.92 g (49% d. Th.) des Produktes als ein Gemisch von E- und Z-Isomeren erhalten, das ohne weitere Aufreinigung eingesetzt wurde.
LCMS (Methode 9): Rₜ (Isomer 1) = 1.66 min. (m/z = 253 (M+H)⁺); Rₜ (Isomer 2) = 1.87 min. (m/z = 253 (M+H)⁺)

### Beispiel 38A

### 1-(Propan-2-yl)piperidin-4-carbohydrazid

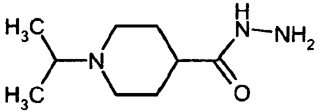

3.60 g (18.1 mmol) Ethyl-1-(1-methylethyl)piperidin-4-carboxylat und 1.00 g (19.9 mmol) Hydrazinhydrat wurden in 15 ml Ethanol vorlegt und 16 h bei Rückfluss nachgerührt. Das Reaktionsgemisch wurde auf RT abgekühlt und eingeengt, und der Rückstand wurde lyophilisiert. Das Rohprodukt wurde mit Diethylether (25 ml) nachgerührt, abfiltriert und getrocknet. Es wurden 1.91 g (57% d. Th.) des Produktes erhalten.
GC/MS (Methode 10): Rₜ = 5.68 min. (m/z = 185 (M⁺)
¹H-NMR (400MHz, DMSO-d₆): δ = 8.90 (s, 1H), 4.15 (br, 2H), 2.76 (br d, 2H), 2.64 (quintett, 1H), 2.06-1.95 (m, 3H), 1.63-1.47 (m, 4H), 0.94 (d, 6H).

### Beispiel 39A

### 7-(2,4-Dichlorphenyl)-2-[1-(1-methylethyl)piperidin-4-yl][1,2,4]triazolo[1,5-c]pyrimidin-5(6H)-on

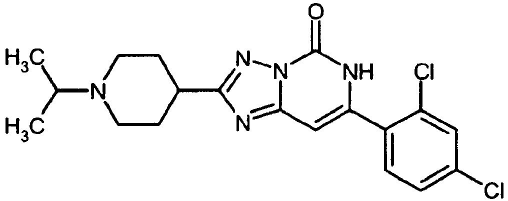

500 mg (1.70 mmol) Ethyl-[2-cyano-1-(2,4-dichlorphenyl)ethenyl]carbamat und 310 mg (1.70 mmol) 1-(1-Methylethyl)piperidin-4-carbohydrazid (Beispiel 38A) wurden in 2 ml 1-Methyl-2-pyrrolidon in einem Rundkolben ausgestattet mit Rückflusskondenser und einem mit Calciumchlorid gefüllten Trockenrohr gelöst, und das Reaktionsgemisch wurde 3 h bei 160°C gerührt. Die Reaktionslösung wurde auf 100°C abgekühlt und mit Wasser (20 ml) versetzt, danach wurde auf RT abgekühlt und 15 min nachgerührt. Der Reaktionsgemisch wurde mit Dichlormethan (3 x 50 ml) extrahiert, und die vereinigten organischen Phasen wurden über Magnesiumsulfat getrocknet und eingeengt. Das Rohprodukt wurde an basischem Aluminiumoxid chromatographiert (Laufmittel Dichlormethan-Methanol 10:1, dann Dichlormethan-Methanol-17%ige wässrige Ammoniak-Lösung 15:2:0.2). Es wurden 2.55 g (78% d. Th.) des Produktes erhalten.
LCMS (Methode 8): Rₜ = 0.75 min. (m/z = 406 (M+H)⁺)
¹H-NMR (400MHz, DMSO-d₆): δ = 12.38 (s, 1H), 10.42 (br s, 1H), 7.86 (s, 1H), 7.63 (s, 2H), 7.66 (d, 1H) 6.86 (s, 1H), 3.46 (m, 3H), 3.22-3.07 (m, 3H), 2.24 (m, 4H), 1.32 (d, 6H).

### Beispiel 40A

### 5-Chlor-7-(2,4-dichlorphenyl)-2-[1-(propan-2-yl)piperidin-4-yl][1,2,4]triazolo[1,5-c]pyrimidin

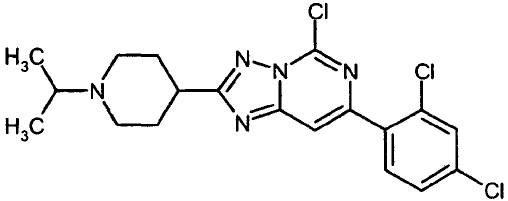

530 mg (1.30 mmol) 7-(2,4-Dichlorphenyl)-2-[1-(1-methylethyl)piperidin-4-yl][1,2,4]triazolo[1,5-c]pyrimidin-5(6H)-on wurde in Phosphoroxychlorid (16 ml) vorgelegt und 1.19 g (5.22 mmol) Benzyltriethylammoniumchlorid wurden zugegeben. Anschließend wurde 2.5 h bei 120°C nachgerührt. Das Reaktionsgemisch wurde auf gesättigte wässrige Natriumhydrogen-carbonatlösung (150 ml) gegossen, und so lange mit festem Natriumhydrogencarbonat versetzt, bis der pH-Wert 7 erreicht wurde. Es wurde mit Dichlormethan (3 x 50 ml) extrahiert, und die vereinigten organischen Phasen wurden mit Wasser (50 ml) gewaschen, über Magnesiumsulfat getrocknet und eingeengt. Es wurden 502 mg (89% d. Th.) des Produktes als Feststoff erhalten.
LCMS (Methode 6): Rₜ = 1.26 min. (m/z = 424 (M+H)⁺)
¹H-NMR (400MHz, DMSO-d₆): δ = 9.29 (br s, 1H), 8.22 (s, 1H), 7.85 (d, 1H), 7.73 (d, 1H), 7.65 (dd, 1H), 3.52 (m, 3H), 3.49 (m, 1H), 3.20 (m, 2H), 2.34 (m, 2H), 2.14 (m, 2H), 1.31 (d, 6H).

### Beispiel 41A

### tert-Butyl-4-(hydrazinylcarbonyl)piperidin-1-carboxylat

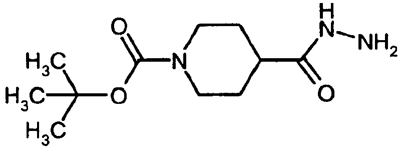

10.0 g (38.9 mmol) 1-tert-Butyl-4-ethyl-piperidin-1,4-dicarboxylat wurden in 35 ml Ethanol vorlegt und unter Rühren mit 3.8 ml (3.90 g, 78 mmol) Hydrazinhydrat versetzt. Es wurde 9 h bei Rückfluss nachgerührt. Das Reaktionsgemisch wurde auf RT abgekühlt, 1.9 ml (39 mmol) Hydrazinhydrat wurden zugegeben, und die Reaktionslösung wurde weitere 24 h bei Rückfluss nachgerührt. Das Lösemittel wurde eingeengt, Ethanol (50 ml) wurde zugegeben und es wurde wieder eingeengt. Diethylether (150 ml) wurde zugegeben, und das Gemisch wurde 5 min auf dem Ultraschallbad nachgerührt. Das Produkt wurde abfiltriert und getrocknet. Es wurden 9.20 g (97% d. Th.) des Produktes erhalten.
LCMS (Methode 6): Rₜ = 0.95 min. (m/z = 244 (M+H)⁺)
¹H-NMR (400MHz, DMSO-d₆): δ = 8.99 (s, 1H), 4.17 (br, 2H), 3.95 (br d, 2H), 2.71 (br, 2H), 2.23 (m, 1H), 1.60 (m, 2H), 1.40 (m, 11H).

### Beispiel 42A

### tert-Butyl-4-[7-(2,4-dichlorphenyl)-5-oxo-5,6-dihydro[1,2,4]triazolo[1,5-c]pyrimidin-2-yl]piperidin-1-carboxylat

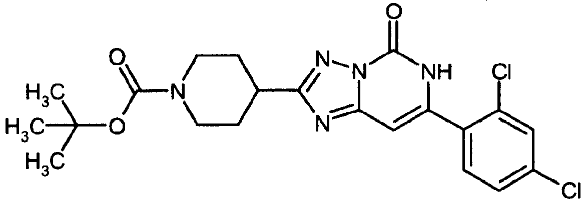

5.86 g (20.6 mmol) Ethyl-[2-cyano-1-(2,4-dichlorphenyl)ethenyl]carbamat und 5.00 g (20.6 mmol) tert-Butyl-4-(hydrazinylcarbonyl)piperidin-1-carboxylat wurden in 11 ml 1-Methyl-2-pyrrolidon in einem Rundkolben ausgestattet mit Rückflusskondenser und einem mit Calciumchlorid gefüllten Trockenrohr gelöst, und das Reaktionsgemisch wurde 3 h bei 150°C gerührt. Die Reaktionslösung wurde auf RT abgekühlt und mit Wasser (150 ml) versetzt, danach wurde 2 min auf dem Ultraschallbad nachgerührt. Der Feststoff wurde abgesaugt und im Hochvakuum getrocknet. Es wurden 7.23 g (76% d. Th.) des Produktes erhalten.
LCMS (Methode 3): Rₜ = 2.36 min. (m/z = 464 (M+H)⁺)
¹H-NMR (400MHz, DMSO-d₆): δ = 12.35 (br s, 1H), 7.84 (s, 1H), 7.61 (s, 2H), 6.82 (s, 1H), 4.96 (m, 2H), 3.05 (m, 1H), 2.96 (m, 2H), 1.98 (m, 2H), 1.64 (m, 2H), 1.43 (s, 9H).

### Beispiel 43A

### 7-(2,4-Dichlorphenyl)-2-(piperidin-4-yl)[1,2,4]triazolo[1,5-c]pyrimidin-5(6H)-on Hydrochlorid

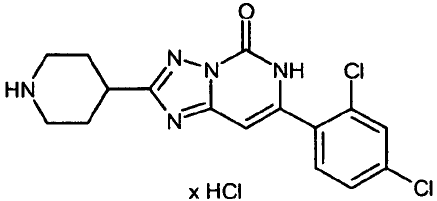

8.47 g (18.2 mmol) tert-Butyl-4-[7-(2,4-dichlorphenyl)-5-oxo-5,6-dihydro[1,2,4]triazolo[1,5-c]-pyrimidin-2-yl]piperidin-1-carboxylat (Beispiel 42A) und Chlorwasserstoff in Dioxan (4M, 90 ml) wurden 3 h bei RT gerührt, anschließend wurde das Lösemittel zur Hälfte eingeengt, Diethylether (50 ml) wurde zugegeben, und das Reaktionsgemisch wurde 2 min nachgerührt. Der Feststoff wurde abfiltriert und getrocknet. Es wurden 7.22 g (99% d. Th.) des Produktes erhalten.
LCMS (Methode 6): Rₜ = 0.80 min. (m/z = 364 (M+H)⁺)

### Beispiel 44A

### 2-(1-Cyclopropylpiperidin-4-yl)-7-(2,4-dichlorphenyl)[1,2,4]triazolo[1,5-c]pyrimidin-5(6H)-on

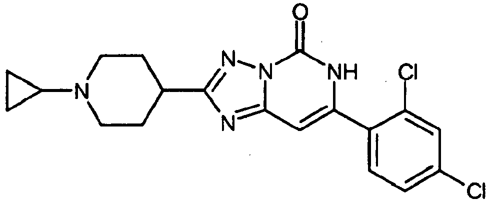

1.00 g (2.50 mmol) 7-(2,4-Dichlorphenyl)-2-(piperidin-4-yl)[1,2,4]triazolo[1,5-c]pyrimidin-5(6H)-on Hydrochlorid wurde zusammen mit 3Å Molekularsieb (1 g) in 15 ml trockenem Methanol vorgelegt und anschließend mit Essigsäure (1.43 ml, 25 mmol), 3.01 ml (2.61 g, 15 mmol) [(1-Ethoxy-1-cyclopropyl)-oxy]-trimethylsilan und 690 mg (11 mmol) Natriumcyanoborhydrid versetzt. Es wurde über Nacht bei Rückfluss gerührt. Es wurde auf RT abgekühlt und das Reaktionsgemisch wurde filtriert. Das Filtrat wurde mit 50 ml Dichlormethan und 50 ml Wasser versetzt und extrahiert. Die organische Phase wurde über Magnesiumsulfat getrocknet und einrotiert. Es wurden 540 mg (53% d. Th.) des Produktes erhalten.
LCMS (Methode 6): Rₜ = 0.87 min. (m/z = 404 (M+H)⁺)
¹H-NMR (400MHz, DMSO-d₆): δ = 12.0 (br, 1H), 7.85 (s, 1H), 7.63 (s, 3H), 6.86 (s, 1H), 6.66 (s, 1H), 3.37 (m, 3H), 3.10 (m, 2H), 2.18 (m, 2H), 2.03 (m, 1H), 1.91 (m, 1H), 0.86 (m, 1H), 0.76 (m, 2H), 0.69 (m, 2H).

### Beispiel 45A

### 5-Chlor-2-(1-cyclopropylpiperidin-4-yl)-7-(2,4-dichlorphenyl)[1,2,4]triazolo[1,5-c]pyrimidin

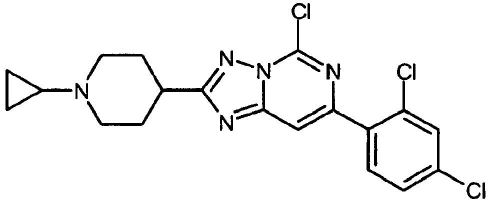

450 mg (1.11 mmol) 2-(1-Cyclopropylpiperidin-4-yl)-7-(2,4-dichlorphenyl)[1,2,4]triazolo[1,5-c]pyrimidin-5(6H)-on (Beispiel 44A) wurde in Phosphoroxychlorid (10 ml) vorgelegt und 1.01 g (4.45 mmol) Benzyltriethylammoniumchlorid wurden zu gegeben, und es wurde anschließend 2.5 h bei 120°C nachgerührt. Das Reaktionsgemisch wurde auf gesättigte wässrige Natriumhydrogen-carbonatlösung (150 ml) gegossen, und so lange mit festem Natriumhydrogencarbonat versetzt, bis der pH-Wert 7 erreicht wurde. Es wurde mit Dichlormethan (3 x 50 ml) extrahiert, und die vereinigten organischen Phasen wurden mit Wasser (50 ml) gewaschen, über Magnesiumsulfat getrocknet und eingeengt. Das Reaktionsgemisch wurde in Acetonitril aufgenommen und mittels präparativer HPLC (Methode 11) gereinigt. Nach Lyophilisierung wurden 238 mg (50% d. Th.) des Produktes erhalten.
LCMS (Methode 8): Rₜ = 1.00 min. (m/z = 422 (M+H)⁺)
¹H-NMR (400MHz, DMSO-d₆): δ = 10.05 (br, 1H), 8.21 (s, 1H), 7.86 (d, 1H), 7.73 (d, 1H), 7.65 (dd, 1H), 3.64 (m, 2H), 2.83 (m, 1H), 2.33 (m, 2H), 2.17 (m, 2H), 1.12 (m, 2H), 0.84 (m, 2H). 3H sind unter dem Wassersignal verborgen.

### Beispiel 46A

### 2-[1-(Cyclopropylmethyl)piperidin-4-yl]-7-(2,4-dichlorphenyl)[1,2,4]triazolo[1,5-c]pyrimidin-5(6H)-on Hydrochlorid

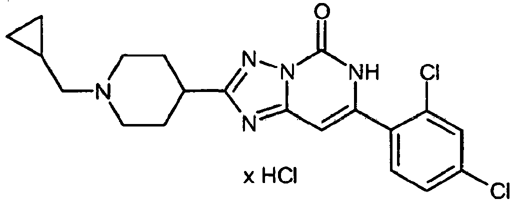

1.00 g (2.50 mmol) 7-(2,4-Dichlorphenyl)-2-(piperidin-4-yl)[1,2,4]triazolo[1,5-c]pyrimidin-5(6H)-on Hydrochlorid wurde in DMF (6 ml) gelöst und 268 µl (2.75 mmol) 1-Brommethylcyclopropan und 759 mg (5.49 mmol) Kaliumcarbonat wurden zugegeben. Das Reaktionsgemisch wurde 16 h bei 80°C gerührt, anschliessend wurde die Lösung filtriert und mit Ethylacetat (100 ml) und Wasser (75 ml) versetzt. Die Phasen wurden getrennt, und die wässrige Phase wurde mit Ethylacetat (zweimal 15 ml) extrahiert. Die vereinigten organischen Phasen wurden mit gesättigter wässriger Natriumchloridlösung gewaschen, getrocknet (Magnesiumsulfat) und eingeengt. Das Produkt wurde in Acetonitril aufgenommen und mittels präparativer RP-HPLC (Methode 11) gereinigt. Es wurden 413 mg (39% d. Th.) des Produktes erhalten.
LCMS (Methode 9): Rₜ = 1.25 min. (m/z = 364 (M-HCl+H)⁺)

### Beispiel 47A

### 5-Chlor-2-[1-(cyclopropylmethyl)piperidin-4-yl]-7-(2,4-dichlorphenyl)[1,2,4]triazolo[1,5-c]pyrimidin

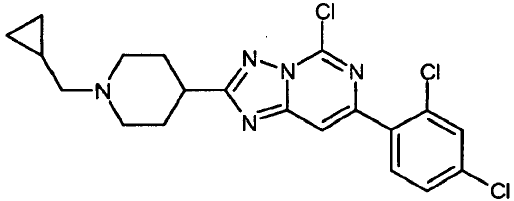

356 mg (0.85 mmol) 2-[1-(Cyclopropylmethyl)piperidin-4-yl]-7-(2,4-dichlorphenyl)[1,2,4]-triazolo[1,5-c]pyrimidin-5(6H)-on Hydrochlorid (Beispiel 46A) wurde in Phosphoroxychlorid (6 ml) vorgelegt und 775 mg (3.40 mmol) Benzyltriethylammoniumchlorid wurden zugegeben, und es wurde anschließend 2.5 h bei 120°C nachgerührt. Das Reaktionsgemisch wurde auf gesättigte wässrige Natriumhydrogencarbonatlösung (150 ml) gegossen, und so lange mit festem Natriumhydrogencarbonat versetzt, bis der pH-Wert 7 erreicht wurde. Es wurde mit Dichlormethan (3 x 50 ml) extrahiert, und die vereinigten organischen Phasen wurden mit Wasser (50 ml) gewaschen, über Magnesiumsulfat getrocknet und eingeengt. Es wurden 307 mg (83% d. Th.) des Produktes als Feststoff erhalten.
LCMS (Methode 6): Rₜ = 1.30 min. (m/z = 436 (M+H)⁺)
¹H-NMR (400MHz, DMSO-d₆): δ = 8.18 (s, 1H), 7.85 (d, 1H), 7.72 (d, 1H), 7.64 (dd, 1H), 3.47 (m, 1H), 3.06 (m, 2H), 2.355-2.0 (m, 6H), 0.87 (m, 2H), 0.49 (m, 2H), 0.40 (m, 1H), 0.13 (m, 2H),

### Beispiel 48A

### 7-(2,4-Dichlorphenyl)-2-(1-methylpiperidin-4-yl)[1,2,4]triazolo[1,5-c]pyrimidin-5(6H)-on

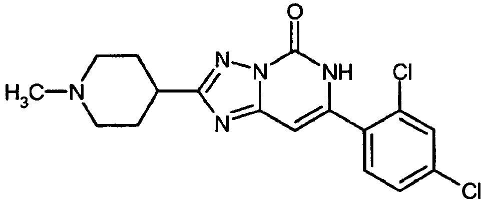

500 mg (1.25 mmol) 7-(2,4-Dichlorphenyl)-2-(piperidin-4-yl)[1,2,4]triazolo[1,5-c]pyrimidin-5(6H)-on Hydrochlorid wurde in Methanol (4 ml) bei 0°C vorgelegt, 3Å Molekularsieb (50 mg), Formalin (210 µl, 7.50 mmol) und Natriumcyanoborhydrid (470 mg, 7.50 mmol) wurden zugegeben. Der Ansatz wurde 1 h bei 0°C gerührt, und anschliessend 12 h bei RT nachgerührt. Das Reaktionsgemisch wurde filtriert, und das Filtrat wurde mit Dichlormethan (100 ml) und Wasser (75 ml) versetzt. Es wurde extrahiert, und die organische Phase wurde getrocknet (Magnesiumsulfat) und eingeengt. Es wurden 274 mg (58% d. Th.) des Rohproduktes erhalten.
LCMS (Methode 8): Rₜ = 0.74 min. (m/z = 378 (M+H)⁺)

### Beispiel 49A

### 5-Chlor-7-(2,4-dichlorphenyl)-2-(1-methylpiperidin-4-yl)[1,2,4]triazolo[1,5-c]pyrimidin

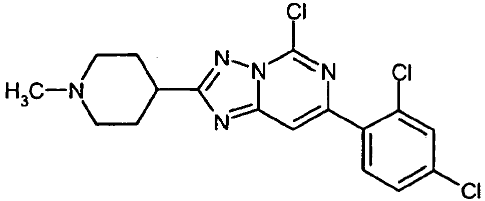

270 mg (0.71 mmol) 7-(2,4-Dichlorphenyl)-2-(1-methylpiperidin-4-yl)[1,2,4]triazolo[1,5-c]pyrimidm-5(6H)-on (Beispiel 48A) wurde in Phosphoroxychlorid (2 ml) vorgelegt, 650 mg (2.90 mmol) Benzyltriethylammoniumchlorid wurden zugegeben, und es wurde anschließend 3 h bei 120°C nachgerührt. Das Reaktionsgemisch wurde auf gesättigte wässrige Natriumhydrogencarbonatlösung (150 ml) gegossen, und so lange mit festem Natriumhydrogen-carbonat versetzt, bis der pH-Wert 7 erreicht wurde. Der Feststoff wurde abgesaugt und getrocknet. Es wurden 190 mg (65% d. Th.) des Produktes als Feststoff erhalten.
LCMS (Methode 8): Rₜ = 0.98 min. (m/z = 396 (M+H)⁺)
¹H-NMR (400MHz, DMSO-d₆): δ = 10.47 (br, 1H), 8.21 (s, 1H), 7.84 (d, 1H), 7.72 (d, 1H), 7.64 (dd, 1H), 7.53 (s, 1H), 3.53 (m, 2H), 3.30-3.10 (m, 2H), 2.79 (d, 3H), 2.67 (m, 1H), 2.32 (m, 3H), 2.14 (m, 2H).

### Beispiel 50A

### 7-(2-Chlor-4-fluorphenyl)-2-[1-(propan-2-yl)piperidin-4-yl][1,2,4]triazolo[1,5-c]pyrimidin-5(6H)-on

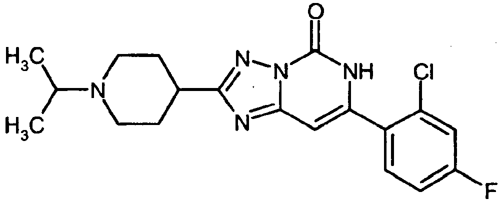

In 27 ml N-Methylpyrrolidon wurden 1.5 g (5.14 mmol) Ethyl-[(Z)-1-(2-chlor-4-fluorphenyl)-2-cyanoethenyl]-carbamat (Beispiel 36A) und 952 mg (5.14 mmol) 1-(Propan-2-yl)piperidin-4-carbohydrazid (Beispiel 38A) gelöst und unter Argon 3 h bei einer Ölbadtemperatur von 160°C in einem Kolben mit aufgesetztem Calciumchlorid-Trockenrohr gerührt. Das Reaktionsgemisch wurde auf RT abgekühlt, mit Wasser (50 ml) versetzt und 15 min nachgerührt. Der ausgefallene Niederschlag wurde abfiltriert, mit wenig Wasser gewaschen und anschließend im Hochvakuum getrocknet. Es wurden 1.25 g (61% d. Th.) des Produktes erhalten.
LCMS (Methode 8): Rₜ = 0.68 min. (m/z = 390 (M+H)⁺)
¹H-NMR (400MHz, DMSO-d₆): δ = 7.59-7.68 (m, 2H), 7.37 (dt, 1H), 6.70 (s, 1H), 3.30 (s, br, 1H), 2.9-3.1 (m, 2H), 2.77-2.9 (m, 2H), 2.35-2.48 (m, 2H), 2.04 (d, 2H), 1.79 (dd, 2H), 1.04 (d, 6H).

### Beispiel 51A

### 7-(2,4-Difluorphenyl)-2-[1-(propan-2-yl)piperidin-4-yl][1,2,4]triazolo[1,5-c]pyrimidin-5(6H)-on

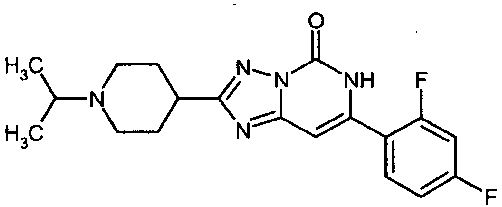

In 24 ml N-Methylpyrrolidon wurden 2.0 g (4.12 mmol) Ethyl-[(Z)-2-cyano-1-(2,4-difluorphenyl)ethenyl]carbamat (Beispiel 37A) und 916 mg (4.95 mmol) 1-(Propan-2-yl)piperidin-4-carbohydrazid (Beispiel 38A) gelöst und unter Argon 6 h bei einer Ölbadtemperatur von 160°C in einem Kolben mit aufgesetztem Calciumchlorid-Trockenrohr gerührt. Das Reaktionsgemisch wurde auf RT abgekühlt, mit Wasser (50 ml) versetzt und 15 min nachgerührt. Es wurde mit Essigsäureethylester extrahiert (dreimal mit je 50 ml) und die vereinigten organischen Phasen vom Lösungsmittel befreit. Der Rückstand wurde mittels präparativer HPLC (Methode 11) gereinigt. Es wurden 660 mg (41% d. Th.) des Produktes erhalten.
LCMS (Methode 6): Rₜ = 0.63 min. (m/z = 374 (M+H)⁺)
¹H-NMR (400MHz, DMSO-d₆): δ = 7.83 (dd, 1H), 7.41 (dt, 1H), 7.23 (dt, 1H), 6.80 (s, 1H), 3.32 (m, 1H), 2.93-3.1 (m, 3H), 2.81-2.93 (m, 1H), 2.53-2.62 (m, 2H), 2.07 (d, 2H), 1.84 (dd, 2H), 1.08 (d, 6H).

### Beispiel 52A

### 5-Chlor-7-(2-chlor-4-fluorphenyl)-2-[1-(propan-2-yl)piperidin-4-yl][1,2,4]triazolo[1,5-c]pyrimidin Hydrochlorid

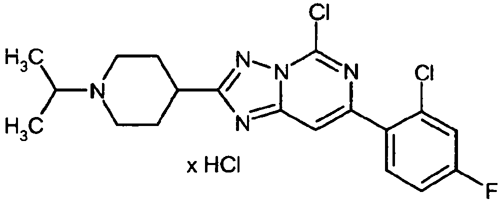

1.25 g (3.2 mmol) 7-(2-Chlor-4-fluorphenyl)-2-[1-(propan-2-yl)piperidin-4-yl][1,2,4]triazolo[1,5-c)pyrimidin=5(6H)-on (Beispiel 50A) wurde mit 4.5 ml Phosphorylchlorid versetzt, 1.46 g (6.41 mmol) Benzyltriethylammoniumchlorid wurde zugegeben, und das Reaktionsgemisch für 16 h bei 120°C gerührt. Das Reaktionsgemisch wurde eingeengt und vorsichtig unter kräftigem Rühren auf Eis gegossen. Es wurde 10 min nachgerührt. Man extrahierte die wäßrige Phase dreimal mit Dichlormethan. Die vereinigten organischen Phasen wurden vom Lösungsmittel befreit und der Rückstand im Hochvakuum getrocknet. Man erhielt 715 mg (55% d. Th.) des Produktes.
LCMS (Methode 3): Rₜ = 1.41 min. (m/z = 408 (M+H)⁺)

### Beispiel 53A

### 5-Chlor-7-(2,4-difluorphenyl)-2-[1-(propan-2-yl)piperidin-4-yl][1,2,4]triazolo[1,5-c]pyrimidin Hydrochlorid

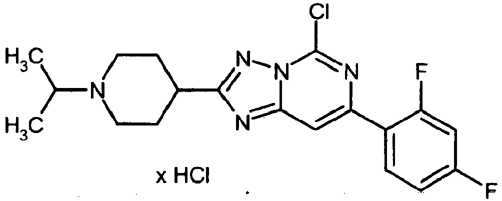

670 mg (1.72 mmol) 7-(2,4-Difluorphenyl)-2-[1-(propan-2-yl)piperidin-4-yl][1,2,4]triazolo[1,5-c]pyrimidin-5(6H)-on (Beispiel 51A) wurde mit 4.5 ml Phosphorylchlorid versetzt, 785 mg (3.45 mmol) Benzyltriethylammoniumchlorid wurde zugegeben, und das Reaktionsgemisch für 7 h bei 120°C gerührt. Das Reaktionsgemisch wurde eingeengt und vorsichtig unter kräftigem Rühren auf Eis gegossen. Man extrahierte die wäßrige Phase dreimal mit Essigsäureethylester. Die vereinigten organischen Phasen wurden vom Lösungsmittel befreit und ohne weitere Aufreinigung eingesetzt. Man erhielt 590 mg (80% d. Th.) des Produktes.
LCMS (Methode 3): Rₜ = 1.39 min. (m/z = 392 (M+H)⁺)

### Beispiel 54A

### Ethyl-{4-[7-(2,4-dichlorphenyl)-5-oxo-5,6-dihydro[1,2,4]triazolo[1,5-c]pyrimidin-2-yl]piperidin-1-yl}acetat

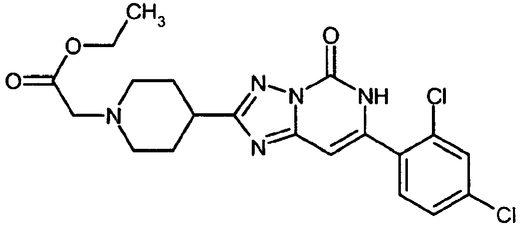

1.00 g (2.50 mmol) 7-(2,4-Dichlorphenyl)-2-(piperidin-4-yl)[1,2,4]triazolo[1,5-c]pyrimidin-5(6H)-on Hydrochlorid (Beispiel 43A) wurden in DMF (6 ml) gelöst und 332 µl (3 mmol) 1-Bromessigsäurcethylester und 862 mg (6.24 mmol) Kaliumcarbonat wurden zugegeben. Das Reaktionsgemisch wurde 16 h bei 80°C gerührt, anschliessend wurde die Lösung filtriert und mit Ethylacetat und Wasser versetzt. Die Phasen wurden getrennt, und die wässrige Phase wurde mit Ethylacetat (zweimal 15 ml) extrahiert. Die vereinigten organischen Phasen wurden mit gesättigter wässriger Natriumchloridlösung gewaschen, getrocknet (Magnesiumsulfat) und eingeengt. Das Produkt wurde auf Kieselgur aufgezogen und über Kieselgel mit Dichlormethan/Methanol (70:30) chromatographiert. Es wurden 505 mg (45% d. Th.) des Produktes erhalten.
LCMS (Methode 8): Rₜ = 0.79 min. (m/z = 450 (M+H)⁺)
¹H-NMR (400MHz, DMSO-d₆): δ = 12.29 (s, br, 1H), 7.84 (s, 1H), 7.62 (s, 2H), 6.82 (s, 1H), 4.09 (q, 2H), 3.32 (s, br, 2H), 2.87-2.94 (m, 2H), 2.75-2.85 (m, 1H), 2.36 (dt, 2H), 1.95-2.02 (m, 2H), 1.73-1.86 (m, 2H), 1.20 (t, 3H).

### Beispiel 55A

### Ethyl-{4-[5-chlor-7-(2,4-dichlorphenyl)[1,2,4]triazolo[1,5-c]pyrimidin-2-yl]piperidin-1-yl}acetat

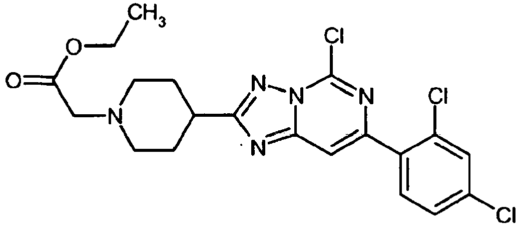

500 mg (1.11 mmol) Ethyl-{4-[7-(2,4-dichlorphenyl)-5-oxo-5,6-dihydro[1,2,4]triazolo[1,5-c]-pyrimidin-2-yl]piperidin-1-yl}acetat (Beispiel 54A) wurde mit 5 ml Phosphorylchlorid versetzt, 1.01 g (4.44 mmol) Benzyltriethylammoniumchlorid wurde zugegeben und das Reaktionsgemisch für 2.5 h bei 120°C gerührt. Das Reaktionsgemisch wurde eingeengt und vorsichtig unter kräftigem Rühren auf gesättigte wässrige Natriumhydrogencarbonatlösung gegossen. Es wurde pH = 7 durch Zugabe von festem Natriumhydrogencarbonat eingestellt. Man extrahierte die wäßrige Phase dreimal mit Dichlormethan. Die vereinigten organischen Phasen wurden einmal mit Wasser gewaschen, mit Magnesiumsulfat getrocknet und dann vom Lösungsmittel befreit. Ohne weitere Aufreinigung erhielt man 506 mg (88% d. Th.) des Produktes.
LCMS (Methode 3): Rₜ = 1.59 min. (m/z = 468 (M+H)⁺)

### Beispiel 56A

### Ethyl-(4-{5-[(2-{[6-amino-5-(trifluoracetyl)pyridin-2-yl]amino}ethyl)amino]-7-(2,4-dichlorphenyl)[1,2,4]triazolo[1,5-c]pyrimidin-2-yl}piperidin-1-yl)acetat

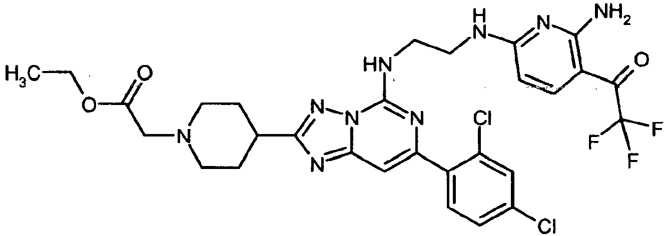

120 mg (0.23 mmol) Ethyl-{4-[5-chlor-7-(2,4-dichlorphenyl)[1,2,4]triazolo[1,5-c]pyrimidin-2-yl]piperidin-1-yl}acetat (Beispiel 55A), 78.7 mg (0.28 mmol) 1-{2-Amino-6-[(2-aminoethyl)-amino]pyridin-3-yl}-2,2,2-trifluorethanon Hydrochlorid (Beispiel 13A) und 0.24 ml (1.38 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 141 mg (90% d. Th.) des Produktes als Feststoff.
LCMS (Methode 6): Rₜ = 1.58 min. (m/z = 680 (M+H)⁺)

### Beispiel 57A

### Ethyl-{4-[5-({2-[(5-cyanopyridin-2-yl)amino]ethyl}amino)7-(2,4-dichlorphenyl)[1,2,4]-triazolo[1,5-c]pyrimidin-2-yl]piperidin-1-yl}acetat

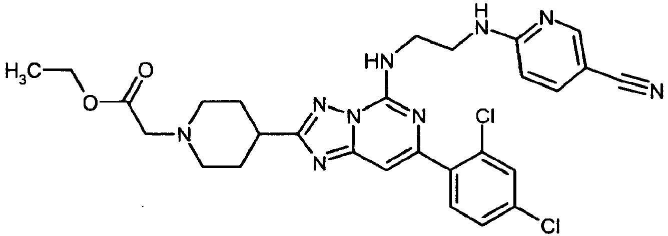

120 mg (0.23 mmol) Ethyl-{4-[5-chlor-7-(2,4-dichlorphenyl)[1,2,4]triazolo[1,5-c]pyrimidin-2-yl]piperidin-1-yl}acetat (Beispiel 55A), 54.9 mg (0.28 mmol) 6-[(2-Aminoethyl)amino]pyridin-3-carbonitril Dihydrochlorid (Beispiel 2A) und 0.24 ml (1.38 mmol) N,N-Diisopropylethylamin wurden in 2.33 ml DMSO vorgelegt. Es wurde bei 130°C für 30 min in der Mikrowelle erhitzt. Man erhielt nach Reinigung des Rohproduktes mittels präparativer HPLC (Methode 11) 113 mg (77% d. Th.) des Produktes als Feststoff.
LCMS (Methode 6): Rₜ = 1.41 min. (m/z = 594 (M+H)⁺)

### Ausführungsbeispiele

### Beispiel 1

### 1-(2-Amino-6-{[2-({7-(2,4-dichlorphenyl)-2-[1-(propan-2-yl)piperidin-4-yl][1,2,4]triazolo[1,5-c]-pyrimidin-5-yl}amino)ethyl]amino}pyridin-3-yl)-2,2,2-trifluorethanon

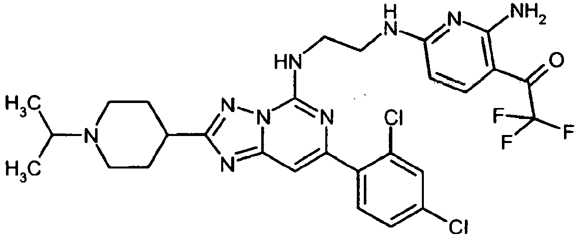

250 mg (0.59 mmol) 5-Chlor-7-(2,4-dichlorphenyl)-2-[1-(propan-2-yl)piperidin-4-yl][1,2,4]-triazolo[1,5-c]pyrimidin Hydrochlorid (Beispiel 40A), 201 mg (0.71 mmol) 1-{2-Amino-6-[(2-aminoethyl)amino]pyridin-3-yl}-2,2,2-trifluorethanon Hydrochlorid (Beispiel 13A) und 0.615 ml (3.5 mmol) N,N-Diisopropylethylamin wurden in 4 ml DMSO vorgelegt. Es wurde bei 130°C für 30 min in der Mikrowelle erhitzt. Man erhielt nach Reinigung des Rohproduktes mittels präparativer HPLC (Methode 11) 210 mg (56% d. Th.) des Produktes als Feststoff.
LCMS (Methode 8): Rₜ = 1.15 min. (m/z = 636 (M+H)⁺)
¹H-NMR (400MHz, DMSO-d₆): δ = 10.64 (br, 1H), 8.37 (t, 1H), 7.66 (s, 1H), 7.59 (d, 1H), 7.50 (d, 1H), 7.41 (dd, 1H), 7.20 (s, 1H), 5.91 (d, 1H), 3.87-3.75 (br m, 4H), 3.46 (m, 4H), 3.28-3.07 (m, 4H), 2.39 (m, 1H), 2.28 (m, 4H), 1.33 (d, 6H).

### Beispiel 2

### 2-Amino,-6-[2-({7-(2,4-dichlorphenyl)-2-[1-(propan-2-yl)piperidin-4-yl][1,2,4]triazolo[1,5-c]-pyrimidin-5-yl}amino)ethyl]amino}pyridin-3-carbonitril

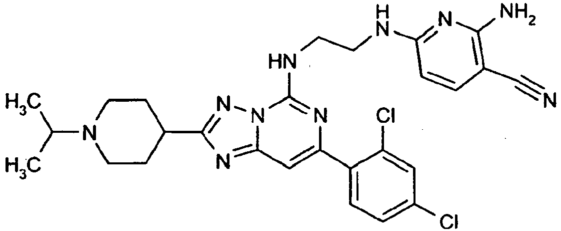

50 mg (0.12 mmol) 5-Chlor-7-(2,4-dichlorphenyl)-2-[1-(propan-2-yl)piperidin-4-yl][1,2,4]triazolo-[1,5-c]pyrimidin Hydrochlorid (Beispiel 40A), 30 mg (0.14 mmol) 2-Amino-6-[(2-aminoethyl)amino]pyridin-3-carbonitril Dihydrochlorid (Beispiel 9A) und 0.125 ml (3.5 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 45 mg (68% d. Th.) des Produktes als Feststoff.
LCMS (Methode 3): Rₜ = 1.63 min. (m/z = 565 (M+H)⁺)
¹H-NMR (400MHz, DMSO-d₆): δ = 10.21 (br, 1H), 8.37 (t, 1H), 7.75 (d, 1H), 7.60 (d, 1H), 7.51 (dd, 1H), 7.38 (br, 1H), 7.20 (s, 1H), 5.82 (br, 1H), 3.74 (m, 2H), 3.64 (m, 2H), 3.47 (m, 4H), 3.16 (m, 4H), 2.41 (m, 1H), 2.27 (m, 4H), 1.32 (d, 6H).

### Beispiel 3

### 6-{[2-({7-(2,4-Dichlorphenyl)-2-[1-(propan-2-yl)piperidin-4-yl][1,2,4]triazolo[1,5-c]pyrimidin-5-yl}amino)ethyl]amino}pyridin-3-carbonitril

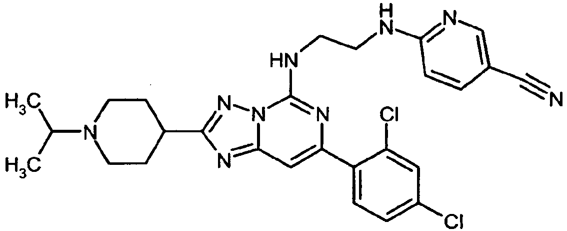

50 mg (0.12 mmol) 5-Chlor-7-(2,4-dichlorphenyl)-2-[1-(propan-2-yl)piperidin-4-yl][1,2,4]triazolo-[1,5-c]pyrimidin Hydrochlorid (Beispiel 40A), 28 mg (0.14 mmol) 6-[(2-Aminoethyl)-amino]pyridin-3-carbonitril Dihydrochlorid (Beispiel 2A) und 0.125 ml (3.5 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 48 mg (74% d. Th.) des Produktes als Feststoff.
LCMS (Methode 8): Rₜ = 1.06 min. (m/z= 550 (M+H)⁺)
¹H-NMR (400MHz, DMSO-d₆): δ = 10.17 (br, 1H), 8.36 (m, 2H), 7.88 (br, 1H), 7.73 (d, 1H), 7.60-7.49 (m, 4H), 7.18 (s, 1H), 6.50 (br, 1H), 3.72 (m, 2H), 3.64 (m, 2H), 3.48 (m, 4H), 3.29-3.11 (m, 4H), 2.41 (m, 1H), 2.26 (m, 4H), 1.32 (d, 6H).

### Beispiel 4

### 1-(2-Amino-6-{[2-({7-(2,4-dichlorphenyl)-2-[1-(propan-2-yl)piperidin-4-yl][1,2,4]triazolo[1,5-c]-pyrimidin-5-yl}amino)ethyl]amino}pyridin-3-yl)propan-1-on

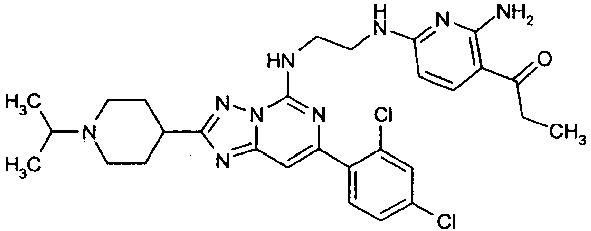

30 mg (0.07 mmol) 5-Chlor-7-(2,4-dichlorphenyl)-2-[1-(propan-2-yl)piperidin-4-yl][1,2,4]triazolo-[1,5-c]pyrimidin Hydrochlorid (Beispiel 40A), 21 mg (0.09 mmol) 1-{2-Amino-6-[(2-aminoethyl)amino]pyridin-3-yl}propan-1-on Hydrochlorid (Beispiel 25A) und 75 µl (0.4 mmol) N,N-Diisopropylethylamin wurden in 1 ml DMSO vorgelegt. Es wurde bei 120°C für 30 min in der Mikrowelle erhitzt. Man erhielt nach Reinigung des Rohproduktes mittels präparativer HPLC (Methode 11) 21 mg (50% d. Th.) des Produktes als Feststoff.
LCMS (Methode 6): Rₜ = 1.19 min. (m/z = 596 (M+H)⁺)
¹H-NMR (400MHz, DMSO-d₆): δ = 10.06 (br, 1H), 8.36 (br, 1H), 7.81 (m, 1H), 7.68 (m, 1H), 7.57 (dd, 1H), 7.44 (br m, 1H), 7.20 (s, 1H), 5.75 (br, 1H), 3.90-3.71 (m, 4H), 3.49 (m, 4H), 3.27-3.12 (m, 4H), 2.74 (m, 2H), 2.40 (m, 1H), 2.33-2.16 (m, 4H), 1.33 (d, 6H), 1.06 (t, 3H).

### Beispiel 5

### 1-{2-Amino-6-[(1-{7-(2,4-dichlorphenyl)-2-[1-(propan-2-yl)piperidin-4-yl][1,2,4]triazol[1,5-c]-pyrimidin-5-yl}piperidin-3-yl)amino]pyridin-3-yl}-2,2,2-trifluorethanon

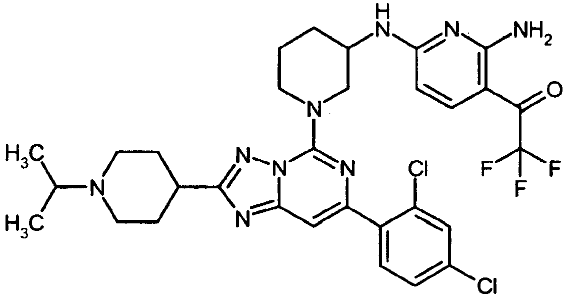

30 mg (0.07 mmol) 5-Chlor-7-(2,4-dichlorphenyl)-2-[1-(propan-2-yl)piperidin-4-yl][1,2,4]triazolo-[1,5-c]pyrimidin, 28 mg (0.09 mmol) 1-[2-Amino-6-(piperidin-3-ylamino)pyridin-3-yl]-2,2,2-trifluorethanon Hydrochlorid (Beispiel 22A) und 75 µl (0.4 mmol) N,N-Diisopropylethylamin wurden in 1 ml DMSO vorgelegt. Es wurde bei 120°C für 30 min in der Mikrowelle erhitzt. Man erhielt nach Reinigung des Rohproduktes mittels präparativer HPLC (Methode 11) 20 mg (42% d. Th.) des Produktes als Feststoff.
LCMS (Methode 9): Rₜ = 2.16 min. (m/z = 676 (M+H)⁺)
¹H-NMR (400MHz, DMSO-d₆): δ = 9.59 (br, 1H), 8.51 (br, 1H), 8.02 (br, 1H), 7.77-7.67 (m, 3H), 7.54-7.47 (m, 2H), 7.38 (s, 1H), 5.95 (d, 1H), 4.36-4.23 (m, 2H), 3.48 (m, 1H) 3.41 (m, 2H), 3.22-3.04 (m, 2H), 2.26 (m, 2H), 2.07 (m, 2H), 1.72 (m, 1H), 1.29 (d, 6H). Einige weitere Signale sind unter dem breiten Wassersignal verborgen.

### Beispiel 6

### 1-{2-Amino-6-[(1-{7-(2,4-difluorphenyl)-2-[1-(propan-2-yl)piperidin-4-yl][1,2,4]triazolo[1,5-c]-pyrimidin-5-yl}piperidin-3-yl)amino]pyridin-3-yl}propan-1-on

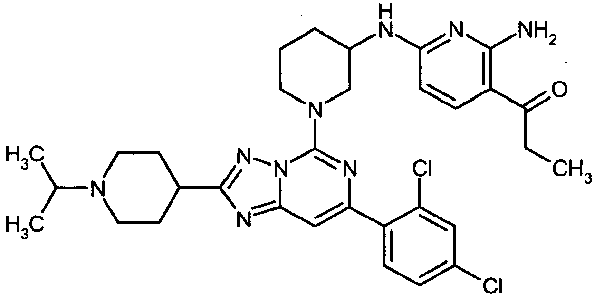

30 mg (0.07 mmol) 5-Chlor-7-(2,4-dichlorphenyl)-2-[1-(propan-2-yl)piperidin-4-yl][1,2,4]triazolo-[1,5-c]pyrimidin, 24 mg (0.09 mmol) 1-[2-Amino-6-(piperidin-3-ylamino)pyridin-3-yl]propan-1-on Hydrochlorid (Beispiel 27A) und 75 µl (0.4 mmol) N,N-Diisopropylethylamin wurden in 1 ml DMSO vorgelegt. Es wurde bei 120°C für 30 min in der Mikrowelle erhitzt. Man erhielt nach Reinigung des Rohproduktes mittels präparativer HPLC (Methode 11) 20 mg (42% d. Th.) des Produktes als Feststoff.
LCMS (Methode 6): Rₜ = 1.35 min. (m/z = 636 (M+H)⁺)
¹H-NMR (400MHz, DMSO-d₆): δ = 9.84 (br, 1H), 7.90 (br, 1H), 7.74 (m, 1H), 7.70 (d, 1H), 7.53 (m, 1H), 7.38 (s, 1H), 5.96 (br, 1H), 4.45-4.27 (br, 2H), 4.20-4.03 (br, 2H), 3.51-3.38 (m, 4H), 3.10 (m, 4H), 2.79 (m, 2H), 2.29-1.96 (m, 8H), 1.77 (m, 2H), 1.29 (d, 6H), 1.06 (t, 3H).

### Beispiel 7

### 1-(2-Amino-6-{[2-({7-(2,4-difluorphenyl)-2-[1-(propan-2-yl)piperidin-4-yl][1,2,4]triazolo[1,5-c]-pyrimidin-5-yl}amino)ethyl]amino}pyridin-3-yl)-2,2,2-trifluorethanon Hydrochlorid

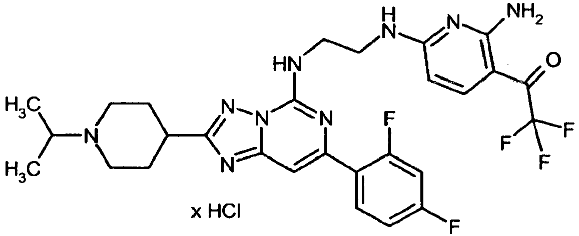

55 mg (0.14 mmol) 5-Chlor-7-(2,4-difluorphenyl)-2-[1-(propan-2-yl)piperidin-4-yl][1,2,4]triazolo-[1,5-c]pyrimidin Hydrochlorid (Beispiel 53A), 48.9 mg (0.17 mmol) 1-{2-Amino-6-[(2-aminoethyl)amino]pyridin-3-yl}-2,2,2-trifluorethanon Hydrochlorid (Beispiel 13A) und 0.147 ml (0.84 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 21 mg (23% d. Th.) des Produktes als Feststoff.
LCMS (Methode 3): Rₜ = 1.71 min. (m/z = 604 (M+H)⁺)
¹H-NMR (400MHz, DMSO-d₆): δ = 10.12 (s, br, 1H), 8.29 (t, 1H), 7.95 (dd, 1H), 7.29-7.43 (m, 1H), 7.28 (s, 1H), 6.99 (t, 1H), 5.81 (d, 1H), 3.87 (m, 2H), 3.69 (m, 2H), 3.42-3.55 (m, 4H), 3.08-3.28 (m, 4H), 2.35-2.41 (m, 1H), 2.13-2.31 (m, 4H), 1.31 (d, 6H).

### Beispiel 8

### 6-[(1-{7-(2,4-Difluorphenyl)-2-[1-(propan-2-yl)piperidin-4-yl][1,2,4]triazolo [1,5-c]pyrimidin-5-yl}piperidin-3-yl)amino]pyridin-3-carbonitril

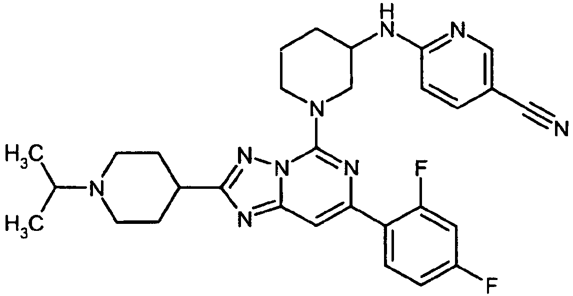

80 mg (0.2 mmol) 5-Chlor-7-(2,4-difluorphenyl)-2-[1-(propan-2-yl)piperidin-4-yl][1,2,4]triazolo-[1,5-c]pyrimidin Hydrochlorid (Beispiel 53A), 60.9 mg (0.25 mmol) 6-(Piperidin-3-ylamino)pyridin-3-carbonitril Hydrochlorid (Beispiel 19A) und 0.21 ml (1.23 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 39 mg (33% d. Th.) des Produktes als Feststoff.
LCMS (Methode 9): Rₜ = 1.83 min. (m/z = 558 (M+H)⁺)
¹H-NMR (400MHz, DMSO-d₆): δ = 8.38 (d, 1H), 8.13 (dd, 1H), 7.61 (t, 1H), 7.38-7.45 (m, 2H), 7.23 (dt, 1H), 6.52 (d, 1H), 4.36-4.45 (m, 1H), 4.13-4.28 (m, 2H), 3.87-3.98 (m, 1H), 2.82 (d, 2H), 2.6-2.72 (m, 3H), 2.18-2.28 (m, 2H), 2.03 (m, 2H), 1.88 (m, 2H), 1.6-1.78 (m, 4H), 0.98 (d, 6H).

### Beispiel 9

### 2-Amino-6-[(1-{7-(2,4-difluorphenyl)-2-[1-(propan-2-yl)piperidin-4-yl][1,2,4]triazolo[1,5-c]-pyrimidin-5-yl}piperidin-3-yl)amino]pyridin-3-carbonitril

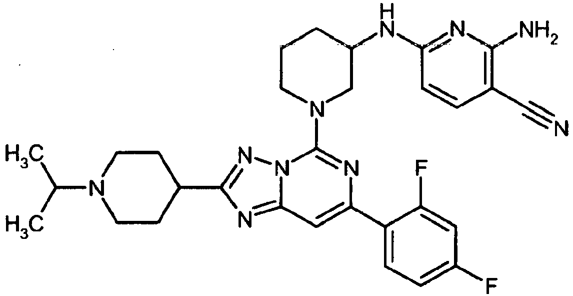

80 mg (0.2 mmol) 5-Chlor-7-(2,4-difluorphenyl)-2-[1-(propan-2-yl)piperidin-4-yl][1,2,4]triazolo-[1,5-c]pyrimidin Hydrochlorid (Beispiel 53A), 69.1 mg (0.25 mmol) 2-Amino-6-(piperidin-3-ylamino)pyridin-3-carbonitril Hydrochlorid (Beispiel 20A) und 0.213 ml (1.23 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 34 mg (28% d. Th.) des Produktes als Feststoff.
LCMS (Methode 9): Rₜ = 1.79 min. (m/z = 573 (M+H)⁺)
¹H-NMR (400MHz, DMSO-d₆): δ = 8.11 (dd, 1H), 7.36-7.45 (m, 2H), 7.19-7.3 (m, 2H), 7.04-7.16 (s, br, 1H), 6.23 (s, 1H), 5.76 (s, br, 1H), 4.08-4.45 (m, br, 3H), 3.78-3.95 (m, br, 2H), 2.79-2.88 (m, 3H), 2.65-2.77 (m, 2H), 2.25 (t, 2H), 1.89-2.04 (m, 4H), 1.6-1.78 (m, 4H), 0.99 (d, 6H).

### Beispiel 10

### 1-{2-Amino-6-[(1-{7-(2,4-difluorphenyl)-2-[1-(propan-2-yl)piperidin-4-yl][1,2,4]triazolo[1,5-c]-pyrimidin-5-yl}piperidin-3-yl)amino]pyridin-3-yl}-2,2,2-tritluorethanon

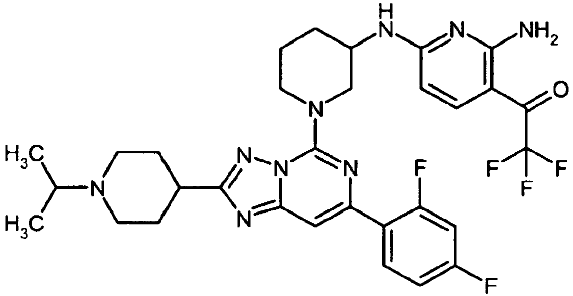

80 mg (0.2 mmol) 5-Chlor-7-(2,4-difluorphenyl)-2-[1-(propan-2-yl)piperidin-4-yl][1,2,4]triazolo-[1,5-c]pyrimidin, 81.2 mg (0.24 mmol) 1-[2-Amino-6-(piperidin-3-ylamino)pyridin-3-yl]-2,2,2-trifluorethanon Hydrochlorid (Beispiel 22A) und 0.213 ml (1.23 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 56 mg (42% d. Th.) des Produktes als Feststoff.
LCMS (Methode 3): Rₜ = 1.84 min. (m/z = 644 (M+H)⁺)
¹H-NMR (400MHz, DMSO-d₆): δ = 8.52 (s, br, 1H), 8.12 (dd, 1H), 7.91 (d, 1H), 7.65 (s, br, 1H), 7.36-7.49 (m, 3H), 7.18 (t, 1H), 5.88 (d, 1H), 4.33-4.51 (m, br, 2H), 4.07-4.22 (m, 2H), 3.44-3.57 (m, 1H), 2.7-2.81 (m, 2H), 2.58-2.69 (m, 2H), 2.08-2.2 (m, 2H), 1.99-2.09 (m, 2H), 1.82-1.94 (m, 2H), 1.57-1.78 (m, 4H), 0.94 (d, 6H).

Die Enantiomerentrennung von racemischem 1-{2-Amino-6-[(1-{7-(2,4-difluorphenyl)-2-[1-(propan-2-yl)piperidin-4-yl][1,2,4]triazolo[1,5-c]-pyrimidin-5-yl}piperidin-3-yl)amino]pyridin-3-yl}-2,2,2-trifluorethanon (Beispiel 10) wurde unter den folgenden Bedingungen durchgerührt:

Eine Probe von Beispiel 10 (46 mg) wurde in 2-Propanol gelöst und über eine Daicel Chiralpak AS-H, 5µm, 250 mm x 20 mm Säule chromatographiert (Fluß: 15 ml/min; Detektion bei 220 nm; Injektionsvolumen: 1000 µl; Eluent: iso-Hexan:2-Propanol + 0.2% Diethylamin (70:30), Temperatur: 40°C). Es wurden zwei Fraktionen isoliert:
**Beispiel Ent-A-10:** Es wurden 14 mg Produkt in > 99% ee isoliert.
Retentionszeit 4.25 min
**Beispiel Ent-B-10:** Es wurden 20 mg Produkt in > 99% ee isoliert.
Retentionszeit 6.01 min

### Beispiel 11

### 6- {[2-({7-(2-Chlor-4-fluorphenyl)-2-[1-(propan-2-yl)piperidin-4-yl][1,2,4]triazolo[1,5-c]-pyrimidin-5-yl}amino)ethyl]amino}pyridin-3-carbonitril Hydrochlorid

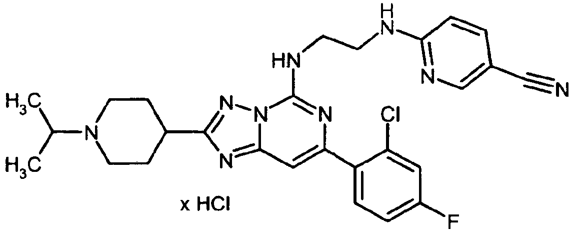

55 mg (0.11 mmol) 5-Chlor-7-(2-chlor-4-fluorphenyl)-2-[1-(propan-2-yl)piperidin-4-yl][1,2,4]-triazolo[1,5-c]pyrimidin Hydrochlorid (Beispiel 52A), 27.3 mg (0.14 mmol) 6-[(2-Aminoethyl)amino]pyridin-3-carbonitril Hydrochlorid (Beispiel 2A) und 0.12 ml (0.69 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 28 mg (43% d. Th.) des Produktes als Feststoff.
LCMS (Methode 8): Rₜ = 1.01 min. (m/z = 534 (M+H)⁺)
¹H-NMR (400MHz, DMSO-d₆): δ= 10.19 (s, br, 1H), 8.3-8.39 (m, 1H), 7.93 (s, br, 1H), 7.51-7.67 (m, 3H), 7.26-7.36 (dt, 1H), 7.14 (s, 1H), 6.50 (s, br, 1H), 3.71 (m, 2H), 3.63 (m, 2H), 3.42-3.53 (m, 2H), 3.3-3.4 (m, 1H), 3.1-3.29 (m, 3H), 2.38-2.43 (m, 1H), 2.18-2.32 (m, 4H), 1.31 (d, 6H).

### Beispiel 12

### 2-Amino-6-[(1-{7-(2-chlor-4-fluorphenyl)-2-[1-(propan-2-yl)piperidin-4-yl][1,2,4]triazolo[1,5-c]-pyrimidin-5-yl}piperidin-3-yl)amino]pyridin-3-carbonitril

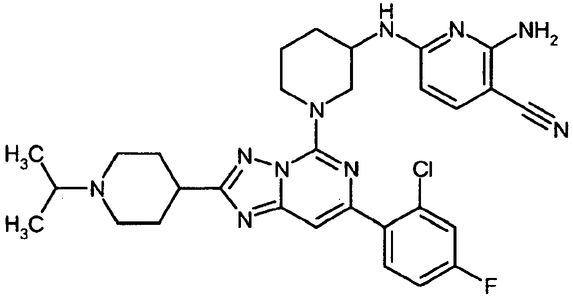

80 mg (0.17 mmol) 5-Chlor-7-(2-chlor-4-fluorphenyl)-2-[1-(propan-2-yl)piperidin-4-yl][1,2,4]-triazolo[1,5-c]pyrimidin Hydrochlorid (Beispiel 52A), 56.3 mg (0.2 mmol) 2-Amino-6-(piperidin-3-ylamino)pyridin-3-carbonitril Hydrochlorid (Beispiel 20A) und 0.17 ml (0.99 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 47 mg (44% d. Th.) des Produktes als Feststoff.
LCMS (Methode 3): Rₜ = 1.66 min. (m/z = 589 (M+H)⁺)
¹H-NMR (400MHz, DMSO-d₆): δ = 9.87 (s, br, 1H), 7.75 (dd, 1H), 7.58 (dd, 1H), 7.3-7.4 (m, 3H), 5.85 (s, br, 1H), 4.2-4.6 (m, 4H), 4.7-4,9 (m, 2H), 3.4-3.54 (m, 3H), 3.05-3.20 (m, 3H), 2.29-2.36 (m, 1H), 2.17-2.28 (m, 2H), 2.06-2.17 (m, 2H), 1.92-2.06 (m, 2H), 1.62-1.76 (m, 2H), 1.31 (d, 6H).

### Beispiel 13

### 1-{2-Amino-6-[(1-{7-(2-chlor-fluorphenyl)-2-[1-(propan-2-yl)piperidin-4-yl][1,2,4]triazolo[1,5-c]pyrimidin-5-yl}piperidin-3-yl)amino]pyridin-3-yl}-2,2,2-trifluorethanon

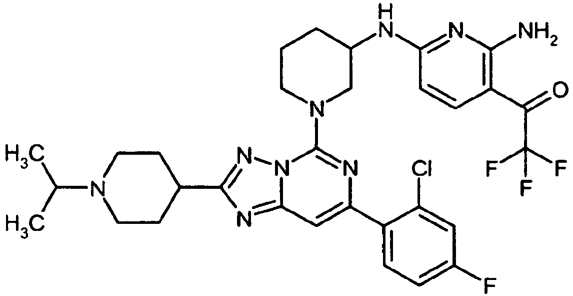

80 mg (0.17 mmol) 5-Chlor-7-(2-chlor-4-fluorphenyl)-2-[1-(propan-2-yl)piperidin-4-yl][1,2,4]-triazolo[1,5-c]pyrimidin Hydrochlorid, 64.9 mg (0.2 mmol) 1-[2-Amino-6-(piperidin-3-ylamino)-pyridin-3-yl]-2,2,2-trifluorethanon Hydrochlorid (Beispiel 22A) und 0.17 ml (0.99 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 32 mg (28% d. Th.) des Produktes als Feststoff.
LCMS (Methode 3): Rₜ = 1.84 min. (m/z = 660 (M+H)⁺)
¹H-NMR (400MHz, DMSO-d₆): δ = 8.50 (s, br, 1H), 7.93 (d, 1H), 7.75 (dd, 1H), 7.64 (s, br, 1H), 7.56 (dd, 1H), 7.46 (d, 1H), 7.27-7.38 (m, 2H), 5.91 (d, 1H), 4.2-4.4 (m, br, 3H), 4.02-4.13 (m, 1H), 3.87-3.97 (m, 1H), 2.7-2.8 (m, 2H), 2.6-2.7 (m, 2H), 2.09-2.2 (m, 2H), 1.95-2.08 (m, 2H), 1.82-1.93 (m, 2H), 1.58-1.77 (m, 4H), 0.94 (d, 6H).

Die Enantiomerentrennung von racemischem 1-{2-Amino-6-[(1-{7-(2-chlor-4-fluorphenyl)-2-[1-(propan-2-yl)piperidin-4-yl][1,2,4]triazolo[1,5-c]pyrimidin-5-yl}piperidin-3-yl)amino]pyridin-3-yl}-2,2,2-trifluorethanon (Beispiel 13) wurde unter den folgenden Bedingungen durchgeführt:

Eine Probe von Beispiel 13 (22 mg) wurde in 1 ml 2-Propanol gelöst und über eine Daicel Chiralpak AS-H, 5µm, 250 mm x 20 mm Säule chromatographiert (Fluß: 15 ml/min; Detektion bei 220 nm; Injektionsvolumen: 1000 µl; Eluent: iso-Hexan:2-Propanol + 0.2% Diethylamin (70:30), Temperatur: 40°C). Es wurden zwei Fraktionen isoliert:
**Beispiel Ent-A-13:** Es wurden 12 mg Produkt in > 99% ee isoliert.
Retentionszeit 4.62 min
**Beispiel Ent-B-13:** Es wurden 17 mg Produkt in > 99% ee isoliert.
Retentionszeit 7.67 min

### Beispiel 14

### 6-[(1-{7-(2-Chlor-4-fluorphenyl)-2-[1-(propan-2-yl)piperidin-4-yl][1,2,4]triazolo[1,5-c]pyrimidin-5-yl}piperidin-3-yl)amino]pyridin-3-carbonitril

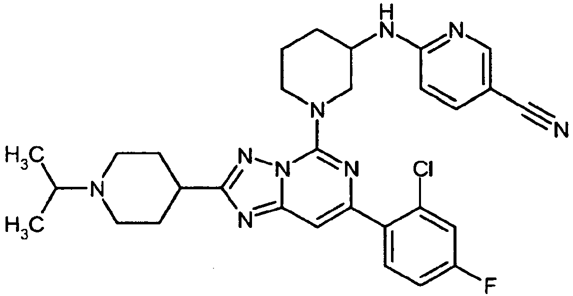

80 mg (0.2 mmol) 5-Chlor-7-(2-chlor-4-fluorphenyl)-2-[1-(propan-2-yl)piperidin-4-yl][1,2,4]-triazolo[1,5-c]pyrimidin Hydrochlorid (Beispiel 52A), 50 mg (0.2 mmol) 6-(Piperidin-3-ylamino)pyridin-3-carbonitril Hydrochlorid (Beispiel 19A) und 0.17 ml (0.99 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 39 mg (37% d. Th.) des Produktes als Feststoff.
LCMS (Methode 9): Rₜ = 1.87 min. (m/z = 574 (M+H)⁺)
¹H-NMR (400MHz, DMSO-d₆): δ = 9.78 (s, br, 1H), 8.36 (d, 1H), 7.74 (dd, 1H), 7.53-7.67 (m, 3H), 7.3-7.4 (m, 2H), 6.53 (d, 1H), 4.2-4.5 (m, 3H), 4.14 (m, 1H), 3.81 (m, 1H), 3.25-3.4 (m, 2H), 3.08-3.2 (m, 3H), 2.25-2.36 (m, 1H), 1.93-2.24 (m, 5H), 1.63-1.78 (m, 2H), 1.30 (d, 6H).

### Beispiel 15

### 1-(2-Amino-6-{[2-({7-(2-chlor-4-fluorphenyl)-2-[1-(propan-2-yl)piperidin-4-yl][1,2,4]triazolo[1,5-c]pyrimidin-5-yl}amino)ethyl]amino}pyridin-3-yl)-2,2,2-trifluorethanon

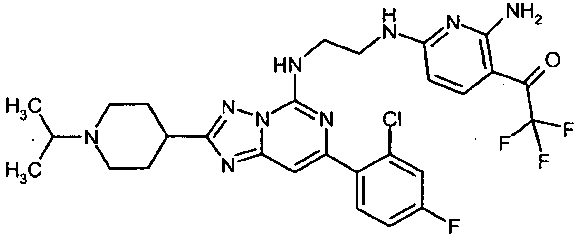

55 mg (0.11 mmol) 5-Chlor-7-(2-chlor-4-fluorphenyl)-2-[1-(propan-2-yl)piperidin-4-yl][1,2,4]-triazolo[1,5-c]pyrimidin Hydrochlorid (Beispiel 52A), 39.9 mg (0.14 mmol) 1-{2-Amino-6-[(2-aminoethyl)amino]pyridin-3-yl}-2,2,2-trifluorethanon Hydrochlorid (Beispiel 13A) und 0.12 ml (0.69 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 58 mg (81% d. Th.) des Produktes als Feststoff.
LCMS (Methode 3): Rₜ = 1.74 min. (m/z = 620 (M+H)⁺)
¹H-NMR (400MHz, DMSO-d₆): δ = 8.52 (s, br, 1H), 8.24 (t, 1H), 8.09 (t, 1H), 7.63 (dd, 1H), 7.53 (dd, 2H), 7.44 (dd, 1H), 7.31 (d, 1H), 7.22 (dt, 1H), 7.14 (s, 1H), 5.87 (d, 1H), 3.7-3.78 (m, 2H), 3.62-3.70 (m, 2H), 3.2-3.5 (m, 3H), 2.8-2.9 (m, 3H), 2.66-2.75 (m, 1H), 2.21-2.31 (m, 2H), 0.99 (d, 6H).

### Beispiel 16

### 2-Amino-6-[(2-{[2-(1-cyclopropylpiperidin-4-yl)-7-(2,4-dichlorphenyl)[1,2,4]triazolo[1,5-c]-pyrimidin-5-yl]amino}ethyl)amino]pyridin-3-carbonitril

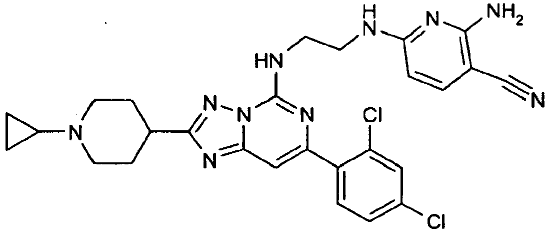

50 mg (0.12 mmol) 5-Chlor-7-(2,4-dichlorphenyl)-2-(1-cyclopropylpiperidin-4-yl)-[1,2,4]-triazolo[1,5-c]pyrimidin Hydrochlorid (Beispiel 45A), 30 mg (0.14 mmol) 2-Amino-6-[(2-aminoethyl)amino]pyridin-3-carbonitril Dihydrochlorid (Beispiel 9A) und 0.125 ml (3.5 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 43 mg (64% d. Th.) des Produktes als Feststoff.
LCMS (Methode 8): Rₜ = 1.03 min. (m/z = 563 (M+H)⁺)
¹H-NMR (400MHz, DMSO-d₆): δ = 10.46 (br, 1H), 8.37 (t, 1H), 7.73 (d, 1H), 7.62-7.58 (m, 2H), 7.51 (dd, 1H), 7.36 (m, 1H), 7.20 (s, 1H), 5.83 (br, 1H), 3.72 (m, 4H), 3.61 (m, 2H), 3.45 (m, 1H), 3.39-3.18 (m, 4H), 2.82 (m, 1H), 2.39-2.16 (m, 4H), 1.16 (m, 2H), 0.83 (m, 2H).

### Beispiel 17

### 1-{2-Amino-6-[(2-{[2-(1-cyclopropylpiperidin-4-yl)-7-(2,4-dichlorphenyl)[1,2,4]triazolo[1,5-c]-pyrimidin-5-yl]amino}ethyl)amino]pyridin-3-yl}-2,2,2-trifluorethanon

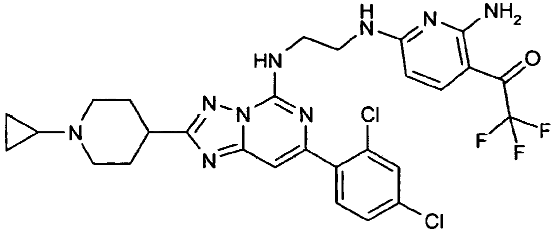

50 mg (0.12 mmol) 5-Chlor-7-(2,4-dichlorphenyl)-2-(1-cyclopropylpiperidin-4-yl)-[1,2,4]-triazolo[1,5-c]pyrimidin Hydrochlorid (Beispiel 45A), 92 mg (0.71 mmol) 1-{2-Amino-6-[(2-aminoethyl)amino]pyridin-3-yl}-2,2,2-trifluorethanon Hydrochlorid (Beispiel 13A) und 0.125 ml (3.5 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 59 mg (78% d. Th.) des Produktes als Feststoff.
LCMS (Methode 8): Rₜ = 1.17 min. (m/z = 634 (M+H)⁺)
¹H-NMR (400MHz, DMSO-d₆): δ = 10.88 (br, 1H), 8.38 (t, 1H), 7.66-7.57 (m, 2H), 7.50 (dd, 1H), 7.40 (dd, 1H), 7.21 (s, 1H), 5.90 (d, 1H), 3.85 (m, 2H), 3.78 (m, 2H), 3.46-3.17 (m, 4H), 2.80 (m, 1H), 2.39 (m, 1H), 2.26 (m, 4H), 1.24 (m, 2H), 0.81 (m, 2H).

### Beispiel 18

### 6-[(2-{[2-(1-Cyclopropylpiperidin-4-yl)-7-(2,4-dichlorphenyl)[1,2,4]triazolo[1,5-c]pyrimidin-5-yl]-amino}ethyl)amino]pyridin-3-carbonitril

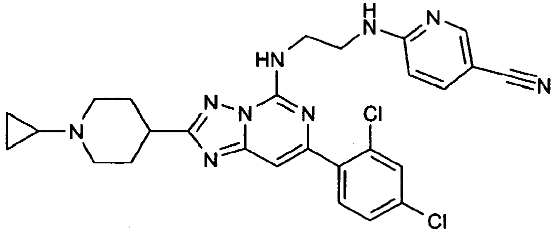

21 mg (0.10 mmol) 5-Chlor-7-(2,4-dichlorphenyl)-2-(1-cyclopropylpiperidin-4-yl)-[1,2,4]triazolo-[1,5-c]pyrimidin Hydrochlorid (Beispiel 45A), 40 mg (0.10 mmol) 6-[(2-Aminoethyl)-amino]pyridin-3-carbonitril Hydrochlorid (Beispiel 2A) und 0.10 ml (0.57 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 32 mg (62% d. Th.) des Produktes als Feststoff.
LCMS (Methode 8): Rₜ = 1.05 min. (m/z = 548 (M+H)⁺)
¹H-NMR (400MHz, DMSO-d₆): δ = 10.57 (br, 1H), 8.38 (m, 2H), 7.95 (br, 1H), 7.74 (d, 1H), 7.60-7.49 (m, 3H), 7.17 (s, 1H), 6.51 (br, 1H), 3.74 (m, 2H), 3.62 (m, 4H), 3.44 (m, 1H), 3.31 (m, 2H), 3.22 (m, 1H), 2.82 (m, 1H), 2.35 (m, 1H), 2.24 (m, 4H), 1.18 (m, 2H), 0.84 (m, 2H).

### Beispiel 19

### 1-[2-Amino-6-({1-[2-(1-cyclopropylpiperidin-4-yl)-7-(2,4-dichlorphenyl)[1,2,4]triazolo[1,5-c]-pyrimidin-5-yl]piperidin-3-yl}amino)pyridin-3-yl]propan-1-on

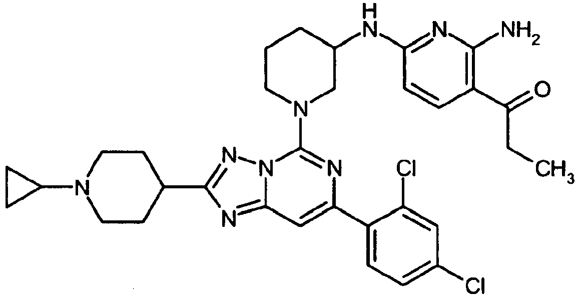

25 mg (0.06 mmol) 5-Chlor-7-(2,4-dichlorphenyl)-2-(1-cyclopropylpiperidin-4-yl)-[1,2,4]triazolo-[1,5-c]pyrimidin Hydrochlorid, 20 mg (0.07 mmol) 1-[2-Amino-6-(piperidin-3-ylamino)pyridin-3-yl]propan-1-on Hydrochlorid (Beispiel 27A) und 65 µl (0.4 mmol) N,N-Diisopropytethylamin wurden in 1.25 ml DMSO vorgelegt. Es wurde bei 120°C für 30 min in der Mikrowelle erhitzt. Man erhielt nach Reinigung des Rohproduktes mittels präparativer HPLC (Methode 11) 21 mg (56% d. Th.) des Produktes als Feststoff.
LCMS (Methode 8): Rₜ = 1.12 min. (m/z = 634 (M+H)⁺)
¹H-NMR (400MHz, DMSO-d₆): δ = 10.07 (br, 1H), 7.88 (br, 1H), 7.74 (br, 1H), 7.69 (d, 1H), 7.54 (br, 1H), 7.37 (s, 1H), 5.94 (br, 1H), 4.42-3.95 (br, 4H), 3.35-3.10 (m, 4H), 2.80 (m, 2H), 2.28-1.95 (m, 6H), 1.74 (m, 2H), 1.08 (m, 5H), 0.81 (m, 2H).

### Beispiel 20

### 2-Amino-6-{[2-({2-[1-(cyclopropylmethyl)piperidin-4-yl]-7-(2,4-dichlorphenyl)[1,2,4]triazolo[1,5-c]pyrinnidin-5-yl}amino)ethyl]amino}pyridin-3-carbonitril

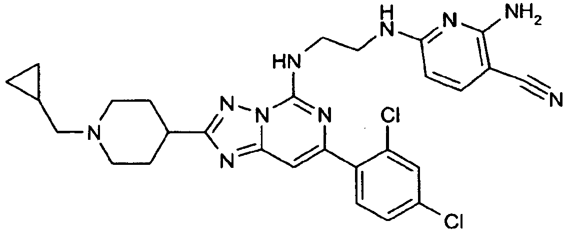

50 mg (0.10 mmol) 5-Chlor-2-[1-(cyclopropylmethyl)piperidin-4-yl]-7-(2,4-dichlorphenyl)-[1,2,4]triazolo[1,5-c]pyrimidin (Beispiel 47A), 26 mg (0.12 mmol) 2-Amino-6-[(2-aminoethyl)amino]pyridin-3-carbonitril Dihydrochlorid (Beispiel 9A) und 0.110 ml (0.6 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 35 mg (59% d. Th.) des Produktes als Feststoff.
LCMS (Methode 8): Rₜ = 1.06 min. (m/z = 577 (M+H)⁺)
¹H-NMR (400MHz, DMSO-d₆): δ = 10.18 (br, 1H), 8.36 (t, 1H), 7.74 (d, 1H), 7.61 (d, 1H), 7.52 (d, 1H), 7.34 (m, 1H), 7.20 (s, 1H), 5.82 (br, 1H), 3.49 (m, 1H), 3.15 (m, 3H), 2.98 (m, 2H), 2.36-2.12 (m, 4H), 1.15 (m, 1H), 0.67 (m, 2H), 0.42 (m, 2H). Weitere H sind unter dem breiten Wassersignal verborgen.

### Beispiel 21

### 1-(2-Amino-6-{[2-({2-[1-(cyclopropylmethyl)piperidin-4-yl]-7-(2,4-dichlorphenyl)[1,2,4]triazolo-[1,5-c]pyrimidin-5-yl}amino)ethyl]amino}pyridin-3-yl)-2,2,2-trifluorethanon

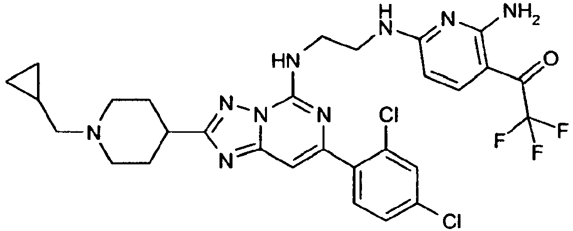

50 mg (0.10 mmol) 5-Chlor-2-[1-(cyclopropylmethyl)piperidin-4-yl]-7-(2,4-dichlorphenyl)[1,2,4]-triazolo[1,5-c]pyrimidin (Beispiel 47A), 35 mg (0.12 mmol) 1-{2-Amino-6-[(2-aminoethyl)-amino]pyridin-3-yl}-2,2,2-trifluorethanon Hydrochlorid (Beispiel 13A) und 0.110 ml (0.6 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 32 mg (48% d. Th.) des Produktes als Feststoff.
LCMS (Methode 6): Rₜ = 1.52 min. (m/z = 648 (M+H)⁺)
¹H-NMR (400MHz, DMSO-d₆): δ = 10.43 (br, 1H), 8.67 (br, 1H), 7.68 (d, 1H), 7.61 (d, 1H), 7.47 (d, 1H), 7.43 (dd, 1H), 7.20 (s, 1H), 5.88 (d, 1H), 3.79 (m, 2H), 3.67 (m, 4H), 3.48 (m, 1H), 3.14 (m, 3H), 3.00 (m, 2H), 2.43-2.17 (m, 4H), 1.16 (m, 1H), 0.67 (dd, 2H), 0.43 (dd, 2H).

### Beispiel 22

### 6-{[2-({2-[1-(Cyclopropylmethyl)piperidin-4-yl]-7-(2,4-dichlorphenyl)[1,2,4]triazolo[1,5-c]-pyrimidin-5-yl}amino)ethyl]amino}pyridin-3-carbonitril

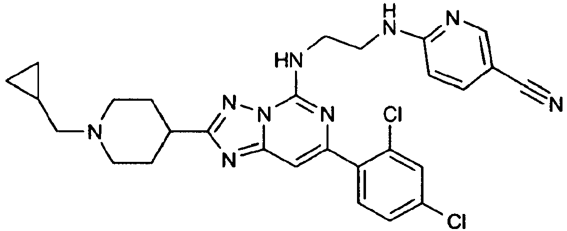

50 mg (0.10 mmol) 5-Chlor-2-[1-(cyclopropylmethyl)piperidin-4-yl]-7-(2,4-dichlorphenyl)-[1,2,4]triazolo[1,5-c]pyrimidin (Beispiel 47A), 25 mg (0.12 mmol) 6-[(2-Aminoethyl)amino]-pyridin-3-carbonitril Hydrochlorid (Beispiel 2A) und 0.110 ml (0.6 mmol) N,N-Diisopropylethylamin wurden in 1 ml DMSO vorgelegt. Es wurde bei 130°C für 30 min in der Mikrowelle erhitzt. Man erhielt nach Reinigung des Rohproduktes mittels präparativer HPLC (Methode 11) 22 mg (38% d. Th.) des Produktes als Feststoff.
LCMS (Methode 8): Rₜ = 1.11 min. (m/z = 562 (M+H)⁺)
¹H-NMR (400MHz, DMSO-d₆): δ = 10.22 (br, 1H), 8.35 (m, 2H), 7.84 (br, 1H), 7.74 (d, 1H), 7.55 (m, 3H), 7.17 (s, 1H), 6.50 (br, 1H), 3.74 (m, 2H), 3.65 (m, 4H), 3.48 (m, 1H), 3.15 (m, 3H), 3.01 (m, 2H), 2.44-2.12 (m, 5H), 1.16 (m, 1H), 0.70 (m, 2H), 0.42 (dd, 2H).

### Beispiel 23

### (4-{5-[(2-{[6-Amino-5-(trifluoracetyl)pyridin-2-yl]amino}ethyl)amino]-7-(2,4-dichlorphenyl)-[1,2,4]triazolo[1,5-c]pyrimidin-2-yl}piperidin-1-yl)essigsäure

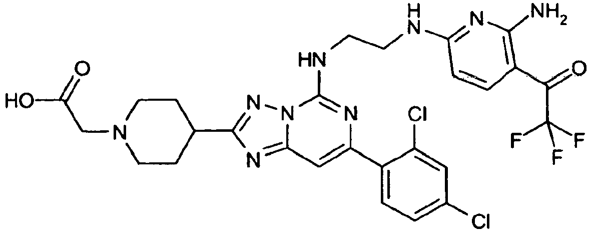

130 mg (0.19 mmol) Ethyl-(4-{5-[(2-{[6-amino-5-(trifluoracetyl)pyridin-2-yl]amino}ethyl)amino]-7-(2,4-dichlorphenyl)[1,2,4]triazolo[1,5-c]pyrimidin-2-yl}piperidin-1-yl)acetat (Beispiel 56A) wurden in 1,2-Dimethoxyethan (7.5 ml) gelöst und mit Wasser (5 ml) versetzt, 31 mg (0.76 mmol) Natriumhydroxid wurden zugegeben, und das Reaktionsgemisch wurde 16 h bei RT gerührt. Anschliessend wurde der Ansatz durch Lyophilisierung eingeengt. Man erhielt nach Reinigung des Rückstandes mittels präparativer HPLC (Methode 11) 60 mg (48% d. Th.) des Produktes als Feststoff.
LCMS (Methode 9): Rₜ = 2.13 min. (m/z = 652 (M+H)⁺)
¹H-NMR (400MHz, DMSO-d₆): δ = 10.32 (br, 1H), 8.71 (br, 1H), 8.35 (br, 1H), 7.69 (s, 1H), 7.62 (d, 1H), 7.49 (d, 1H), 7.43 (d, 1H), 7.21 (s, 1H), 5.90(d, 1H), 3.80 (m, 2H), 3.69 (m, 4H), 3.47 (m, 1H), 3.33-3.15 (m, 3H), 2.43-2.14 (m, 5H).

### Beispiel 24

### {4-[5-({2-[(5-Cyanpyridin-2-yl)amino]ethyl}amino)-7-(2,4-dichlorphenyl)[1,2,4]triazolo[1,5-c]pyrimidin-2-yl]piperidin-1-yl}essigsäure

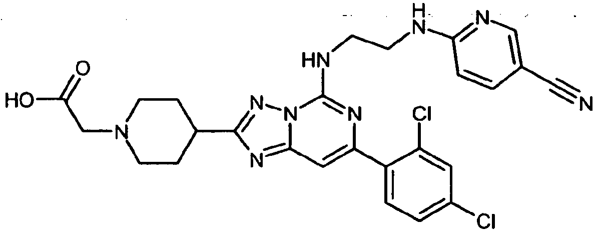

105 mg (0.18 mmol) Ethyl-{4-[5-({2-[(5-cyanopyridin-2-yl)amino]ethyl}amino)-7-(2,4-dichlorphenyl)[1,2,4]triazolo[1,5-c]pyrimidin-2-yl]piperidin-1-yl}acetat (Beispiel 57A) wurden in 1,2-Dimethoxyethan (7.5 ml) gelöst und mit Wasser (5 ml) versetzt, 31 mg (0.76 mmol) Natriumhydroxid wurden zugegeben, und das Reaktionsgemisch wurde 16 h bei RT gerührt. Anschliessend wurde der Ansatz durch Lyophilisierung eingeengt. Man erhielt nach Reinigung des Rückstandes mittels präparativer HPLC (Methode 11) 65 mg (65% d. Th.) des Produktes als Feststoff.
LCMS (Methode 9): Rₜ = 1.93 min. (m/z = 566 (M+H)⁺)
¹H-NMR (400MHz, DMSO-d₆): δ = 10.11 (br, 1H), 8.36 (m, 2H), 7.79 (br, 1H), 7.75s, 1H), 7.60-7.50 (m, 3H), 7.18 (s, 1H), 6.49 (br, 1H), 4.17 (br, 2H), 3.85 (m, 4H), 3.71 (m, 2H), 3.64 (m, 2H), 3.27 (m, 3H), 2.31 (m, 3H), 2.16 (m, 2H).

### Beispiel 25

### 2-Amino-6-[(2-{[7-(2,4-dichlorphenyl)-2-(1-methylpiperidin-4-yl)[1,2,4]triazolo[1,5-c]pyrimidin-5-yl]amino}ethyl)amino]pyridin-3-carbonitril

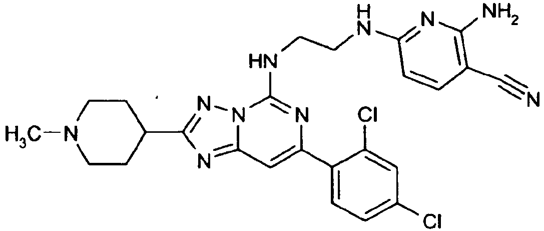

50 mg (0.12 mmol) 5-Chlor-7-(2,4-dichlorphenyl)-2-(1-methylpiperidin-4y1)[1,2,4]triazolo[1,5-c]-pyrimidin Hydrochlorid (Beispiel 49A), 32 mg (0.15 mmol) 2-Amino-6-[(2-aminoethyl)-amino]pyridin-3-carbonitril Dihydrochlorid (Beispiel 9A) und 0.13 ml (0.75 mmol) N,N-Diisopropylethylamin wurden in 1.5 ml DMSO vorgelegt. Es wurde bei 130°C für 30 min in der Mikrowelle erhitzt. Man erhielt nach Reinigung des Rohproduktes mittels präparativer HPLC (Methode 11) 43 mg (64% d. Th.) des Produktes als Feststoff.
LCMS (Methode 8): Rₜ = 0.99 min. (m/z = 537 (M+H)⁺)
¹H-NMR (400MHz, DMSO-d₆): δ = 10.80 (br, 1H), 8.38 (t, 1H), 7.75 (d, 1H), 7.60 (d, 1H), 7.53 (m, 1H), 7.30 (m, 1H), 7.19 (s, 1H), 5.81 (br, 1H), 3.70 (m, 2H), 3.60 (m, 2H), 3.51 (m, 2H), 3.35 (m, 1H), 3.14 (m, 4H), 2.75 (d, 3H), 2.70 (m, 1H), 2.35-2.12 (m, 4H).

### Beispiel 26

### 6-[(2-{[7-(2,4-Dichlorphenyl)-2-(1-methylpiperidin-4-yl)[1,2,4]triazolo[1,5-c]pyrimidin-5-yl]-amino} ethyl)amino]pyridin-3-carbonitril

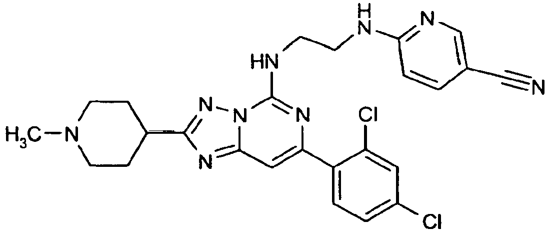

50 mg (0.13 mmol) 5-Chlor-7-(2,4-dichlorphenyl)-2-(1-methylpiperidin-4-yl)[1,2,4]triazolo[1,5-c]-pyrimidin Hydrochlorid (Beispiel 49A), 30 mg (0.15 mmol) 6-[(2-Aminoethyl)amino]pyridin-3-carbonitril Hydrochlorid (Beispiel 2A) und 0.13 ml (0.76 mmol) N,N-Diisopropylethylamin wurden in 1.5 ml DMSO vorgelegt. Es wurde bei 130°C für 30 min in der Mikrowelle erhitzt. Man erhielt nach Reinigung des Rohproduktes mittels präparativer HPLC (Methode 11) 25 mg (37% d. Th.) des Produktes als Feststoff.
LCMS (Methode 3): Rₜ = 1.57 min. (m/z = 522 (M+H)⁺)
¹H-NMR (400MHz, DMSO-d₆): δ = 10.40 (br, 1H), 8.36 (m, 2H), 7.87 (br, 1H), 7.74 (d, 1H), 7.63-7.49 (m, 3H), 7.18 (s, 1H), 6.50 (br, 1H), 3.72 (m, 2H), 3.62 (m, 2H), 3.54 (m, 2H), 3.39 (m, 1H), 3.15 (m, 4H), 2.2.78 (d, 3H), 2.72 (m, 1H), 2.35-2.05 (m, 4H).

### Beispiel 27

### 1-{2-Amino-6-[(2-{[7-(2,4-dichlorphenyl)-2-(1-methylpiperidin-4-yl)[1,2,4]triazolo[1,5-c]-pyrimidin-5-yl]amino}ethyl)amino]pyridin-3-yl}-2,2,2-trifluorethanon

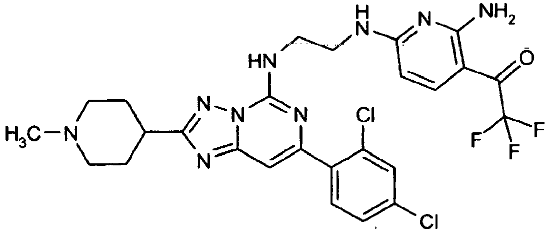

50 mg (0.13 mmol) 5-Chlor-7-(2,4-dichlorphenyl)-2-(1-methylpiperidin-4-yl)[1,2,4]triazolo[1,5-c]pyrimidin Hydrochlorid (Beispiel 49A), 43 mg (0.15 mmol) 1-{2-Amino-6-[(2-aminoethyl)-amino]pyridin-3-yl}-2,2,2-trifluorethanon Hydrochlorid (Beispiel 13A) und 0.130 ml (0.76 mmol) N,N-Diisopropylethylamin wurden in 1.5 ml DMSO vorgelegt. Es wurde bei 130°C für 30 min in der Mikrowelle erhitzt. Man erhielt nach Reinigung des Rohproduktes mittels präparativer HPLC (Methode 11) 52 mg (67% d. Th.) des Produktes als Feststoff.
LCMS (Methode 8): Rₜ = 1.13 min. (m/z = 608 (M+H)⁺)
¹H-NMR (400MHz, DMSO-d₆): δ = 10.86 (br, 1H), 8.65 (br, 1H), 8.38 (m, 1H), 7.70 (d, 1H), 7.59 (d, 1H), 7.47 (d, 1H), 7.42 (dd, 1H), 7.20 (s, 1H), 5.89 (d, 1H), 3.78 (m, 2H), 3.69 (m, 2H), 3.52 (m, 4H), 3.13 (m, 2H), 2.76 (d, 3H), 2.40 (m, 1H), 2.22 (m, 4H).

### B) Bewertung der physiologischen Wirksamkeit

Die Eignung der erfindungsgemäßen Verbindungen zur Behandlung von hämatologischen Erkrankungen kann in folgenden Assaysystemen gezeigt werden:

### In vitro Assay

Die inhibitorische Aktivität von Wirksubstanzen wird in einem biochemischen Assay bestimmt. Die dazu benötigten Bestandteile werden in einer schwarzen 384-Loch-Mikrotitterplatte mit transparentem Boden (Firma Greiner, Katalognummer 781092) gemischt. Benötigt werden dabei pro Loch der 384-Loch-Mikrotitterplatte 5 nM GSK3ß (Firma Upstate, Katalognummer 14-306), 40 µM GSK3ß-Substrat GSM (Sequenz H-RRRPASVPPSPSLSRHS-(pS)-HQRR, Firma Upstate, Katalognummer 2-533), 30 µM Nicotinsäureamid-Adenin-Dinucleotid NADH (Roche Diagnostics, Katalognummer 10107735), 50 µM Adenosin-triphoshat ATP (Firma Sigma, Katalognummer A7966), 2 mM Phosphoenolpyruvat (Firma Roche, Katalognummer 128112), sowie ca. 1 U/ml Pyruvatkinase und ca. 1 U/ml Lactatdehydrogenase, die zusammen in einer Stammformulierung vorliegen (Firma Roche, Katalognummer 10737291001, Suspension mit ca. 450 U/ml Pyruvatkinase-Aktivität, ca. 450 U/ml Lactatdehydrogenase-Aktivität in 3.2 mM Ammoniumsulfat-Lösung pH 6). Wobei 1 Unit Pyruvatkinase 1 µmol Phosphoenolpyruvat zu Pyruvat pro Minute bei pH 7.6 und 37°C umsetzt und wobei 1 Unit Laktatdehydrogenase 1 µmol Pyruvat zu Lactat pro Minute bei pH 7.5 und 37°C reduziert. Der benötigte Reaktionspuffer, in dem die biochemische Reaktion abläuft, besteht aus 50 mM Trizma Hydrochlorid Tris- HCl pH: 7.5 (Firma Sigma, Katalognummer T3253), 5 mM Magnesiumchlorid MgCl₂ (Firma Sigma, Katalognummer M8266), 0.2 mM DL-Dithiothreitol DTT (Firma Sigma, Katalognummer D9779), 2 mM Ethylendiaminethertetrasäure EDTA (Firma Sigma, Katalognummer E6758), 0.01% Triton X-100 (Firma Sigma, Katalognummer T8787) und 0.05% Bovines Serumalbumin BSA (Firma Sigma, Katalognummer B4287).

Wirksubstanzen werden in Dimethylsulfoxid DMSO (Firma Sigma, Katalognummer D8418) in einer Konzentration von 10 mM gelöst. Wirksubstanzen werden in Konzentrationsreihen von 10 µM, 1 µM, 0.1 µM, 0.01 µM, 0.001 µM, 0.0001 µM, 0.00001 µM, 0.000001 µM zu den Ansätzen der biochemischen Reaktion zugegeben. Als Kontrolle wird statt Substanz Dimethylsulfoxid in einer Endkonzentration von 0.1 % zugesetzt.

Die Reaktion wird für 2 Stunden bei 30°C inkubiert und anschließend die entstandene Fluoreszenz in einem Tecan Safire-XFLUOR4-Gerät, Version V4.50 (Serienummer 12901300283) unter den Spezifikationen: Messmodus - Fluoreszenz, von unten gemessen, Extinktionswellenlänge 340 nm, Emissionswellenlänge 465 nm, Spaltbreite Extinktion 5 nm, Spaltbreite Emission 5 nm, Verstärkermodus 120, Verzögerung 0 µs, Anzahl Lichtblitze pro Messung 3, und einer Integrationszeit von 40 µs gemessen.

Die Aktivität der GSK3ß wird in Fluoreszenz-Einheiten ermittelt, wobei die Werte von nichtinhibierter Kinase gleich 100% und vollständig inhibierter Kinase gleich 0% gesetzt werden. Die Aktivität der Wirksubstanzen wird auf diese 0% und 100% verrechnet.

Tabelle A zeigt IC₅₀-Werte, die in dem oben beschriebenen Assay ermittelt wurden:

**Tabelle A**

| **Beispiel-Nr.** | **IC₅₀ [nM]** |
|---|---|
| **1** | 15 |
| **5** | 10 |
| **13** | 4 |
| **21** | 20 |
| **23** | 9 |
| **Ent-A-13** | 2 |

### CD34+-Proliefrationsassays zur Testung von GSK3ß-Inhibitoren

Adulte hämatopoetische Stammzellen sind durch die spezifische Ausprägung von membranständigen Proteinen gekennzeichnet. Entsprechend ihrem Molekulargewicht sind diese Oberflächenmarker mit einer entsprechenden Nummer versehen. Zu dieser Klasse gehört auch das als CD34 bezeichnete Molekül, welches zur Identifizierung, Charakterisierung und Isolierung von adulten hämtopoetischen Stammzellen dient. Diese Stammzellen können dabei aus dem Knochenmark, dem peripheren Blut oder aus Nabelschnurblut isoliert werden. In in vitro-Kulturen sind diese Zellen begrenzt lebensfähig, können aber durch unterschiedlichste Zusätze zum Kulutrmedium zu Proliferation und Differenzierung angeregt werden. CD34-positive Zellen werden hier verwendet, um den Einfluss von Substanzen auf die Aktivität der Glykogen Synthase Kinase 3 zu testen. Zu diesem Zweck werden in einem ersten Schritt über differentielle Zentrifugationsschritte mononukleare Zellen aus Nabelschnurblut isoliert.

Dazu wird Nabelschnurblut 1:4 mit Phosphat-gepufferter Salzlösung verdünnt. 50 Milliliter Zentrifugationgefäße werden mit 17 Milliliter Ficoll (Dichte 1.077, Ficoll Paque Plus; Pharmacia, Katalognummer 17-1440-02) beschickt. Darauf werden 30 Milliliter des 1:4 verdünnten Nabelschnurblutes aufgeschichtet und anschließend für 30 Minuten bei 400 x g bei Raumtemperatur zentrifuigert. Die Bremsen der Zentrifuge sind dabei ausgeschaltet. Die mononukleären Zellen sammeln sich durch die Zentrifugation in der Interphase. Diese wird mit Hilfe einer 30 Milliliter-Pipette abgenommen und in ein neues 50 Milliliter Zentrifugationsgefäß überführt und das Volumen anschließend mit der Phosphat-gepufferten Salzlösung auf 30 ml aufgefüllt. Diese Zellen werden für 10 Minuten bei Raumtemperatur mit eingeschalteter Bremse bei 300 x g zentrifuigert. Der Überstand wird verworfen und das entstandene Zellpellet in 30 Milliliter Phosphat-gepufferter Salzlösung resuspendiert. Diese Zellen werden erneut für 15 Minuten bei 20 °C bei 200 x g und eingeschalteter Bremse zentrifugiert.

Zur Isolierung der CD34-positiven Zellen aus die angereicherten mononukleären Zellen in einer Konzentration von 1 X 10⁸ Zellen pro 300 Mikroliter MACS-Puffer (0.5% Endotoxin-freies bovines Serumalbumin in Phosphat-gepufferter Salzlösung) resuspendiert. Es erfolgt die Zugabe von 100 Mikrolitern FCR Blocking Reagenz (Miltenyi Biotec, Katalognummer 130-046-702) sowie 100 Mikrolitern an CD34 Micro Beads (Miltenyi Biotec, Katalognummer 130-046-702). Diese Suspension wird für 30 Minuten bei 4°C inkubiert. Anschließend werden die Zellen mit dem 20-fachern Volumen MACS Puffer verdünnen und 10 Minuten bei 300 x g zentrifugiert. Der Überstand wird verworfen und die Zellen in 500 Mikrolitern MACS Puffer resuspendiert. Die so behandelten Zellen werden auf einer LS-Säule (Miltenyi Biotec, katalognummer 130-042-401) aufgetragen und unter Verwendung eines Midi MACS Magneten (Miltenyi Biotec, Katalognummer 130-042-303) aufgereinigt.

Die Anzahl an CD34-positiven Zellen wird über das auszählen der Zellen unter Verwendung einer Neubauer-Kammer durchgeführt. Die Bestimmung der Reinheit der Zellen erfolgt nach Standardprotokollen unter Verwendung der Fluorescent Activated Cell Sorting -Methode (Becton Dickinson, BD FACS™ Sample Prep Assistant SPAII Upgrade Kit, Katalognummer 337642).

Zur Bestimmung des Einflusses einer Modulation der GSK3-Aktivität werden CD34-positive Zellen über 7 Tage in einer 96-Loch-Mikrotitterplatte bei 37°C und 5% Kohlendioxid inkubiert und anschließend die Proliferationsraten anhand der Zellzahlen bestimmt.

Zu diesem Zweck werden 5000 CD34-positive Zellen pro Loch einer 96-U-Boden-Loch-Mikrotitterplatte (Greiner Bio-One, Katalognummer 650 180) in 100 Mikroliter IMDM-Medium (Life Technology, Katalognummer 12440-046), 10% fetales Kälberserum (Life Technology, Katalognummer 10082-139) und 20 Nanogramm pro Milliliter Stem Cell Factor (R&D, Katalognummer 255-SC-010) aufgenommen. Zusätzlich werden die Zellen noch unterschiedlichen Konzentrationen an mit Dimethylsulfoxid (Sigma Aldrich, Katalognummer D5879-1L) gelösten Substanzen versetzt. Dabei werden jeweils 4 Löcher mit der angegebenen Zellzahl von 5000 CD34-positiven Zellen pro Loch mit 10 Mikromol, 4 Löcher mit 5 Mikromol, 4 Löcher mit 2.5 Mikromol, 4 Löcher mit 1.25 Mikromol, 4 Löcher mit 0.625 Mikromol, 4 Löcher mit 0.3125 Mikromol, 4 Löcher mit 0.156 Mikromol, 4 Löcher mit 0.078 Mikromol und als Kontrolle 4 Löcher mit 0.1% Dimethylsulfoxid als Endkonzentration versehen.

Diese so behandelten Zellen werden für 7 Tage in einem Zellkultur-Brutschrank bei 37°C und 5% Kohlendioxid inkubiert. Durch erneutes zählen der Zellen unter Verwendung einer Neubauer-Zählkammer wird die Proliferationsrate bestimmt, wobei die nur mit dem Stem Cell Factor versehenen Zellen als 100%-Wert gesetzt und alle anderen Werte auf diesen Wert bezogen sind.

### In-vivo Assay

Die Untersuchungen der in vivo-Wirkung der erfindungsmäßigen Verbindungen erfolgt unter Verwendung von 6 Wochen alten, 18 - 22 g schweren, männlichen C57BL/6-Mäusen (Charles River, Sulzfeld, Deutschland). Diese Tiere werden artgerecht mit 12-stündigen Licht- und Dunkelzyklen unter konstanten klimatischen Bedingungen gehalten und mit Wasser und Mausfutter *ad libitum* ernährt. Die Konzentrationen an verwendeten Chemotherapeutika werden den Tieren gemäß den Angaben der Hersteller mittels intra-peritonealen (i.p.) Injektionen im kaudalen Drittel des Bauches verabreicht. Gleichermaßen wird mit den erfindungsrelevanten Substanzen verfahren. Blutabnahmen erfolgen mit Hilfe von Pasteur-Pipetten aus dem retrobulbären Venenplexus. Die Bestimmung der Anzahl neutrophiler Granulozyten erfolgt vollautomatisch unter Verwendung von Durchflußzytometriesystemen.

### CYP-Inhibitionstest

Die Fähigkeit von Substanzen, CYP1A2, CYP 2C8, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-Isoform-spezifische Metaboliten bilden. Die Inhibitionseffekte werden bei sechs verschiedenen Konzentrationen der Testverbindungen untersucht (1.5, 3.1, 6.3, 12.5, 25 sowie 50 µM), mit dem Ausmaß der CYP-Isoform-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, dient als Kontrolle der erhaltenen Ergebnisse.

### Durchführung:

Die Inkubation von Phenacetin, Amodiaquin, Diclofenac, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.3 mM NADP, 3.3 mM MgCl₂ x 6 H₂O, 3.3 mM Glukose-6-phosphat, Glukose-6-phosphat-Dehydrogenase (0.4 U/ml) und 100 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

### Bestimmung der Löslichkeit

### Benötigte Reagenzien:

- PBS-Puffer pH 6.5: 61.86 g Natriumchlorid p.a. (z.B. Fa. Merck, Art.-Nr. 1.06404.1000), 39.54 g Natriumdihydrogenphosphat p.a. (z.B. Fa. Merck, Art.-Nr. 1.06346.1000) und 83.35 g 1 N Natriumhydroxid-Lösung (z.B. Fa. Bernd Kraft GmbH, Art.-Nr. 01030.4000) in einen 1 Liter-Messkolben einwiegen, mit Wasser auffüllen und ca. 1 Stunde rühren. Von dieser Lösung 500 ml in einen 5 Liter-Messkolben geben und mit Wasser auffüllen. Mit 1 N Natriumhydroxid-Lösung auf pH 6.5 einstellen.
- Dimethylsulfoxid (z.B. Fa. Baker, Art.-Nr. 7157.2500)
- destilliertes Wasser
- Acetonitril Chromasolv (z.B. Riedel-de Haen Art. Nr. 34851)
- 50%ige Ameisensäure p.a. (z.B. Fluka Art. Nr. 09676)

### Herstellung der Ausgangslösung:

Mindestens 1.5 mg der Testsubstanz werden in ein Wide Mouth 10mm Screw V-Vial (Fa. Glastechnik Gräfenroda GmbH, Art.-Nr. 8004-WM-H/V15µ) mit passender Schraubkappe und Septum genau eingewogen, mit Dimethylsulfoxid zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) (z.B. HJ-Bioanalytik GmbH Art.-Nr. 850289) mittels eines Liquid-Handling-Roboters. Als Lösemittel wird ein Gemisch aus Acetonitril Chromasolv / destilliertem Wasser 8:2 verwendet.

*Herstellung der Ausgangslösung für Kalibrierlösungen (Stammlösung):* 10 µl der Ausgangslösung werden mit 833 µl des Lösemittelgemisch versetzt (Konzentration = 600 µg/ml) und homogenisiert. Es werden von jeder Testsubstanz zwei 1:100 Verdünnungen in separaten DWP's hergestellt und wiederum homogenisiert. Eine der 1:100 Verdünnungen wird für die Herstellung der Kalibrierlösungen verwendet, die zweite Verdünnung wird für die Optimierung der MS/MS-Parameter verwendet.

*Kalibrierlösung 5 (600 nglml):* 30 µl der Stammlösung werden mit 270 µl Lösemittelgemisch versetzt und homogenisiert.

*Kalibrierlösung 4 (60 nglml):* 30 µl der Kalibrierlösung 5 werden mit 270 µl Lösemittelgemisch versetzt und homogenisiert.

*Kalibrierlösung 3 (12 nglml):* 100 µl der Kalibrierlösung 4 werden mit 400 µl Lösemittelgemisch versetzt und homogenisiert.

*Kalibrierlösung 2 (1.2 ng*/*ml):* 30 µl der Kalibrierlösung 3 werden mit 270 µl Lösemittelgemisch versetzt und homogenisiert.

*Kalibrierlösung 1 (0.6 nglml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP (z.B. HJ-Bioanalytik GmbH Art.-Nr. 850289) mittels eines Liquid-Handling-Roboters.

10.1 µl der Stammlösung werden mit 1000 µl PBS-Puffer pH 6.5 versetzt.

### Durchführung:

Die notwendigen Pipettierschritte erfolgen in 1.2 ml 96er DWP (z.B. HJ-Bioanalytik GmbH Art.-Nr. 850289) mittels eines Liquid-Handling-Roboters.

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 7) Probenlösung 1:10.

### HPLC/MS-MS Methode

HPLC: Agilent 1100 , quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min, Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS:WATERS Quattro Micro Tandem MS/MS; Z-Spray API-Interface; HPLC-MS-Eingangssplitter 1:20; Messung im ESI-Mode

Die Geräteparameter werden für jede Testsubstanz durch Injektion der weiter oben beschriebenen Stammlösung (zweite 1:100 Verdünnung) mittels der MassLynx/QuanOptimize-Software automatisch optimiert.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 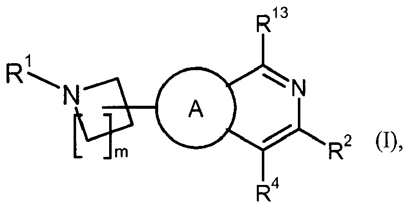 in welcher
A für eine Gruppe der Formel 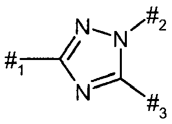 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
m für die Zahl 1, 2 oder 3 steht,
R¹ für Wasserstoff, C₁-C₆-Alkyl, C₃-C₆-Cycloalkyl, C₃-C₆-Cycloalkylmethyl oder Hydroxycarbonylmethyl steht,
R² für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Akyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
R⁴ für Wasserstoff, Chlor oder Fluor steht,
R¹³ für eine Gruppe der Formel 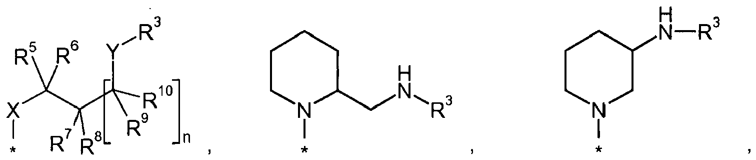 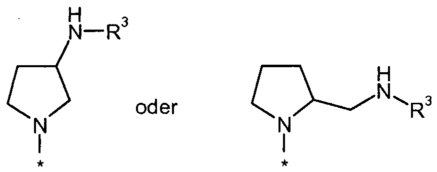 steht,
wobei
* die Anknüpfstelle an den Heterocyclus bedeutet,
n für die Zahl 0 oder 1 steht,
X für NR¹¹, S oder O steht,
wobei
R¹¹ für Wasserstoff, C₁-C₃-Alkyl oder Cyclopropyl steht,
Y für NR¹², S oder O steht,
wobei
R¹² für Wasserstoff, C₁-C₃-Alkyl oder Cyclopropyl steht,
R³ für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht,
wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
R⁵ für Wasserstoff, C₁-C₃-Alkyl oder Cyclopropyl steht,
R⁶ für Wasserstoff oder C₁-C₃-Alkyl steht,
R⁷ für Wasserstoff, C₁-C₃-Alkyl oder Cyclopropyl steht,
R⁸ für Wasserstoff oder C₁-C₃-Alkyl steht,
R⁹ für Wasserstoff, C₁-C₃-Alkyl oder Cyclopropyl steht,
R¹⁰ für Wasserstoff oder C₁-C₃-Alkyl steht,
oder eines ihrer Salze, ihrer Solvate oder der Solvate ihrer Salze.

2. Verbindung nach Anspruch 1, **dadurch gekennzeichnet, dass**
A für eine Gruppe der Formel 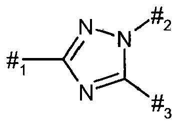 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
m für die Zahl 1, 2 oder 3 steht,
R¹ für Wasserstoff, C₁-C₆-Alkyl, C₃-C₆-Cycloalkyl, C₃-C₆-Cycloalkylmethyl oder Hydroxycarbonylmethyl steht,
R² für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl, worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
R⁴ für Wasserstoff oder Chlor steht,
R¹³ für eine Gruppe der Formel 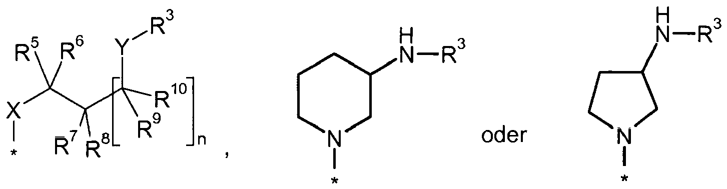 steht,
wobei
* die Anknüpfstelle an den Heterocyclus bedeutet,
n für die Zahl 0 steht,
X für NR¹¹ steht,
wobei
R¹¹ für Wasserstoff oder Methyl steht,
Y für NR¹² steht,
wobei
R¹² für Wasserstoff oder Methyl steht,
R³ für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht,
wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
oder eines ihrer Salze, ihrer Solvate oder der Solvate ihrer Salze.

3. Verbindung nach einem der Ansprüche 1 oder 2, **dadurch gekennzeichnet, dass**
A für eine Gruppe der Formel 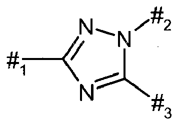 steht,
wobei
#₁ die Anknüpfstelle an den mit R¹ substituierten Heterocyclus bedeutet,
#₂ die Anknüpfstelle an das Kohlenstoffatom bedeutet, an das R¹³ gebunden ist,
#₃ die Anknüpfstelle an das Kohlenstoffatom bedeutet, an das R⁴ gebunden ist,
m für die Zahl 1, 2 oder 3 steht,
R¹ für C₁-C₄-Alkyl, Cyclopropyl, Cyclopropylmethyl oder Hydroxycarbonylmethyl steht,
R² für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl und Methyl,
R⁴ für Wasserstoff steht,
R¹³ für eine Gruppe der Formel 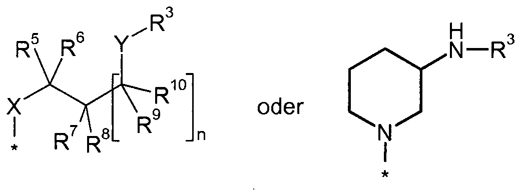 steht,
wobei
* die Anknüpfstelle an den Heterocyclus bedeutet,
n für die Zahl 0 steht,
X für NR¹¹ steht,
wobei
R¹¹ für Wasserstoff steht,
Y für NR¹² steht,
wobei
R¹² für Wasserstoff steht,
R³ für 2-Pyridyl steht,
wobei 2-Pyridyl substituiert ist mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R⁵ für Wasserstoff steht,
R⁶ für Wasserstoff steht,
R⁷ für Wasserstoff steht,
R⁸ für Wasserstoff steht,
R⁹ für Wasserstoff steht,
R¹⁰ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 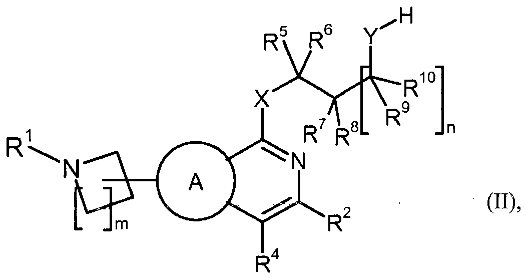 in welcher
A, m, X, Y, R¹, R², R⁴, R⁵, R⁶, R⁷, R⁸, R⁹ und R¹⁰ die in Anspruch 1 angegebene Bedeutung haben,
mit einer Verbindung der Formel
R³-X¹ (III),
in welcher
R³ die in Anspruch 1 angegebene Bedeutung hat, und
X¹ für Halogen, bevorzugt Chlor oder Fluor, steht,
umgesetzt wird,
oder
[B] eine Verbindung der Formel 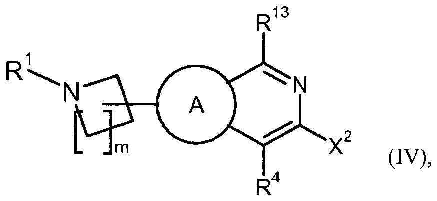 in welcher
A, m, R¹, R⁴ und R¹³ die in Anspruch 1 angegebene Bedeutung haben, und
X² für Iod, Brom, Chlor oder Trifluormethansulfonyl, bevorzugt Iod oder Brom, steht,
mit einer Verbindung der Formel
Q-R² (V),
in welcher
R² die in Anspruch 1 angegebene Bedeutung hat, und
Q für -B(OH)₂, einen Boronsäure-Ester, bevorzugt Boronsäurepinakolester, oder -BF₃⁻ K⁺ steht,
unter Suzuki-Kupplungsbedingungen umgesetzt wird,
oder
[C] eine Verbindung der Formel 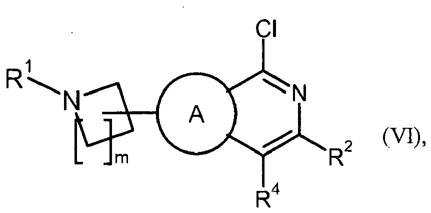 in welcher
A, m, R¹, R² und R⁴ die in Anspruch 1 angegebene Bedeutung haben,
mit einer Verbindung der Formel
H-R¹³ (IX),
in welcher
R¹³ die in Anspruch 1 angegebene Bedeutung hat,
umgesetzt wird.

5. Verbindung nach einem der Ansprüche 1 bis 3 zur Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von hämatologischen Erkrankungen.

8. Arzneimittel enthaltend eine Verbindung nach einem der Ansprüche 1 bis 3 in Kombination mit einem inerten, nichttoxischen, pharmazeutisch geeigneten Hilfsstoff.

9. Arzneimittel nach Anspruch 8 zur Behandlung und/oder Prophylaxe von hämatologischen Erkrankungen.

10. Verwendung einer Verbindung nach einem der Ansprüche 1 bis 3, zur effizienten ex vivo Vermehrung von adulten hämatopoetischen Stammzellen aus dem Knochenmark und/oder aus peripherem Blut und/oder zur *ex vivo* Vermehrung von embryonalen Stammstellen aus Nabelschnurblut.

11. Verfahren zur *ex vivo* Vermehrung von adulten hämatopoetischen Stammzellen aus dem Knochenmark und/oder aus peripherem Blut und/oder zur *ex* vivo Vermehrung von embryonalen Stammztellen aus Nabelschnurblut, **dadurch gekennzeichnet, dass** eine wirksame Menge einer Verbindung nach einem der Ansprüche 1 bis 3 zugegeben wird.

12. Verbindung, wie in einem der Ansprüche 1 bis 3 definiert, zur Verwendung in einem Verfahren zur Behandlung und/oder Prophylaxe von hämatologischen Erkrankungen.

## Claims

1. Compound of the formula 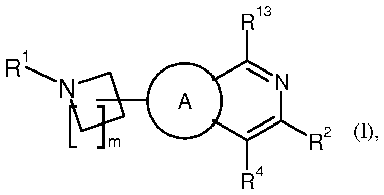 in which
A represents a group of the formula 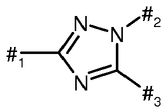 where
#₁ represents the point of attachment to the heterocycle substituted by R¹,
#₂ represents the point of attachment to the carbon atom to which R¹³ is attached,
#₃ represents the point of attachment to the carbon atom to which R⁴ is attached,
m represents the number 1, 2 or 3,
R¹ represents hydrogen, C₁-C₆-alkyl, C₃-C₆-cycloalkyl, C₃-C₆-cycloalkylmethyl or hydroxycarbonylmethyl,
R² represents phenyl,
where phenyl may be substituted by 1 to 3 substituents, where the substituents are independently of one another selected from the group consisting of hydroxyl, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulfonyl, C₁-C₄-alkylsulfonylamino, C₁-C₄-alkylaminosulfonyl, phenyl, pyrrolidinyl, piperidinyl, morpholinyl, piperazinyl, pyrrolidinylmethyl, piperidinylmethyl, morpholinylmethyl and piperazinylmethyl,
where phenyl, pyrrolidinyl, piperidinyl, morpholinyl, piperazinyl, pyrrolidinylmethyl, piperidinylmethyl, morpholinylmethyl and piperazinylmethyl may be substituted by 1 to 3 substituents, where the substituents are independently of one another selected from the group consisting of halogen, cyano, trifluoromethyl, trifluoromethoxy and C₁-C₄-alkyl,
R⁴ represents hydrogen, chlorine or fluorine,
R¹³ represents a group of the formula 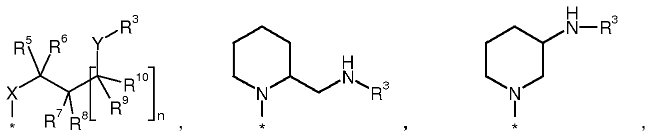 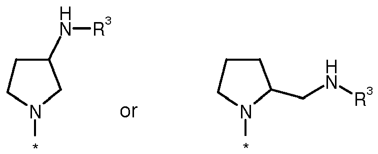 where
* represents the point of attachment to the heterocycle,
n represents the number 0 or 1,
X represents NR¹¹, S or O,
where
R¹¹ represents hydrogen, C₁-C₃-alkyl or cyclopropyl,
Y represents NR¹², S or O,
where
R¹² represents hydrogen, C₁-C₃-alkyl or cyclopropyl,
R³ represents 2-pyridyl, pyrimid-2-yl, 2-aminopyrimid-4-yl, 2-cyclopropylaminopyrimid-4-yl, 2-methylaminopyrimid-4-yl, 2-ethylaminopyrimid-4-yl, 1,3-thiazol-2-yl, 1,3-thiazol-4-yl or 1,3-thiazol-5-yl,
where 2-pyridyl, pyrimid-2-yl, 2-aminopyrimid-4-yl, 1,3-thiazol-2-yl, 1,3-thiazol-4-yl and 1,3-thiazol-5-yl are substituted by 1 to 3 substituents, where the substituents are independently of one another selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl,
where alkyl, alkoxy, alkylamino, alkylcarbonyl, alkoxycarbonyl, alkylaminocarbonyl and cycloalkylcarbonyl may be substituted by one substituent, where the substituent is selected from the group consisting of halogen, cyano, hydroxyl, amino, trifluoromethyl and C₃-C₆-cycloalkyl,
R⁵ represents hydrogen, C₁-C₃-alkyl or cyclopropyl,
R⁶ represents hydrogen or C₁-C₃-alkyl,
R⁷ represents hydrogen, C₁-C₃-alkyl or cyclopropyl,
R⁸ represents hydrogen or C₁-C₃-alkyl,
R⁹ represents hydrogen, C₁-C₃-alkyl or cyclopropyl,
R¹⁰ represents hydrogen or C₁-C₃-alkyl,
or one of its salts, its solvates or the solvates of its salts.

2. Compound according to Claim 1, **characterized in that**
A represents a group of the formula 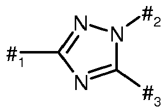 where
#₁ represents the point of attachment to the heterocycle substituted by R¹,
#₂ represents the point of attachment to the carbon atom to which R¹³ is attached,
#₃ represents the point of attachment to the carbon atom to which R⁴ is attached,
m represents the number 1, 2 or 3,
R¹ represents hydrogen, C₁-C₆-alkyl, C₃-C₆-cycloalkyl, C₃-C₆-cycloalkylmethyl or hydroxycarbonylmethyl,
R² represents phenyl,
where phenyl may be substituted by 1 to 3 substituents, where the substituents are independently of one another selected from the group consisting of hydroxyl, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulfonyl, C₁-C₄-alkylsulfonylamino, C₁-C₄-alkylaminosulfonyl, phenyl, pyrrolidinyl, piperidinyl, morpholinyl, piperazinyl, pyrrolidinylmethyl, piperidinylmethyl, morpholinylmethyl and piperazinylmethyl,
where phenyl, pyrrolidinyl, piperidinyl, morpholinyl, piperazinyl, pyrrolidinylmethyl, piperidinylmethyl, morpholinylmethyl and piperazinylmethyl may be substituted by 1 to 3 substituents, where the substituents are independently of one another selected from the group consisting of halogen, cyano, trifluoromethyl, trifluoromethoxy and C₁-C₄-alkyl,
R⁴ represents hydrogen or chlorine,
R¹³ represents a group of the formula 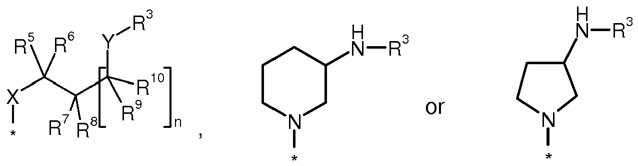 where
* represents the point of attachment to the heterocycle,
n represents the number 0,
X represents NR¹¹,
where
R¹¹ represents hydrogen or methyl,
Y represents NR¹²,
where
R¹² represents hydrogen or methyl,
R³ represents 2-pyridyl, pyrimid-2-yl, 2-aminopyrimid-4-yl, 2-cyclopropylaminopyrimid-4-yl, 2-methylaminopyrimid-4-yl, 2-ethylaminopyrimid-4-yl, 1,3-thiazol-2-yl, 1,3-thiazol-4-yl or 1,3-thiazol-5-yl,
where 2-pyridyl, pyrimid-2-yl, 2-aminopyrimid-4-yl, 1,3-thiazol-2-yl, 1,3-thiazol-4-yl and 1,3-thiazol-5-yl are substituted by 1 to 3 substituents, where the substituents are independently of one another selected from the group consisting of halogen, cyano, nitro, amino, trifluoromethyl, trifluoromethoxy, aminocarbonyl, trifluoromethylcarbonyl, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl, where alkylamino, alkylcarbonyl, alkoxycarbonyl, alkylaminocarbonyl and cycloalkylcarbonyl may be substituted by one substituent, where the substituent is selected from the group consisting of halogen, cyano, hydroxyl, amino, trifluoromethyl and C₃-C₆-cycloalkyl,
R⁵ represents hydrogen or methyl,
R⁶ represents hydrogen,
R⁷ represents hydrogen or methyl,
R⁸ represents hydrogen,
R⁹ represents hydrogen,
R¹⁰ represents hydrogen,
or one of its salts, its solvates or the solvates of its salts.

3. Compound according to Claim 1 or 2, **characterized in that**
A represents a group of the formula 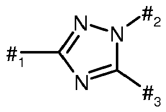 where
#₁ represents the point of attachment to the heterocycle substituted by R¹,
#₂ represents the point of attachment to the carbon atom to which R¹³ is attached,
#₃ represents the point of attachment to the carbon atom to which R⁴ is attached,
m represents the number 1, 2 or 3,
R¹ represents C₁-C₄-alkyl, cyclopropyl, cyclopropylmethyl or hydroxycarbonylmethyl,
R² represents phenyl,
where phenyl may be substituted by 1 to 3 substituents, where the substituents are independently of one another selected from the group consisting of chlorine, fluorine, trifluoromethyl and methyl,
R⁴ represents hydrogen,
R¹³ represents a group of the formula 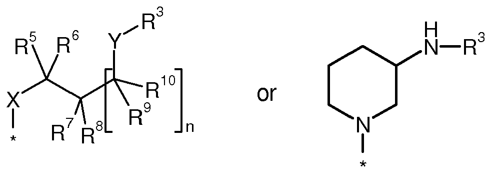 where
* represents the point of attachment to the heterocycle,
n represents the number 0,
X represents NR¹¹,
where
R¹¹ represents hydrogen,
Y represents NR¹²,
where
R¹² represents hydrogen,
R³ represents 2-pyridyl,
where 2-pyridyl is substituted by 1 or 2 substituents, where the substituents are independently of one another selected from the group consisting of cyano, nitro, amino, trifluoromethylcarbonyl, ethylcarbonyl and methylcarbonyl,
R⁵ represents hydrogen,
R⁶ represents hydrogen,
R⁷ represents hydrogen,
R⁸ represents hydrogen,
R⁹ represents hydrogen,
R¹⁰ represents hydrogen,
or one of its salts, its solvates or the solvates of its salts.

4. Process for preparing a compound of the formula (I), or one of its salts, its solvates or the solvates of its salts according to Claim 1, **characterized in that**
[A] a compound of the formula 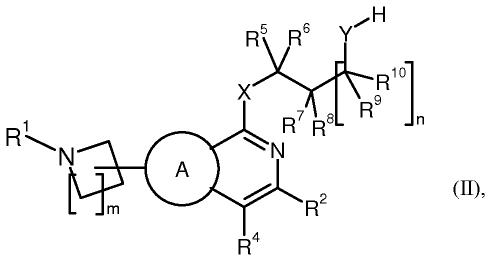 in which
A, m, X, Y, R¹, R², R⁴, R⁵, R⁶, R⁷, R⁸, R⁹ and R¹⁰ have the meaning given in Claim 1,
is reacted with a compound of the formula
R³-X¹ (III),
in which
R³ has the meaning given in Claim 1, and
X¹ represents halogen, preferably chlorine or fluorine,
or
[B] a compound of the formula 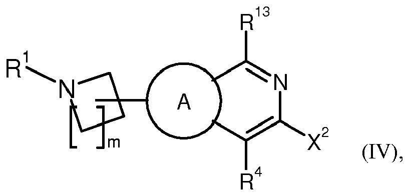 in which
A, m, R¹, R⁴ and R¹³ have the meaning given in Claim 1, and
X² represents iodine, bromine, chlorine or trifluoromethanesulfonyl, preferably iodine or bromine,
is reacted with a compound of the formula
Q-R² (V),
in which
R² has the meaning given in Claim 1, and
Q represents -B(OH)₂, a boronic acid ester, preferably boronic acid pinacol ester, or -BF₃⁻K⁺,
under Suzuki coupling conditions,
or
[C] a compound of the formula 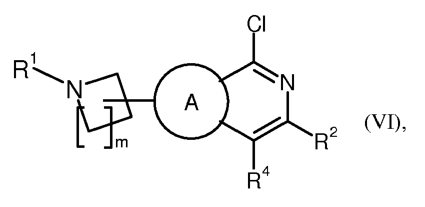 in which
A, m, R¹, R² and R⁴ have the meaning given in Claim 1,
is reacted with a compound of the formula
H-R¹³ (IX),
in which
R¹³ has the meaning given in Claim 1.

5. Compound according to any of Claims 1 to 3 for the treatment and/or prophylaxis of diseases.

6. Use of a compound according to any of Claims 1 to 3 for preparing a medicament for the treatment and/or prophylaxis of diseases.

7. Use of a compound according to any of Claims 1 to 3 for preparing a medicament for the treatment and/or prophylaxis of haematologic disorders.

8. Medicament comprising a compound according to any of Claims 1 to 3 in combination with an inert non-toxic pharmaceutically acceptable auxiliary.

9. Medicament according to Claim 8 for the treatment and/or prophylaxis of haematological disorders.

10. Use of a compound according to any of Claims 1 to 3 for the efficient *ex vivo* expansion of adult haematopoietic stem cells from bone marrow and/or from peripheral blood and/or for the *ex vivo* expansion of embryonal stem cells from umbilical cord blood.

11. Method for the *ex vivo* expansion of adult haematopoietic stem cells from bone marrow and/or from peripheral blood and/or for the *ex vivo* expansion of embryonal stem cells from umbilical cord blood, **characterized in that** an effective amount of a compound according to any of Claims 1 to 3 is added.

12. Compound as defined in any of Claims 1 to 3 for use in a method for the treatment and/or prophylaxis of haematologic disorders.

## Revendications

1. Composé de formule 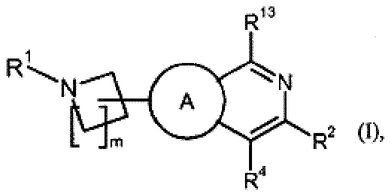 dans laquelle
A représente un groupe de formule 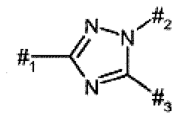 où
#₁ représente le point de liaison à l'hétérocycle substitué par R¹,
#₂ représente le point de liaison à l'atome de carbone auquel est lié R¹³,
#₃ représente le point de liaison à l'atome de carbone auquel est lié R⁴,
m représente le nombre 1, 2 ou 3,
R¹ représente un atome d'hydrogène, un groupe alkyle en C₁-C₆, cycloalkyle en C₃-C₆, cycloalkyl(C₃-C₆)-méthyle ou hydroxycarbonylméthyle,
R² représente un groupe phényle,
le groupe phényle pouvant être substitué par 1 à 3 substituants, les substituants étant choisis chacun indépendamment dans le groupe constitué par hydroxy, halogéno, cyano, trifluorométhyle, trifluorométhoxy, aminocarbonyle, alkyle en C₁-C₄, alcoxy en C₁-C₄, alcoxy(C₁-C₄)méthyle, alkyl(C₁-C₄)-aminométhyle, alkyl(C₁-C₄)carbonyle, alcoxy(C₁-C₄)-carbonyle, alkyl(C₁-C₄)aminocarbonyle, alkyl(C₁-C₄)carbonylamino, alkyl(C₁-C₄)sulfonyle, alkyl(C₁-C₄)sulfonylamino, alkyl(C₁-C₄)amino-sulfonyle, phényle, pyrrolidinyle, pipéridinyle, morpholinyle, pipérazinyle, pyrrolidinylméthyle, pipéridinylméthyle, morpholinylméthyle et pipérazinylméthyle,
où les groupes phényle, pyrrolidinyle, pipéridinyle, morpholinyle, pipérazinyle, pyrrolidinylméthyle, pipéridinylméthyle, morpholinylméthyle et pipérazinylméthyle peuvent être substitués par 1 à 3 substituants, les substituants étant choisis chacun indépendamment dans le groupe constitué par halogéno, cyano, trifluorométhyle, trifluorométhoxy et alkyle en C₁-C₄,
R⁴ représente un atome d'hydrogène, de chlore ou de fluor,
R¹³ représente un groupe de formule 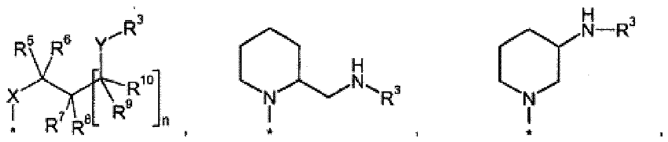 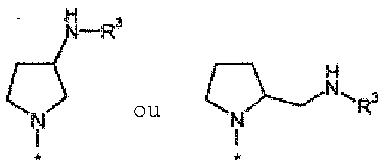 où
* représente le point de liaison à l'hétérocycle,
n représente le nombre 0 ou 1,
X représente NR¹¹, S ou O,
R¹¹ représentant un atome d'hydrogène, un groupe alkyle en C₁-C₃ ou cyclopropyle,
Y représente NR¹², S ou O,
R¹² représentant un atome d'hydrogène, un groupe alkyle en C₁-C₃ ou cyclopropyle,
R³ représente un groupe 2-pyridyle, pyrimid-2-yle, 2-aminopyrimid-4-yle, 2-cyclopropyl-aminopyrimid-4-yle, 2-méthylaminopyrimid-4-yle, 2-éthylaminopyrimid-4-yle, 1,3-thiazol-2-yle, 1,3-thiazol-4-yle ou 1,3-thiazol-5-yle,
les groupes 2-pyridyle, pyrimid-2-yle, 2-aminopyrimid-4-yle, 1,3-thiazol-2-yle, 1,3-thiazol-4-yle et 1,3-thiazol-5-yle étant substitués par 1 à 3 substituants, les substituants étant choisis chacun indépendamment dans le groupe constitué par halogéno, cyano, nitro, amino, trifluorométhyle, trifluorométhoxy, aminocarbonyle, trifluorométhylcarbonyle, alkyle en C₁-C₄, alcoxy en C₁-C₄, alkyl(C₁-C₄)-amino, alkyl(C₁-C₄)carbonyle, alcoxy(C₁-C₄)-carbonyle, alkyl(C₁-C₄)aminocarbonyle et cycloalkyl(C₃-C₆)carbonyle,
les groupes alkyle, alcoxy, alkylamino, alkylcarbonyle, alcoxycarbonyle, alkyl-aminocarbonyle et cycloalkylcarbonyle pouvant être substitués par un substituant, le substituant étant choisi dans le groupe constitué par halogéno, cyano, hydroxy, amino, trifluorométhyle et cycloalkyle en C₃-C₆,
R⁵ représente un atome d'hydrogène, un groupe alkyle en C₁-C₃ ou cyclopropyle,
R⁶ représente un atome d'hydrogène ou un groupe alkyle en C₁-C₃,
R⁷ représente un atome d'hydrogène, un groupe alkyle en C₁-C₃ ou cyclopropyle,
R⁸ représente un atome d'hydrogène ou un groupe alkyle en C₁-C₃,
R⁹ représente un atome d'hydrogène, un groupe alkyle en C₁-C₃ ou cyclopropyle,
R¹⁰ représente un atome d'hydrogène ou un groupe alkyle en C₁-C₃,
ou un de ses sels, de ses produits de solvatation ou des produits de solvatation de ses sels.

2. Composé selon la revendication 1, **caractérisé en ce que**
A représente un groupe de formule 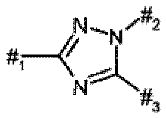 où
#₁ représente le point de liaison à l'hétérocycle substitué par R¹,
#₂ représente le point de liaison à l'atome de carbone auquel est lié R¹³,
#₃ représente le point de liaison à l'atome de carbone auquel est lié R⁴,
m représente le nombre 1, 2 ou 3,
R¹ représente un atome d'hydrogène, un groupe alkyle en C₁-C₆, cycloalkyle en C₃-C₆, cycloalkyl(C₃-C₆)-méthyle ou hydroxycarbonylméthyle,
R² représente un groupe phényle,
le groupe phényle pouvant être substitué par 1 à 3 substituants, les substituants étant choisis chacun indépendamment dans le groupe constitué par hydroxy, halogéno, cyano, trifluorométhyle, trifluorométhoxy, aminocarbonyle, alkyle en C₁-C₄, alcoxy en C₁-C₄, alcoxy(C₁-C₄)méthyle, alkyl(C₁-C₄)-aminométhyle, alkyl(C₁-C₄)carbonyle, alcoxy(C₁-C₄)-carbonyle, alkyl(C₁-C₄)aminocarbonyle, alkyl(C₁-C₄)carbonylamino, alkyl(C₁-C₄)sulfonyle, alkyl(C₁-C₄)sulfonylamino, alkyl(C₁-C₄)amino-sulfonyle, phényle, pyrrolidinyle, pipéridinyle, morpholinyle, pipérazinyle, pyrrolidinylméthyle, pipéridinylméthyle, morpholinylméthyle et pipérazinylméthyle,
où les groupes phényle, pyrrolidinyle, pipéridinyle, morpholinyle, pipérazinyle, pyrrolidinylméthyle, pipéridinylméthyle, morpholinylméthyle et pipérazinylméthyle peuvent être substitués par 1 à 3 substituants, les substituants étant choisis chacun indépendamment dans le groupe constitué par halogéno, cyano, trifluorométhyle, trifluorométhoxy et alkyle en C₁-C₄,
R⁴ représente un atome d'hydrogène ou de chlore,
R¹³ représente un groupe de formule 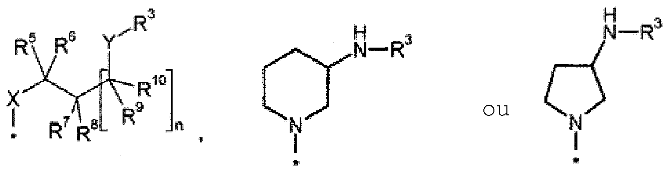 où
* représente le point de liaison à l'hétérocycle,
n représente le nombre 0,
X représente NR¹¹,
R¹¹ représentant un atome d'hydrogène ou le groupe méthyle,
Y représente NR¹²,
R¹² représentant un atome d'hydrogène ou le groupe méthyle,
R³ représente un groupe 2-pyridyle, pyrimid-2-yle, 2-aminopyrimid-4-yle, 2-cyclopropyl-aminopyrimid-4-yle, 2-méthylaminopyrimid-4-yle, 2-éthylaminopyrimid-4-yle, 1,3-thiazol-2-yle, 1,3-thiazol-4-yle ou 1,3-thiazol-5-yle,
les groupes 2-pyridyle, pyrimid-2-yle, 2-aminopyrimid-4-yle, 1,3-thiazol-2-yle, 1,3-thiazol-4-yle et 1,3-thiazol-5-yle étant substitués par 1 à 3 substituants, les substituants étant choisis chacun indépendamment dans le groupe constitué par halogéno, cyano, nitro, amino, trifluorométhyle, trifluorométhoxy, aminocarbonyle, trifluorométhylcarbonyle, alkyl(C₁-C₄)amino, alkyl(C₁-C₄)carbonyle, alcoxy(C₁-C₄)carbonyle, alkyl(C₁-C₄)amino-carbonyle et cycloalkyl(C₃-C₆)carbonyle,
les groupes alkylamino, alkylcarbonyle, alcoxycarbonyle, alkylaminocarbonyle et cycloalkylcarbonyle pouvant être substitués par un substituant, le substituant étant choisi dans le groupe constitué par halogéno, cyano, hydroxy, amino, trifluorométhyle et cycloalkyle en C₃-C₆,
R⁵ représente un atome d'hydrogène ou le groupe méthyle,
R⁶ représente un atome d'hydrogène,
R⁷ représente un atome d'hydrogène ou le groupe méthyle,
R⁸ représente un atome d'hydrogène,
R⁹ représente un atome d'hydrogène,
R¹⁰ représente un atome d'hydrogène,
ou un de ses sels, de ses produits de solvatation ou des produits de solvatation de ses sels.

3. Composé selon la revendication 1 ou 2, **caractérisé en ce que**
A représente un groupe de formule 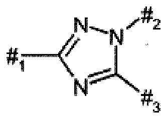 où
#₁ représente le point de liaison à l'hétérocycle substitué par R¹,
#₂ représente le point de liaison à l'atome de carbone auquel est lié R¹³,
#₃ représente le point de liaison à l'atome de carbone auquel est lié R⁴,
m représente le nombre 1, 2 ou 3,
R¹ représente un groupe alkyle en C₁-C₄, cyclopropyle, cyclopropylméthyle ou hydroxycarbonylméthyle,
R² représente un groupe phényle,
le groupe phényle pouvant être substitué par 1 à 3 substituants, les substituants étant choisis chacun indépendamment dans le groupe constitué par chloro, fluoro, trifluorométhyle et méthyle,
R⁴ représente un atome d'hydrogène,
R¹³ représente un groupe de formule 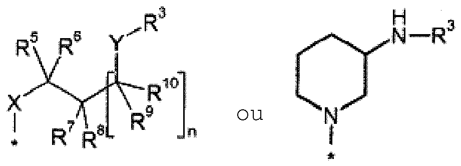 où
* représente le point de liaison à l'hétérocycle,
n représente le nombre 0,
X représente NR¹¹,
R¹¹ représentant un atome d'hydrogène,
Y représente NR¹²,
R¹² représentant un atome d'hydrogène,
R³ représente un groupe 2-pyridyle,
le groupe 2-pyridyle étant substitué par 1 ou 2 substituants, les substituants étant choisis indépendamment l'un de l'autre dans le groupe constitué par cyano, nitro, amino, trifluorométhylcarbonyle, éthylcarbonyle et méthylcarbonyle,
R⁵ représente un atome d'hydrogène,
R⁶ représente un atome d'hydrogène,
R⁷ représente un atome d'hydrogène,
R⁸ représente un atome d'hydrogène,
R⁹ représente un atome d'hydrogène,
R¹⁰ représente un atome d'hydrogène,
ou un de ses sels, de ses produits de solvatation ou des produits de solvatation de ses sels.

4. Procédé pour la préparation d'un composé de formule (I) ou d'un de ses sels, de ses produits de solvatation ou des produits de solvatation de ses sels selon la revendication 1, **caractérisé en ce que**
[A] on fait réagir un composé de formule 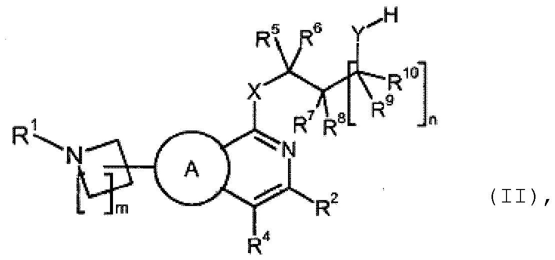 dans laquelle
A, m, X, Y, R¹, R², R⁴, R⁵, R⁶, R⁷, R⁸, R⁹ et R¹⁰ ont la signification indiquée dans la revendication 1,
avec un composé de formule
R³-X¹ (III),
dans laquelle
R³ a la signification indiquée dans la revendication 1, et
X¹ représente un atome d'halogène, de préférence de chlore ou de fluor,
ou
[B] on fait réagir un composé de formule 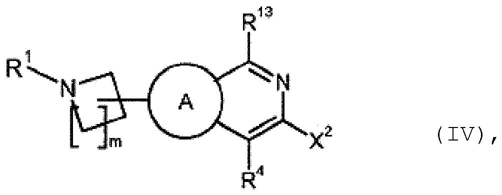 dans laquelle
A, m, R¹, R⁴ et R¹³ ont la signification indiquée dans la revendication 1, et
X² représente un atome d'iode, de brome, de chlore ou le groupe trifluorométhanesulfonyle, de préférence un atome d'iode ou de brome,
avec un composé de formule
Q-R² (V),
dans laquelle
R² a la signification indiquée dans la revendication 1, et
Q représente -B(OH)₂, un ester d'acide boronique, de préférence le boronate de pinacol, ou -BF₃⁻K⁺,
dans des conditions d'un couplage de Suzuki,
ou
[C] on fait réagir un composé de formule 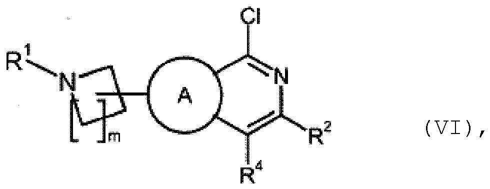 dans laquelle
A, m, R¹, R² et R⁴ ont la signification indiquée dans la revendication 1,
avec un composé de formule
H-R¹³ (IX),
dans laquelle
R¹³ a la signification indiquée dans la revendication 1.

5. Composé selon l'une quelconque des revendications 1 à 3, destiné au traitement et/ou à la prophylaxie de maladies.

6. Utilisation d'un composé selon l'une quelconque des revendications 1 à 3, pour la fabrication d'un médicament destiné au traitement et/ou à la prophylaxie de maladies.

7. Utilisation d'un composé selon l'une quelconque des revendications 1 à 3, pour la fabrication d'un médicament destiné au traitement et/ou à la prophylaxie de maladies hématologiques.

8. Médicament contenant un composé selon l'une quelconque des revendications 1 à 3, en association avec un adjuvant inerte, non toxique, pharmaceutiquement convenable.

9. Médicament selon la revendication 8, destiné au traitement et/ou à la prophylaxie de maladies hématologiques.

10. Utilisation d'un composé selon l'une quelconque des revendications 1 à 3, pour la multiplication efficace ex vivo de cellules souches hématopoïétiques adultes provenant de la moelle osseuse et/ou de sang périphérique et/ou pour la multiplication ex vivo de cellules souches embryonnaires provenant de sang de cordon ombilical.

11. Procédé pour la multiplication ex vivo de cellules souches hématopoïétiques adultes provenant de la moelle osseuse et/ou de sang périphérique et/ou pour la multiplication ex vivo de cellules souches embryonnaires provenant de sang de cordon ombilical, **caractérisé en ce qu'**on ajoute une quantité efficace d'un composé selon l'une quelconque des revendications 1 à 3.

12. Composé, tel que défini dans l'une quelconque des revendications 1 à 3, pour l'utilisation dans un procédé destiné au traitement et/ou à la prophylaxie de maladies hématologiques.
